# EUROPEAN PATENT APPLICATION

(11) **EP 4 495 227 A1**
(43) Date of publication of application: **22.01.2025**
(21) Application number: 23186041.2
(22) Date of filing: 18.07.2023
(51) Int. Cl.: C12N 5/00

(54) **CO-CULTURES OF ORGANOIDS AND STROMAL CELLS**

(71) Applicant: HUB Organoids IP B.V., 3584 CM Utrecht (NL)
(72) Inventor: ALDEGUER, Javier Frias, 3584 CM Utrecht (NL); POTT, Johanna, 3584 CM Utrecht (NL); VRIES, Robert Gerhardus Jacob, 3584 CM Utrecht (NL)
(74) Representative: Carpmaels & Ransford LLP

(57) **Abstract**

The present invention relates to organoid co-cultures and their use in the investigation of diseases.

## Description

All documents cited herein are incorporated by reference in their entirety.

### TECHNICAL FIELD

The present invention relates to organoid co-cultures and their use in the investigation of diseases.

### BACKGROUND

Organoids are a promising tool to study human physiology *in vitro.* Organoids are self-organized epithelial cell structures with physiological features that resemble cellular *in vivo* organization. They have been extensively used to model aspects of disease initiation and progression. Stem-cell-derived organoids therefore provide sophisticated models for studying human development and disease.

Epithelial organoid models may be enhanced by the integration of non-epithelial cells, forming a "co-culture". However, it is difficult to devise protocols and conditions under which very different cell types could co-exist *in vitro,* let alone whether such co-existence would facilitate relevant cell-cell interactions.

Stromal cells are a type of non-epithelial cell. As a constituent part of the "stroma" associated with organs of the body, stromal cells provide structural support to organs. Traditionally considered an 'inert' (or purely structural) component, stromal cells have recently been identified as performing key, active roles in metabolism, signaling, and disease - functions traditionally associated with non-stromal, epithelial cell types.

For example, the stroma is thought play a pivotal role in gastrointestinal disorders, such as inflammatory bowel disease (IBD). Activation of the stromal compartment has been observed in IBD patients, including an overabundance of proinflammatory fibroblasts. At the molecular level, the intestinal cells with which such proinflammatory fibroblasts interact are thought to exhibit increased apoptosis, hyperplasia or hypertrophy, and remodeling of the intestinal stem cell niche. These factors are thought to contribute to the chronicity of IBD, and to resistance to therapy. Nevertheless, the aetiology of IBD disorders remains unknown, and prior *in vitro* models fail to replicate *in vivo* physiology.

The invention provides a co-culture formed by combining at least one organoid and at least one stromal cell. As described in the Examples, such co-cultures have been validated and thereby shown to replicate *in vivo* microscopic structures, cell-cell interactions, and biochemical responses (such as inflammatory responses), and to be competent to do so over an extended period of time. Such co-cultures may be used in *in vitro* methods relating to therapy, diagnosis, and/or prognosis of disease, particularly for diseases concerning inflammation and/or fibrosis.

For instance, the inventors have replicated the *in vivo* inflammatory process by exposing such co-cultures comprising at least one organoid and at least one stromal cell, to at least one proinflammatory stimulus to induce a proinflammatory profile in the co-culture, followed by at least one inflammatory stimulus to induce an inflammatory response. Such co-cultures express expected biomarkers of inflammation (such as IL-6 and CXCL2).

Such co-cultures are useful in the testing of therapeutic agents, for example by determining a reduction in biomarkers of inflammation when a therapeutic agent is applied to the co-culture. A reduction in biomarkers of inflammation indicates, for example, that the therapeutic agent is addressing a specific molecular pathway contributing to the production of biomarkers of inflammation, and therefore indicates that the therapeutic agent may be able to treat disease *in vivo.*

Such co-cultures are also useful in diagnosis and/or prognosis, for example by determining whether diagnostic agents known to induce inflammation through specific molecular pathways are capable of increasing inflammation in co-cultures comprising at least one PDO. If such diagnostic agents do not increase biomarkers of inflammation, this may indicate that the specific molecular pathway targeted by the diagnostic agent is saturated (i.e. cannot be further activated), and therefore that the specific molecular pathway may be operative in the disease of the patient. Similar backward induction may be used when a known therapeutic agent is used as a diagnostic agent; if the known therapeutic agent is able to reduce biomarkers of inflammation in a co-culture comprising at least one PDO, then the molecular element targeted by the therapeutic agent may be operative in the disease of the patient.

### SUMMARY OF INVENTION

The invention provides a method of preparing a co-culture comprising at least one organoid and at least one stromal cell, the method comprising:
combining the at least one organoid with the at least one stromal cell in a co-culture medium to form the co culture,
optionally wherein:
(a) the method comprises preparing the at least one stromal cell by culturing stromal cells in a stromal cell medium; and/or
(b) the method comprises preparing the at least one organoid by culturing epithelial cells in an organoid medium.

The invention further provides a method for determining the presence or absence of at least one change in a co-culture comprising at least one organoid and at least one stromal cell, wherein the method comprises:
combining the at least one organoid with the at least one stromal cell in a co-culture medium to form the co-culture; and
determining the presence or absence of the at least one change in the co-culture, optionally wherein:
   (a) the method comprises preparing the at least one stromal cell by culturing stromal cells in a stromal cell medium; and/or
   (b) the method comprises preparing the at least one organoid by culturing epithelial cells in an organoid medium.

The invention further provides a method for determining the presence or absence of at least one change in at least one organoid, wherein the method comprises:
isolating conditioned medium from at least one stromal cell, and combining the conditioned medium with at least one organoid; and
determining the presence or absence of at least one change in the organoid, optionally wherein:
   (a) the method comprises preparing the at least one stromal cell by culturing stromal cells in a stromal cell medium; and/or
   (b) the method comprises preparing the at least one organoid by culturing epithelial cells in an organoid medium.

The invention further provides a method of testing at least one therapeutic agent for a disease, the method comprising:
combining at least one organoid with at least one stromal cell in a co-culture medium to form a co culture;
applying the at least one therapeutic agent to the co-culture; and
determining the presence or absence of at least one change in the co-culture, optionally wherein:
   (a) the method comprises preparing the at least one stromal cell by culturing stromal cells in a stromal cell medium; and/or
   (b) the method comprises preparing the at least one organoid by culturing epithelial cells in an organoid medium.

The invention further provides a method of testing at least one therapeutic agent for a disease, the method comprising:
isolating conditioned medium from at least one stromal cell, and combining the conditioned medium with at least one organoid;
applying the at least one therapeutic agent to the organoid; and
determining the presence or absence of at least one change in the organoid, optionally wherein:
   (a) the method comprises preparing the at least one stromal cell by culturing stromal cells in a stromal cell medium; and/or
   (b) the method comprises preparing the at least one organoid by culturing epithelial cells in an organoid medium.

The invention further provides a method of determining the presence or absence of a diagnosis and/or prognosis of a disease in a subject, the method comprising:
combining at least one organoid with at least one stromal cell in a co-culture medium to form a co culture;
applying at least one diagnostic agent to the co-culture; and
determining the presence or absence of at least one change in the co-culture,
optionally wherein:
(a) the method comprises preparing the at least one stromal cell by culturing stromal cells in a stromal cell medium; and/or
(b) the method comprises preparing the at least one organoid by culturing epithelial cells in an organoid medium.

The invention further provides a method of determining the presence or absence of a diagnosis and/or prognosis of a disease in a subject, the method comprising:
isolating conditioned medium from at least one stromal cell, and combining the conditioned medium with at least one organoid;
applying at least one diagnostic agent to the at least one organoid; and
determining the presence or absence of at least one change in the at least one organoid, optionally wherein:
   (a) the method comprises preparing the at least one stromal cell by culturing stromal cells in a stromal cell medium; and/or
   (b) the method comprises preparing the at least one organoid by culturing epithelial cells in an organoid medium.

The invention further provides a co-culture of the methods of the invention.

The invention further provides a co-culture medium of the methods of the invention.

The invention further provides a stromal cell medium of the methods of the invention.

### BRIEF DESCRIPTION OF DRAWINGS

Embodiments of the invention will be described, by way of example, with reference to the following drawings, in which:

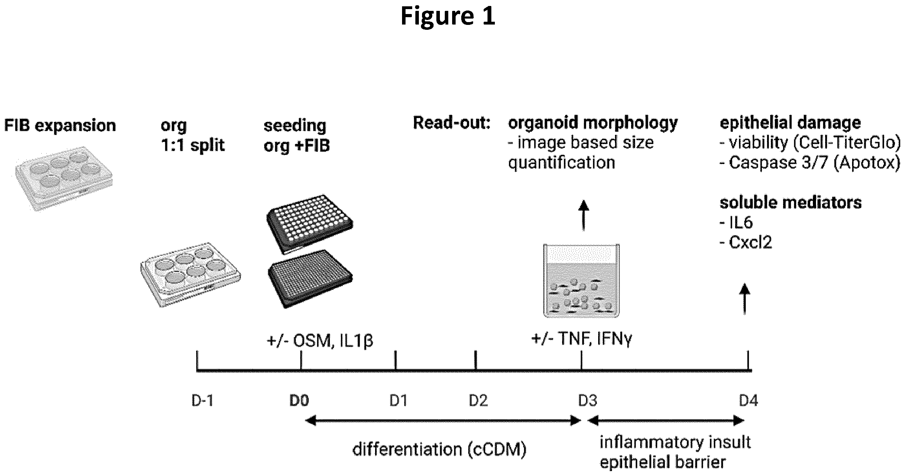

**Figure 1** shows a schematic of the process for preparing a co-culture and inducing a proinflammatory profile for analysis, as described in the Examples. Abbreviations: "FIB", fibroblast; "org", organoid.

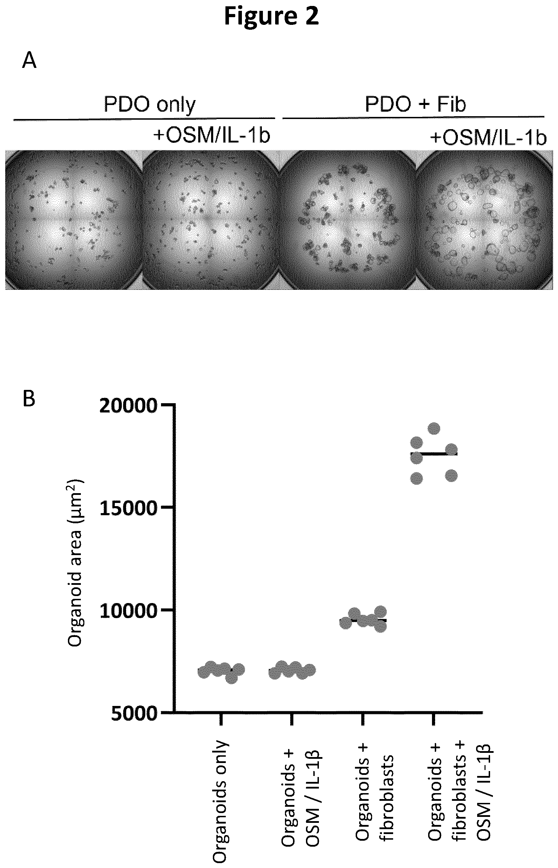

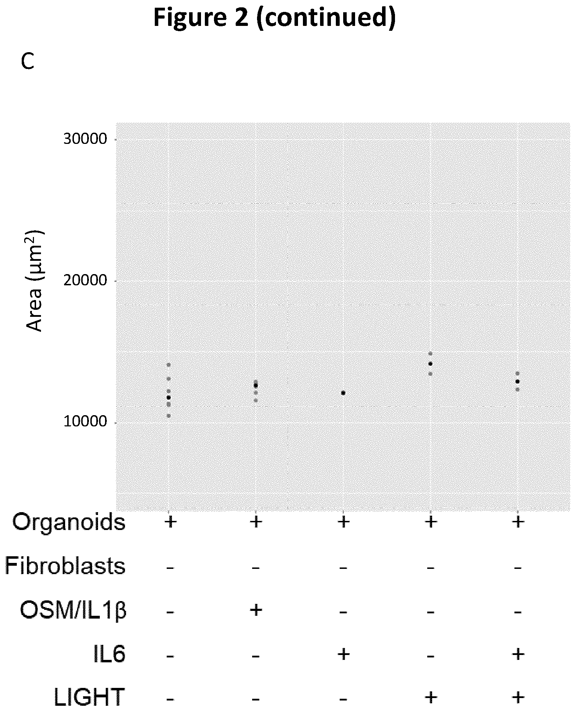

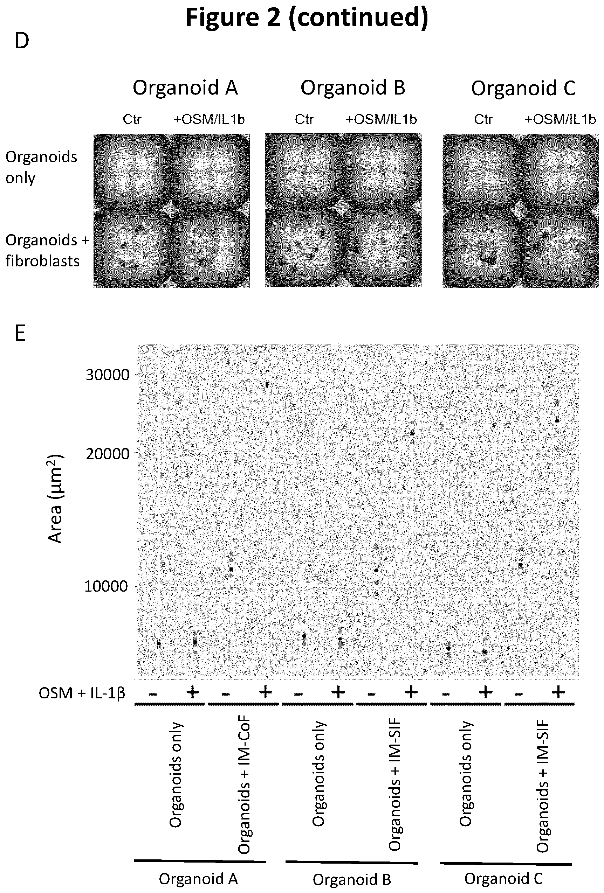

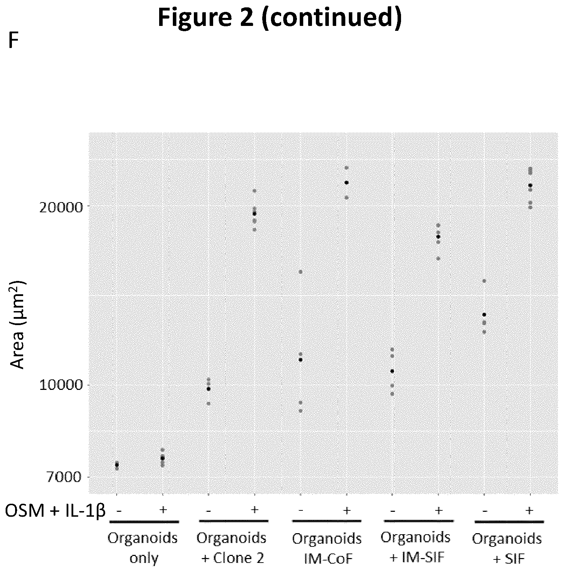

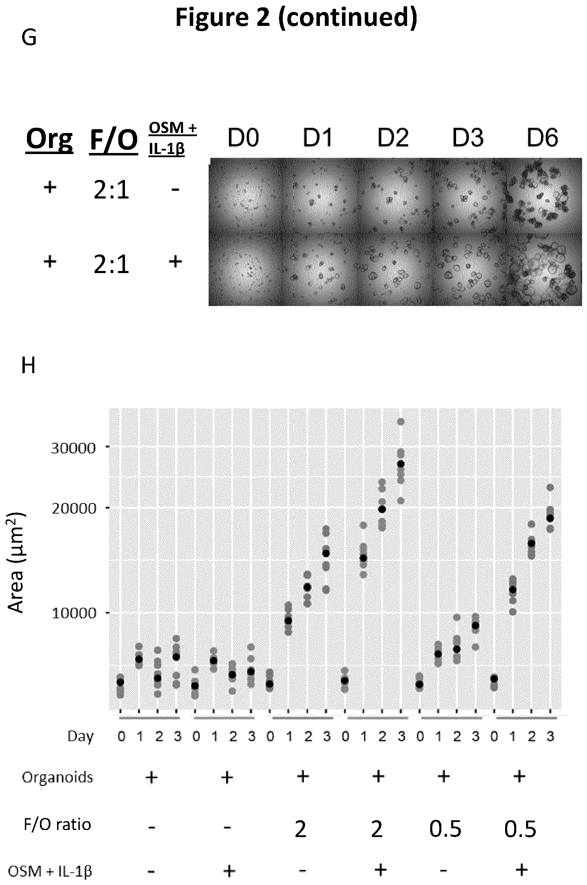

**Figure 2** shows imaging results for patient-derived organoids ("PDOs") cultured with and without fibroblasts ("Fib"), and with and without proinflammatory stimuli ("OSM/IL-1b"), as described in **Example 4.** **Figure 2A** shows brightfield images. **Figure 2B** and **Figure 2C** show organoid area (µm²) under various conditions. **Figure 2D** shows brightfield images for different types of PDO ("A", "B", and "C"), with and without fibroblasts, with proinflammatory stimulation ("+OSM/IL1b") or without proinflammatory stimulation ("Ctr"). **Figure 2E** and **Figure 2F** show organoid area (µm²) for different types of PDO, with and without different types of immortalized fibroblast ("IM-CoF", "IM-SIF") or primary fibroblast ("Clone2", "SIF") - see also **Table 1** below. **Figure 2G** and **Figure 2H** show between them images and area (µm²) measurements for organoids ("Org") with or without fibroblasts at specified ratios ("F/O"), over an extended period (up to day 3, "D3", or up to day 6, "D6"), with and without proinflammatory stimulation ("OSM + IL1β").

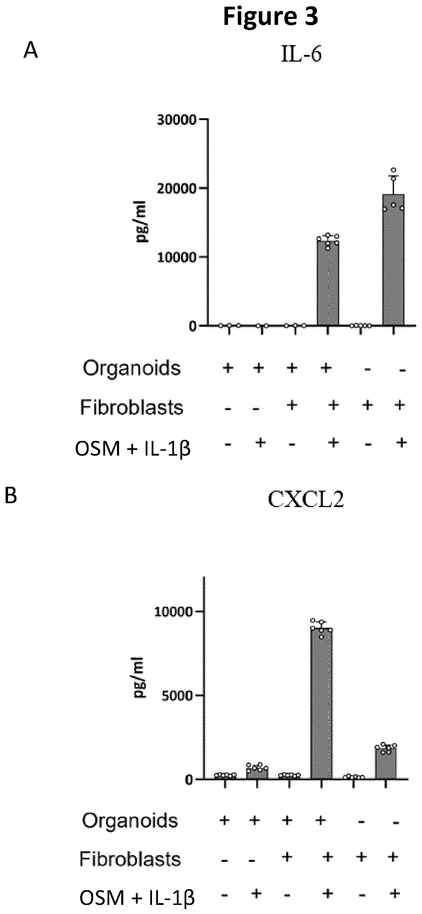

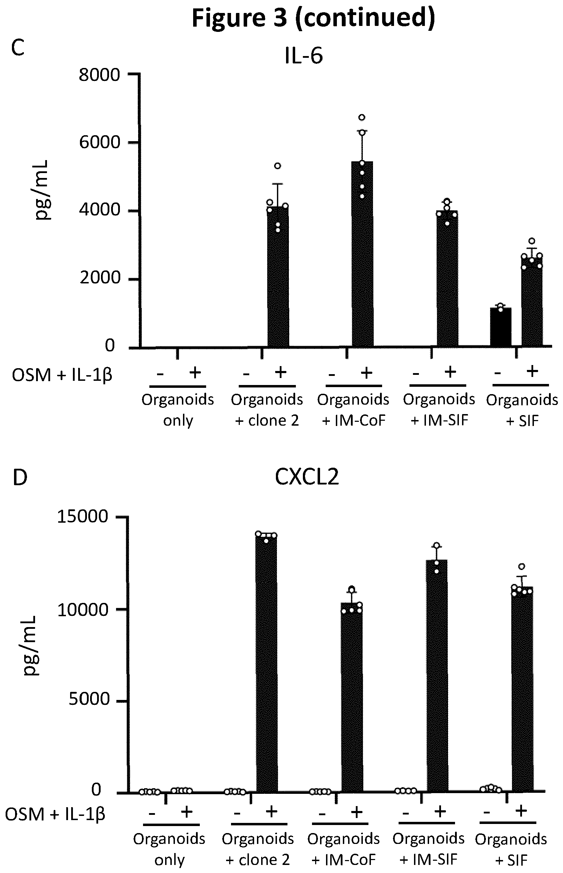

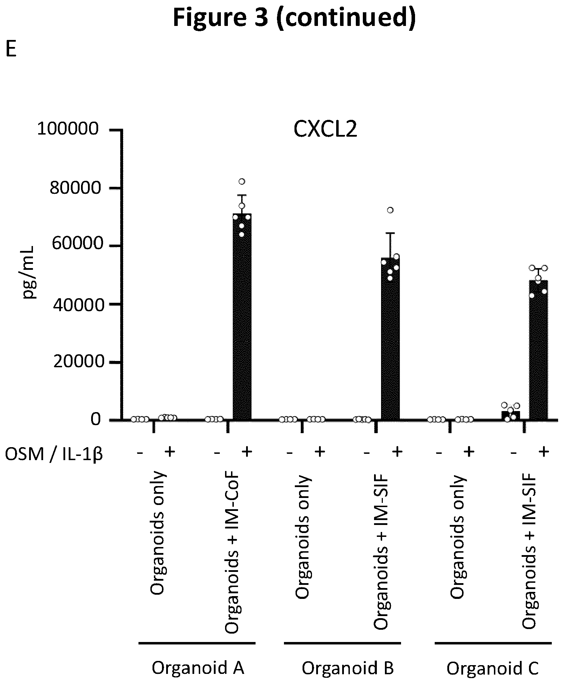

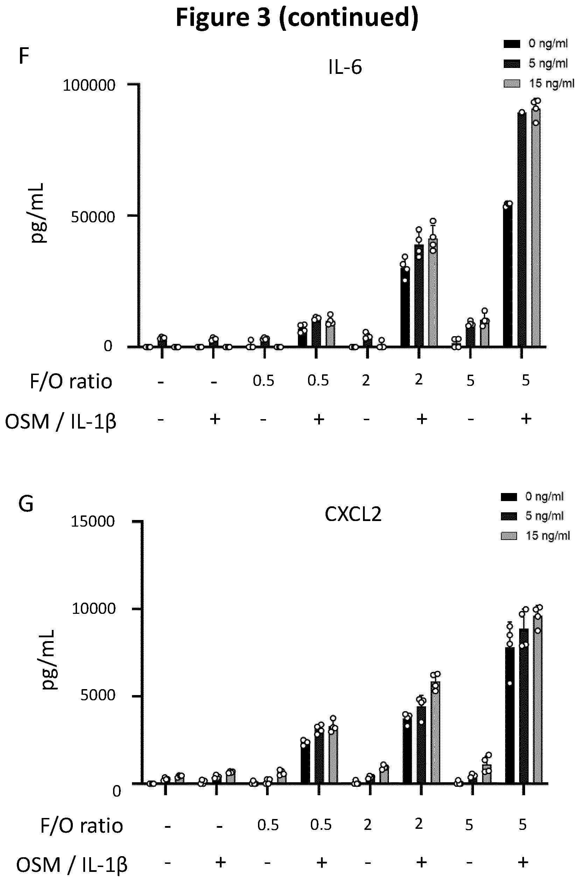

**Figure 3** shows measurements of secretion results for organoids cultured with and without fibroblasts, with and without proinflammatory stimuli ("OSM + IL-1β"), as described in **Example 5**. Shown are secretion of: **(A)** IL-6; **(B)** CXCL2; **(C)** IL-6 for different fibroblast cell lines, and **(D)** IL-6 for different fibroblast cell lines; **(E)** CXCL2 for different primary organoids ("A", "B" and "C") and different fibroblast cell lines; and **(F)** IL-6 and **(G)** CXCL2, for different fibroblast-organoid ratios ("F/O") and different concentrations of IL-1β and OSM proinflammatory stimuli (0 ng/ml, 5 ng/ml, 15 ng/ml). See also **Table 1.**

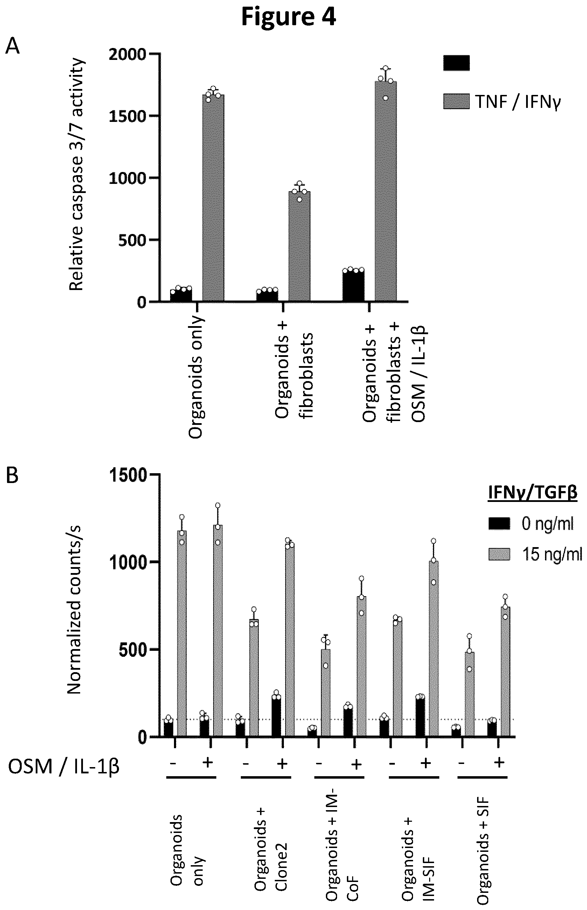

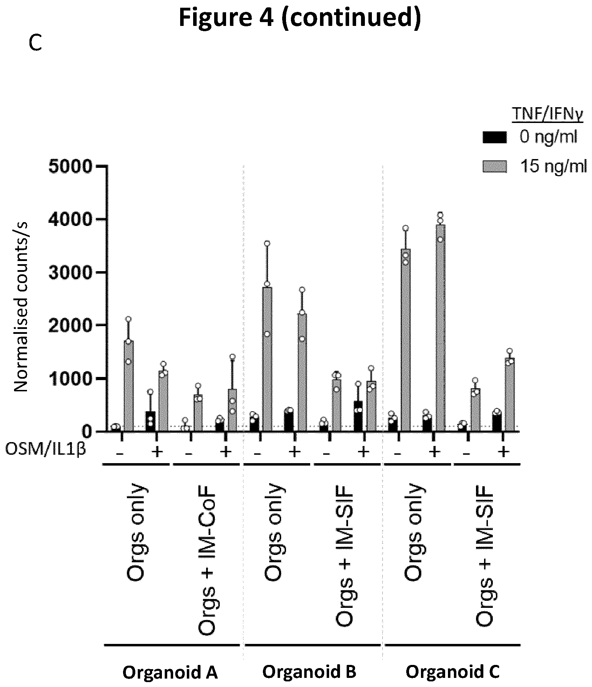

**Figure** 4 shows measurements of **(A)** caspase activity, **(B)** caspase activity in different fibroblast cell lines, and **(C)** caspase activity in different organoids, as described in **Example 6**. "PDO" = patient-derived organoid; "Orgs" = organoids; "A", "B", and "C" = specific organoid types; "IM-CoF", "IM-SIF" = specific immortalized fibroblast lines; "Clone2", "SIF" = specific primary fibroblast lines. See also **Table 1.**

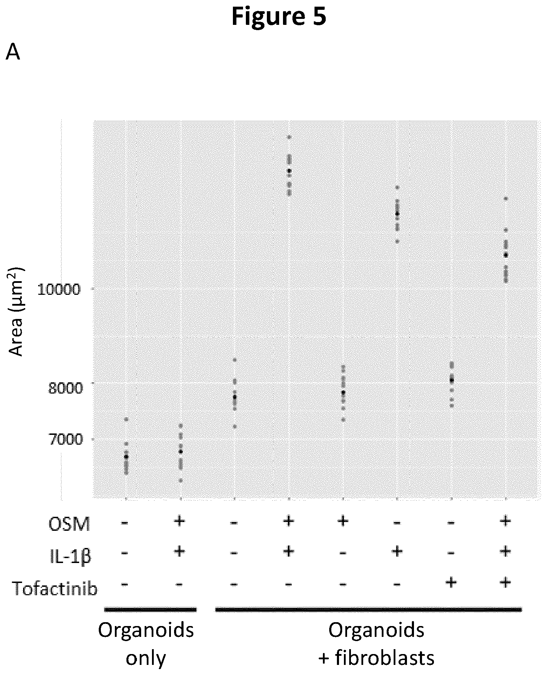

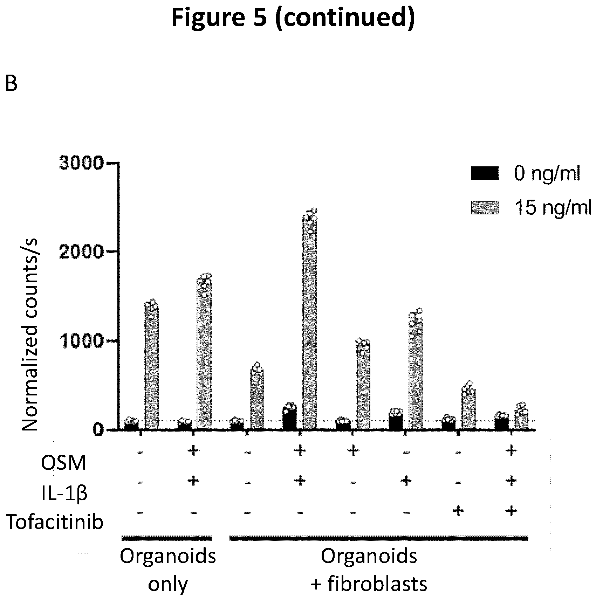

**Figure** 5 shows **(A)** organoid area measurements and **(B)** caspase activity measurements, and how these are affected by proinflammatory stimulation and/or tofacitinib administration, as described in **Example 7.**

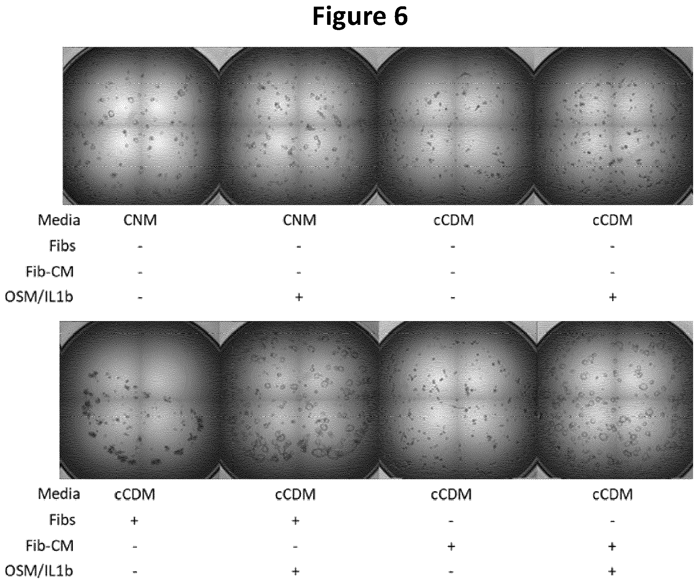

**Figure 6** shows imaging of organoids with or without fibroblasts ("Fibs"), fibroblast conditioned medium ("Fib-CM"), proinflammatory stimuli ("OSM/IL1b"), and when cultured in either expansion medium ("CNM") or differentiation medium ("cCDM"), as described in **Example 8**.

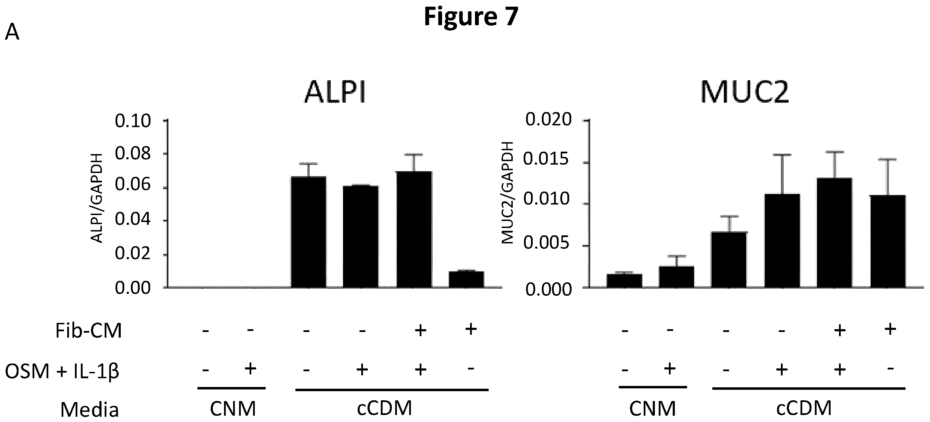

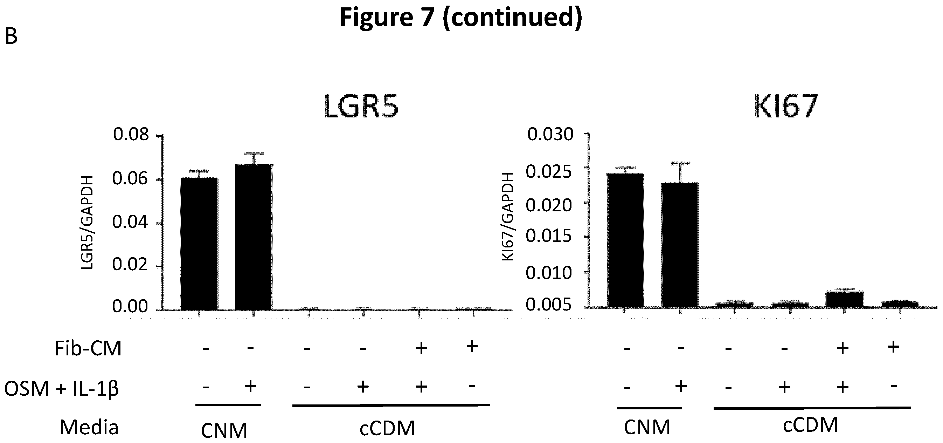

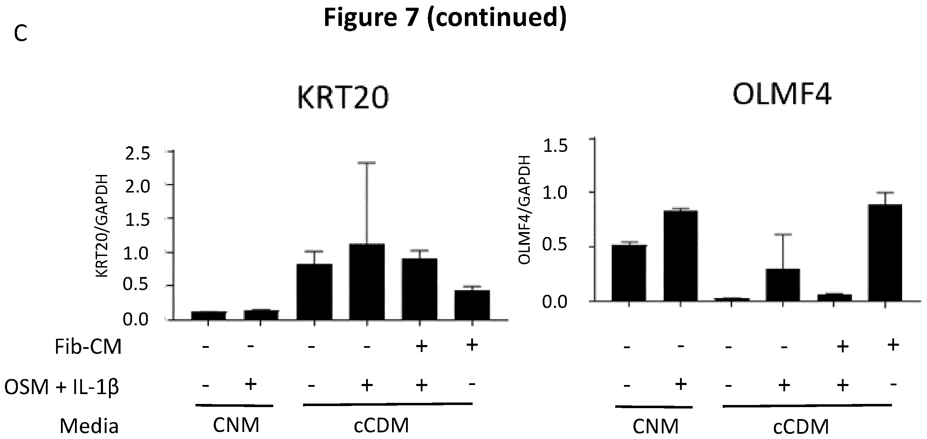

**Figure 7** shows gene expression measurements of co-cultures, with or without fibroblast conditioned medium ("Fib-CM"), proinflammatory stimuli ("OSM + IL1β"), and when cultured in either expansion medium ("CNM") or differentiation medium ("cCDM"), as described in **Example 9.** Shown are expression of **(A)** ALPI and MUC2, **(B)** LGR5 and KI67, and **(C)** KRT20 and OLMF4.

### DETAILED DESCRIPTION OF INVENTION

### Definitions

The practice of the present invention will employ, unless otherwise indicated, conventional methods of chemistry, biochemistry, molecular biology, immunology and pharmacology, within the skill of the art. Such techniques are explained fully in the literature. See, *e.g*., references [1-7], *etc.*

**"Approximately"** or **"about",** as used in this application, are equivalent. Any numerals used in this application with or without about/approximately are meant to cover any normal fluctuations appreciated by the person skilled in the art. As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value). A value can also be read as the exact value and so the term "about" can be omitted. For example, the term "about 100" encompasses 90-110 and also 100.

**"Co-culture"** refers to two or more cell types maintained in conditions suitable for their mutual growth. In the context of the present disclosure, an "organoid co-culture" relates to an epithelial organoid, as defined elsewhere, in culture with a non-epithelial cell type, specifically a stromal cell type. In some embodiments, cell types in co-culture are integrated, in that they may exhibit a structural, biochemical and/or phenomenological association that they do not exhibit in isolation. In some embodiments, cell types in co-culture mimic the structural, biochemical and/or phenomenological association observed between the cell types *in vivo.* In the present application, the term "co-culture" may be used to refer to normal (e.g. non-fibrotic, non-inflammatory) co-cultures, or disease co-cultures. Where co-cultures are described as "disease" co-cultures, this means that the co-culture has a disease phenotype, e.g. typically because the co-culture has been derived from one or more epithelial cells or one or more non-epithelial cells having a disease phenotype, or in some embodiments, because the organoid has been exposed to one or more proinflammatory stimuli to induce characteristics of a disease phenotype.

**"To comprise"** and its conjugations are used in their non-limiting sense to mean that items following the word are included, but items not specifically mentioned are not excluded. In addition the verb "to consist" may be replaced, if necessary, by "to consist essentially of' meaning that a product as defined herein may comprise additional component(s) than the ones specifically identified, said additional component(s) not altering the unique characteristic of the invention. In addition a method as defined herein may comprise additional step(s) than the ones specifically identified, said additional step(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one".

**"Fibroblast"** refers to the principal active cell of connective tissue, characterized by secretion of collagen and other extracellular matrix proteins. Fibroblasts are one of the most abundant cell types in stroma. A fibroblast is a type of stromal cell.

**"Fibrosis"** or **"fibrotic disease"** refers to a disorder, or to symptoms of a disorder, characterized by excessive, uncontrolled, and/or inappropriate deposition of collagen and other extracellular matrix components. Examples of fibrotic diseases include intestinal fibrosis, solitary rectal ulcers, radiation enteropathy, and eosinophilic enteropathy.

The term **"digestive system"** encompasses the gastrointestinal tract and the liver, pancreas and gallbladder.

The term **"gastrointestinal tract"** or **"GI tract"** encompasses the mouth, oral cavity, esophagus, stomach, intestine, rectum and anus.

**"Inflammation"** refers to a collection of biochemical stimuli and cellular responses that lead to physiological disorders traditionally characterized clinically by heat, pain, redness, and swelling, at the site of inflammation. At the biochemical level, inflammation may be mediated by proinflammatory stimuli and inflammatory stimuli. **"Inflammatory stimuli"** are agents that trigger an **"inflammatory response"** in the cells and/or co-cultures on which they act, increasing caspase activity (among other types of cellular damage), and ultimately increasing cell death (apoptosis). Examples of inflammatory stimuli include cytokines, such as TNF and IFNγ. Inflammatory stimuli therefore include "damage-inducing cytokines". **"Proinflammatory stimuli"** are agents that do not directly increase apoptosis, but cause the cells and/or co-cultures on which they act to adopt a **"proinflammatory profile"** in which they exhibit altered gene expression (such as upregulated PDPN and/or TGF-β in the case of stromal cells), an altered secretome (including increased secretion of IL-6 and/or CXCL2), increased organoid size and/or altered aggregation, and/or higher sensitivity to inflammatory stimuli, cytokine-mediated damage, and/or increased caspase activity. Examples of proinflammatory stimuli include cytokines, such as oncostatin M (OSM) and IL-1β. Proinflammatory stimuli typically signal through molecular pathways including STAT, NF-κB, MAPK, JUNK, mTOR, NFAT, and PI3K-AKT, but may be distinguished from inflammatory stimuli by not directly inducing increased apoptosis *per se.*

**"Inflammatory disorder"** refers to a disease in which inflammation is a symptom or cause. An example of an inflammatory disorder includes intestinal bowel disease. Herein, the terms "disease", "disorder", and "condition" are used interchangeably.

**"Inflammatory bowel disease" ("IBD")** refers to conditions characterized by chronic inflammation of the gastrointestinal ("GI") tract. Symptoms may include abdominal pain, diarrhea, weight loss, and bleeding in the GI tract. **"Ulcerative colitis" ("UC")** and **"Crohn's disease" ("CD")** are examples of IBD conditions. UC may present as constant inflammation limited to the colon. CD may present as discontinuous inflammation affecting the entire GI tract.

The term **"intestine"** encompasses colon and small intestine. The intestine is an epithelial tissue, and part of the GI tract and digestive system. Accordingly, the skilled person would appreciate that the methods and uses described herein can be applied to other epithelial tissues, particularly other epithelial tissues from the digestive system.

**"Organoid"** refers to a cellular structure obtained by expansion of adult (post-embryonic) epithelial stem cells, preferably characterized by Lgr5 expression, and consisting of tissue-specific cell types that self-organize through cell sorting and spatially restricted lineage commitment (e.g. as described in [8], see particularly section called "Organoids derived from adult stem cells" at page 1590 onwards). Organoids may be "patient-derived organoids" (PDOs). In the present application, the term "organoid" may be used to refer to normal (e.g. non-fibrotic, non-inflammatory) organoids, or disease organoids. Where organoids are described as "disease" organoids, this means that the organoid has a disease phenotype, e.g. typically because the organoid has been derived from one or more epithelial stem cells having a disease phenotype, or in some embodiments, because the organoid has been genetically modified to display particular characteristics of a disease phenotype.

**"Safe"** or **"tolerable"** refers to a treatment for a disease, which has no side-effects or only has side-effects within a tolerable level according to standard clinical practice.

**"Side effect"** or **"deleterious effect"** refers to a physiological response attributable to a treatment other than desired effects.

The term **"small intestine"** encompasses duodenum, jejunum, and ileum.

**"Stroma"** (sometimes referred to as **"mesenchyma"** or **"mesenchyme")** refers to the supportive structure associated with an epithelial organ, gland, or tumour, comprising connective tissue. Stroma is distinct from parenchyma, which refers to the part of an organ that performs organ-specific functions. **"Stromal cell"** refers to cells that are found in, or that produce, the stroma, such as fibroblasts. Stroma may refer to the digestive stroma (particularly the gastrointestinal stroma, such as the intestinal stroma, for example the colon stroma), renal stroma, or pulmonary stroma.

**"Subject"** or **"patient"** or **"individual"** are used herein interchangeably and may refer to a human or any non-human animal (such as any mouse, rat, rabbit, dog, cat, cattle, swine, sheep, horse or primate). In preferred embodiments, a subject is a mammal, more preferably a human. "Human" may refer to pre- and/or post-natal forms. A subject may be a human without disease. A subject may be human presenting to a medical provider for diagnosis or treatment of a disease. A subject can be afflicted with or be susceptible to a disease or disorder but may or may not display symptoms of the disease or disorder.

**"Suffering from"** refers to a subject who has been diagnosed with or displays one or more symptoms of a disease, disorder, and/or condition.

**"Susceptible to"** refers to a subject who has not been diagnosed with a disease, disorder, and/or condition. In some embodiments, a subject who is susceptible to a disease, disorder, and/or condition may not exhibit symptoms of the disease, disorder, and/or condition. In some embodiments, a subject who is susceptible to a disease, disorder, condition, or event may be characterized by one or more of the following: (1) a genetic mutation associated with development of the disease, disorder, and/or condition; (2) a genetic polymorphism associated with development of the disease, disorder, and/or condition; (3) increased and/or decreased expression and/or activity of a protein associated with the disease, disorder, and/or condition; (4) habits and/or lifestyles associated with development of the disease, disorder, condition, and/or (5) having undergone, planning to undergo, or requiring a transplant. In some embodiments, a subject who is susceptible to a disease, disorder, and/or condition will develop the disease, disorder, and/or condition. In some embodiments, a patient who is susceptible to a disease, disorder, and/or condition will not develop the disease, disorder, and/or condition.

**"Therapeutically effective amount"** refers to an amount of a therapeutic agent that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by the skilled person that a therapeutically effective amount is typically administered via a dosing regimen comprising at least one unit dose.

**"Treating", "treat", "treatment"** refers to any method used to partially or completely alleviate, ameliorate, relieve, inhibit, prevent, delay onset of, reduce severity of and/or reduce incidence of one or more symptoms or features of a particular disease, disorder, and/or condition. Treatment may be administered to a subject who does not exhibit signs of a disease and/or exhibits only early signs of the disease for the purpose of decreasing the risk of developing pathology associated with the disease. Any reference to a method for treatment comprising administering an agent to a subject, also covers that agent for use in said method for treatment, as well as the use of the agent in said method for treatment, and the use of the agent in the manufacture of a medicament.

### General

The invention relates to co-cultures (or "organoid co-cultures") formed by combining at least one organoid and at least one stromal cell, methods of preparing these (sometimes referred to as "co-culturing"), and their uses in *in vitro* methods, such as for testing therapeutic agents for disease, and/or diagnosing and/or prognosing disease. Of particular interest are uses of the co-culture in drug screening, toxicology screening, clinical research, and drug development.

The invention also provides a co-culture obtained by methods of the invention.

Without wishing to be bound by any theory, the inventors consider that co-culture of organoids and stromal cells as described herein recapitulates important *in vivo* interactions between the stroma and epithelium in states of health and disease, on biochemical levels (e.g. caspase activity, secretion of soluble mediators such as IL-6 and CXCL2), cellular levels (e.g. apoptosis), and physiological levels (e.g. increased organoid area and altered aggregation).

### Species

Cells (including epithelial cells and stromal cells), organoids and/or co-cultures of the invention or suitable for use with methods of the invention may be principally derived from any multicellular organism. In some embodiments, the cells, organoids and/or co-cultures of the invention are mammalian (meaning derived from mammals), such as murine, primate, non-human primate, rat, dog, minipig, or human, cells, organoids and/or co-cultures. In a preferred embodiment, the cells, organoids and/or co-cultures of the invention are human (meaning derived from humans).

### Samples

Co-cultures of the invention may comprise or consist of autologous cells, *i.e.* cells obtained from the same subjects. For example, the co-culture may be obtained by preparing epithelial cells (e.g. colorectal cells) derived from a tissue in a subjects, and by preparing stromal cells derived from the same subjects (optionally derived from the same tissue in the same subject), which are combined to form the co-culture. In some embodiments, the epithelial cells and the stromal cells may be derived from the same sample from the same subject. The sample may be a tissue biopsy, for example an intestinal tissue biopsy. The sample may be a healthy sample (e.g. not having an inflammatory disease or a fibrotic disease) or a disease sample (e.g. having an inflammatory disease or a fibrotic disease). In some embodiments, epithelial cells and stromal cells may be obtained from different samples from the same subject.

Alternatively, co-cultures of the invention may comprise or consist of non-autologous cells, *i.e.* cells obtained from different subjects. For example, the co-culture may be obtained by culturing epithelial cells (e.g. colorectal cells) derived from a tissue in a first subjects, and stromal cells derived from a second subject (optionally derived from a corresponding tissue in the second subject), which are combined to form the co-culture. The samples may be tissue biopsies, for example intestinal tissue biopsies. The samples may be healthy samples (e.g. not having an inflammatory disease or a fibrotic disease) or disease samples (e.g. having an inflammatory disease or a fibrotic disease). The different subjects may be antigenically matched, for example the different patients may be blood relatives, such as immediate blood relatives.

Epithelial cells suitable for preparing organoids may in principle be derived from any epithelial tissue. For example, epithelial cells may be derived from intestine, lung, kidney, pancreas, or liver. Particularly preferred are epithelial cells derived from the digestive system, such as from the gastrointestinal tract. Most preferred are epithelial cells derived from the intestine. In some embodiments, the epithelial cells are not derived from lung, for example, are not derived from lung, kidney, pancreas, or liver. A tissue from which the epithelial cells are derived may be healthy (e.g. not having an inflammatory disease or a fibrotic disease), or may be diseased (e.g. having an inflammatory disease or a fibrotic disease).

A stromal cell may in principle be derived from any stroma. For example, a stromal cell may be derived from lung, kidney, or intestine. Particularly preferred is a stromal cell derived from the digestive system, such as from the gastrointestinal tract. Most preferred are stromal cells derived from the intestine. In some embodiments, the stromal cell is not derived from lung, for example, is not derived from lung, kidney, pancreas, or liver. A tissue from which the stromal cell is derived may be healthy (e.g. non-inflammatory, non-fibrotic), or may be diseased (e.g. having an inflammatory disorder or fibrotic disorder).

Epithelial cells and/or stromal cells may be obtained during surgery from normal mucosa or diseased mucosa, for example taken from resected colon, rectum, small intestine and/or ileum of subjects with or without a disease.

### Stromal culture

The invention provides at least one stromal cell, which may be combined with at least one organoid to form a co-culture. The invention also provides at least one reference stromal cell, which may be combined with at least one organoid to form reference co-cultures. The invention also provides stromal cells that may be cultured to prepare the at least one stromal cell. All stromal cells such as these, and other stromal cells discussed herein, may collectively be referred to as "stromal cells of the invention" or "stromal cells", and statements made that refer to "stromal cells" may include any of these stromal cell types.

### Cell types

Characteristics and methods associated with the at least one stromal cell (to be combined with the at least one organoid to form a co-culture) are described herein. These characteristics and methods are generally applicable to the stromal cells that may be cultured to prepare the at least one stromal cell. Therefore, herein, characteristics, methods, and other disclosures apply to both the at least one stromal cell or the stromal cells from which the at least one stromal cell is derived, unless context indicates otherwise.

The at least one stromal cell (or the stromal cells from which the at least one stromal cell is derived) may be obtained from established cell lines available in the art (e.g. from ATCC or similar libraries of cell lines). Alternatively, the stromal cells may be purified from an impure sample from a subject. There are advantages associated with obtaining stromal cells from the same subject as the at least one organoid (or the epithelial cells from which the at least one organoid is derived), because the resulting co-culture is thereby most representative (and so a most faithful model) of the subject from which the cells are derived. This is particularly useful in the context of personalized medicine.

Stromal cells may be derived from the same tissue type, the same organ, and/or the same sample as the epithelial cells from which the at least one organoid is derived.

In some embodiments, the at least one stromal cell is not a T cell, peripheral blood mononuclear cell (PBMC), natural killer (NK) cell, dendritic cell (DC), B cell, macrophage, neutrophil, basophil, eosinophil, monocyte, granulocyte, phagocyte, or mast cell. In some embodiments, the at least one stromal cell is not a lymphocyte. In some embodiments, the at least one stromal cell is not a leukocyte. In some embodiments, the at least one stromal cell is not derived from lymphoid tissue. In some embodiments, the at least one stromal cell is not part of any cell lineage originating in lymphoid tissue. In some embodiments, the at least one stromal cell is not derived from hematopoietic tissue. In some embodiments, the at least one stromal cell is not part of any cell lineage originating in hematopoietic tissue.

A preferred stromal cell is a fibroblast cell (or "fibroblast"). The fibroblast may be an intestinal fibroblast (a fibroblast derived from the intestines), such as a colon fibroblast or a small intestine fibroblast.

The fibroblast cell may be an immortalized fibroblast (derived from an immortalized cell line), or a primary fibroblast (such as a patient-derived fibroblast).

Any of these types of stromal cell may be a human stromal cell, such as a human fibroblast.

### Stromal cell media

Stromal cell medium may be used to prepare at least one stromal cell for co-culturing, for example, by facilitating growth and division (expansion) and/or differentiation of stromal cells to produce at least one stromal cell suitable for co-culture.

Any medium suitable for culturing stromal cells may be used, for example a stromal cell medium comprising fetal calf serum that is suitable for culturing fibroblasts.

In some embodiments, stromal cell medium comprises a basal medium, such as Advanced DMEM/F-12 medium ("Ad-DF"), optionally about 500 ml Ad-DF.

In some embodiments, stromal cell medium comprises an amino acid supplement, such as L-glutamine or a substitute for L-glutamine, preferably a substitute for L-glutamine, such as GlutaMax, optionally about 2 mM GlutaMax.

In some embodiments, stromal cell medium comprises a media buffering agent, such as HEPES, optionally about 25 mM HEPES.

In some embodiments, stromal cell medium comprises one or more antibiotics, such as penicillin and/or streptomycin, optionally about 100 u/ml penicillin and/or streptomycin.

In some embodiments, stromal cell medium comprises serum, such as fetal calf serum (FCS), for example about 10% FCS.

In some embodiments, the stromal cell medium comprises a basal medium, a substitute for L-glutamine, an antibiotic, and optionally a media buffering agent and/or a serum.

In some embodiments, stromal cell medium comprises Ad-DF, GlutaMax, HEPES, and antibiotic (optionally penicillin and/or streptomycin), and optionally a serum (such as FCS). In some embodiments, stromal cell medium comprises about 500 ml Ad-DF, about 2 mM GlutaMax, about 25 mM HEPES, about 100 u/ml penicillin and/or streptomycin, and about 10% FCS.

### Preparing stromal cells

In some embodiments, combining the at least one organoid and at least one stromal cell to form a co-culture, comprises preparing the at least one stromal cell. This comprises culturing stromal cells in stromal cell medium.

Culturing stromal cells may comprise thawing stromal cells and adding the stromal cells to stromal cell medium. Culturing stromal cells may comprise incubating stromal cells, for example overnight.

Culturing stromal cells may comprise aspirating the stromal cell medium and trypsinising stromal cells, followed by re-suspension of the stromal cells in stromal cell medium.

In some embodiments, culturing stromal cells comprises culturing the stromal cells in a vessel (such as a cell culture dish) coated with poly-L-lysine.

In some embodiments, culturing stromal cells comprises passaging the stromal cells before they reach a maximum threshold confluency, and seeding them at above a minimum threshold confluency. Maximum and minimum threshold confluency values may be determined empirically.

### Monolayers

In some embodiments, the at least one stromal cell is prepared as a monolayer. Monolayer culture generally involves a method comprising: digesting or dissociating at least one stromal cell into a suspension of single cells; seeding a semi-permeable membrane with said suspension; and culturing the cells in the presence of an expansion medium until a monolayer is formed. Specific methods are disclosed in WO2023/281122, which is incorporated by reference in its entirety.

### Organoid culture

The invention provides at least one organoid, which may be combined with at least one stromal cell to form a co-culture. The invention also provides references organoids, which may be combined with at least one stromal cell to form a reference co-culture. Organoids such as these, and other organoids discussed herein, may collectively be referred to as "organoids of the invention" or "organoids", and statements made that refer to "organoids" may include the at least one organoid that can be combined with at least one stromal cell to form a co-culture, and to any reference organoid.

### Preparing organoids

Organoids may be prepared by culturing epithelial cells in an organoid medium, as described herein. Organoids of the invention may be characterized by Lgr5 expression.

Culturing epithelial cells may comprise obtaining epithelial cells from a sample, for example a tissue biopsy.

Culturing epithelial cells may comprise liberating crypts for derivation of organoid, for example comprising mechanical shearing of tissue and resuspending crypts in organoid medium.

In some embodiments, organoids of the invention are disrupted (split) into organoid fragments, for example in a 1:1 ratio, followed by passaging. Splitting may occur by mechanical force (termed "shearing"), or enzymatically (by e.g. TrypLE).

### Organoid cell types

Organoids and/or organoid co-cultures of the invention may be obtained from epithelial cells, and so may be described as "epithelial organoids". Any epithelial cell from which an organoid can be generated is suitable for use in the invention. Preferred epithelial cells include intestinal cells, crypt cells, rectal cells, lung cells, liver cells, breast cells, skin cells, pancreatic cells, endocrine cells, exocrine cells, ductal cells, renal cells, adrenal cells, thyroid cells, pituitary cells, parathyroid cells, prostate cells, stomach cells, oesophageal cells, ovary cells, fallopian tube cells and vaginal cells. Particularly preferred epithelial cells are intestinal cells, for example colorectal cells. The epithelial cells may be epithelial stem cells, preferably characterised by Lgr5 expression.

Suitable samples for obtaining epithelial cells include tissue biopsy, such as tissue biopsy from resected colon and/or rectum for colorectal epithelial cells; or urine for kidney epithelial cells.

An organoid of the invention can be a gastrointestinal organoid, a lung organoid, a pancreatic organoid, a trachea organoid, a skin organoid or a vaginal organoid - preferably a gastrointestinal organoid.

Organoids and/or organoid co-cultures may be obtained from normal epithelial cells or from disease epithelial cells (sometimes specifically referred to as `disease organoids' or 'disease co-cultures'). In some embodiments, the epithelial cells are obtained from a sample from a subject with an inflammatory disease. In a particular embodiment, disease epithelial cells and normal epithelial cells are obtained from samples from the same subject, optionally from the same sample.

Organoids can be obtained by culturing epithelial stem cells. The epithelial stem cells may be obtained from adult tissue, i.e. the epithelial stem cells are adult epithelial stem cells. In this context "adult" means mature tissue, i.e. includes newly-born baby or child but excludes embryonic or foetal. Alternatively, the epithelial stem cells are not derived from embryonic stem cells or embryonic stem cell lines, e.g. which have been differentiated *in vitro.* The adult tissue may be healthy adult tissue. In other embodiments, the adult tissue may be from a patient with a disease, such as an inflammatory disease.

The epithelial stem cell may be derived from colorectal, small intestine, lung, stomach, pancreas, liver, breast, prostate, kidney, mouth, nasopharynx, throat, hypopharynx, larynx, trachea, skin, fallopian tube, ovary, salivary gland, esophagus, hair follicle and/or cochlear tissue. In some embodiments the epithelial stem cells are colorectal cells. Methods for culturing epithelial stem cells to obtain organoids from a variety of epithelial tissues have previously been described (e.g. in WO2009/022907, WO2010/090513, WO2012/014076, WO2012/168930, WO2015/173425, WO2016/083613, and WO2016/083612, WO2017/149025, WO2020/234250, and [8]). Cells taken directly from tissue, i.e. freshly isolated cells, are also referred to as "primary cells". In some embodiments the epithelial stem cells are primary epithelial stem cells. In some embodiments, the primary epithelial cells are patient-derived epithelial cells.

In some embodiments, the organoid comprises only epithelial cells, i.e. non-epithelial cells are absent from the organoid. Even if other cell types are transiently present in the medium, e.g. in the tissue fragment that is the starting material, these cells are unlikely to survive and instead will be replaced by the longer term expansion of the stem cells which generate a pure population of epithelial cells.

An organoid obtained using a medium suitable for expansion (an "expansion medium") may be referred to as an "expansion organoid". An expansion organoid comprises at least one epithelial stem cell, which can divide and produce further epithelial stem cells or can generate differentiated progeny. It is to be understood that in a preferred expansion organoid, the majority of cells are expanding cells (i.e. dividing cells) that retain an undifferentiated phenotype. Although some spontaneous differentiation may occur, the cell population is generally an expanding population. The length of time that the organoids can continue to be expand whilst maintaining a core presence of epithelial stem cells and whilst maintaining genotypic and phenotypic integrity of the cells, is an important feature of the organoids. Organoids typically express Lgr5. The organoids also have a distinctive structure that arises rapidly as the cells expand and self-organise *in vitro.* These features are described in detail herein.

In some embodiments, organoids and/or co-cultures of the invention, e.g. derived from intestinal organoids, comprise one or more of the following cell types: Lgr5+ stem cell, enterocyte, goblet cell, Paneth cell and enteroendocrine cell.

Organoids of the invention may comprise, or be derived from, organoid fragments. Organoid fragments include any fragment from an organoid, for example an organoid-derived intestinal crypt. In some embodiments, organoid fragments are cell clumps, preferably consisting of less than 10, less than 5, preferably 2-4 cells. In preferred embodiments, the one or more organoids are digested or dissociated into a suspension comprising single cells and cell clumps.

### Organoid structure

In some embodiments, an organoid is a three-dimensional cellular structure. In some embodiments, an organoid comprises a lumen surrounded by epithelial cells. In some embodiments, the epithelial cells surrounding the lumen are polarized. The polarization may be disrupted in disease organoids. The epithelial cells from which organoids are obtained are preferably primary epithelial cells, such as patient-derived epithelial cells. Preferably, the adult epithelial stem cells are not derived from induced pluripotent stem (iPS) cells.

In some embodiments, the epithelial cells in the organoid surround a lumen. In some embodiments, the organoid does not comprise a lumen (in particular, disease organoids may not have a lumen). In some embodiments, the epithelial cells are polarized, meaning that proteins are differentially expressed on the apical or basolateral side of the epithelial cell. In some embodiments the lumen is a sealed lumen (meaning that a continuous cellular barrier separates the contents of the lumen from the medium surrounding the organoid). In some embodiments the organoids comprise stem cells which are able to actively divide and which are preferably able to differentiate to all major differentiated cell lineages present in the corresponding *in vivo* tissue, e.g. when the organoid or cell is transferred to a differentiation medium. In some embodiments, the organoid comprises basal cells on the outside and more differentiated cells in the centre.

In terms of the structure of intestinal epithelial cells, the apical surface faces the lumen and regulates interactions with lumenal contents, including mediating nutrient absorption, detects microbial products, and secretes molecules that protect the epithelium from potentially harmful agents in the lumen. The basolateral surface anchors epithelial cells to the underlying basement membrane, delivers nutrients from the lumen to the bloodstream, and communicates with nearby cells [9].

In some embodiments, the organoids comprise stratified epithelium. By "stratified" it is meant that there are multiple (more than one) layers of cells. Such cells often tend to have their nuclei more central to the cells, i.e. not polarized. The cells in the multilayer section may organise themselves to include a gap, or lumen between the cells.

In some embodiments, an organoid is at least about 50 µm, at least about 60 µm, at least about 70 µm, at least about 80 µm, at least about 90 µm, at least about 100 µm, at least about 125 µm, at least about 150 µm, at least about 175 µm, at least about 200 µm, at least about 250 µm or more in diameter at the widest point.

In some embodiments, an organoid is about 50 µm, about 60 µm, about 70 µm, about 80 µm, about 90 µm, about 100 µm, about 125 µm, about 150 µm, about 175 µm, about 200 µm, about 250 µm or more in diameter at the widest point.

In some embodiments, an organoid is at most about 50 µm, at most about 60 µm, at most about 70 µm, at most about 80 µm, at most about 90 µm, at most about 100 µm, at most about 125 µm, at most about 150 µm, at most about 175 µm, at most about 200 µm, at most about 250 µm or more in diameter at the widest point.

Within the context of the invention, a tissue fragment is a part of an adult tissue, preferably a human adult tissue. An organoid, by contrast, develops structural features through *in vitro* expansion, and is therefore distinguished from a tissue fragment.

### Organoid medium

Organoid medium may be used to prepare organoids for co-culture, for example, by facilitating growth, division (expansion), structural organization, or other development of epithelial cells to produce an organoid suitable for co-culture. The organoid medium may be particularly suitable for tissues derived from the digestive system, for example gastrointestinal tissues such as the intestine or colorectal tissues. Organoid medium may also be referred to as "expansion medium", "organoid expansion medium", or "colon normal medium" ("CNM").

The medium may comprise a basal medium. The basal medium is any suitable basal medium for animal or human cells, subject to any limitations provided herein, such as complete advanced DMEM/F 12 medium.

The medium may comprise a Wnt agonist, such as (a) WNT conditioned medium optionally at about 50% final volume, or (b) about 0.5 nM NGS Wnt. Particularly preferred is Wnt conditioned medium, such as Wnt3a conditioned medium (optionally at about 10% final volume).

The medium may comprise an R-spondin, for example (a) R-spondin-1 conditioned medium, optionally at about 20% final volume, or (b) Rspo3 at about 250 ng/ml.

The medium may comprise a BMP inhibitor, such as noggin, for example (a) noggin conditioned medium ("NCM"), optionally at about 1-4% final volume, or (b) recombinant noggin, optionally at a concentration of about 100 ng/ml. Particularly preferred is noggin conditioned medium ("NCM"), optionally at about 1-4% final volume.

The medium may comprise B27, optionally about 1x B27 according to manufacturer's instructions.

The medium may comprise N-acetylcysteine ("N-Ac"), for example at about 1.25 mM.

The medium may comprise nicotinamide, for example at about 10 mM.

The medium may comprise a mitogenic growth factor, such as EGF, optionally at about 50 ng/mL.

The medium may comprise gastrin, optionally at about 10 mM or about 5 mM.

The medium may comprise a TGF-β inhibitor, such as A-83-01, optionally at about 500 nM.

The medium may comprise a p38 MAPK inhibitor, such as SB202190, optionally at about 3 µM or about 10 µM.

The medium may comprise a prostaglandin pathway activator, which may be a prostaglandin, such as prostaglandine E2, optionally at about 10 nM.

The medium may comprise antibiotic, such as primocin, optionally at about 100 mg/mL or about 50 µg/ml.

Thus, the medium may comprise a mitogenic growth factor, a BMP inhibitor, and an R-spondin. In some embodiments, the medium comprises EGF, noggin, and an R-spondin. In some embodiments, the medium comprises about 50 ng/mL EGF, noggin conditioned medium at about 1-4% final volume, and about R-spondin-1 conditioned medium at about 20% final volume. Such medium may comprise a basal medium.

The medium may comprise a Wnt agonist, a mitogenic growth factor, a BMP inhibitor, and an R-spondin. In some embodiments, the medium comprises a Wnt agonist, EGF, noggin, and an R-spondin. In some embodiments, the medium comprises WNT3A conditioned medium at about 50% final volume, about 50 ng/mL EGF, noggin conditioned medium at about 1-4% final volume, and R-spondin-1 conditioned medium at about 20% final volume. Such medium may comprise a basal medium.

The medium may comprise a Wnt agonist, a mitogenic growth factor, a BMP inhibitor, and an R-spondin. In some embodiments, the medium comprises a Wnt agonist, EGF, noggin, and an R-spondin. In some embodiments, the medium comprises WNT3A conditioned medium at about 50% final volume, about 50 ng/mL EGF, noggin conditioned medium at about 1-4% final volume, and R-spondin-1 conditioned medium at about 20% final volume. Such medium may comprise a basal medium.

The medium may comprise a Wnt agonist, a mitogenic growth factor, a BMP inhibitor, an R-spondin, and a TGF-β inhibitor. In some embodiments, the medium comprises a Wnt agonist, EGF, noggin, an R-spondin, and A-83-01. In some embodiments, the medium comprises WNT3A conditioned medium at about 50% final volume, about 50 ng/mL EGF, noggin conditioned medium at about 1-4% final volume, R-spondin-1 conditioned medium at about 20% final volume, and about 500 nM A-83-01. Such medium may comprise a basal medium.

The medium may comprise a Wnt agonist, a mitogenic growth factor, a BMP inhibitor, an R-spondin, and a p38 MAPK inhibitor. In some embodiments, the medium comprises a Wnt agonist, EGF, noggin, an R-spondin, and SB202190. In some embodiments, the medium comprises WNT3A conditioned medium at about 50% final volume, about 50 ng/mL EGF, noggin conditioned medium at about 1-4% final volume, R-spondin-1 conditioned medium at about 20% final volume, and about 3 µM SB202190. Such medium may comprise a basal medium.

The medium may comprise a Wnt agonist, a mitogenic growth factor, a BMP inhibitor, an R-spondin, a TGF-β inhibitor, and a p38 MAPK inhibitor. In some embodiments, the medium comprises a Wnt agonist, EGF, noggin, an R-spondin, A-83-01, and SB202190. In some embodiments, the medium comprises WNT3A conditioned medium at about 50% final volume, about 50 ng/mL EGF, noggin conditioned medium at about 1-4% final volume, R-spondin-1 conditioned medium at about 20% final volume, about 500 nM A-83-01, and about 3 µM SB202190. Such medium may comprise a basal medium.

B27 and N-acetylcysteine are optional components of the medium. Thus, the medium may comprise a basal medium, a Wnt agonist, an R-spondin, noggin, B27, N-acetylcysteine, nicotinamide, a mitogenic growth factor, gastrin, a TGF-β inhibitor a p38 MAPK inhibitor, a prostaglandin pathway activator, and primocin. In some embodiments, the basal medium is complete advanced DMEM/F12 medium, the Wnt agonist is WNT3A conditioned medium, the R-spondin is R-spondin-1 conditioned medium, the noggin is noggin conditioned medium, the mitogenic growth factor is EGF, the TGF-β inhibitor is A-83-01, the p38 MAPK inhibitor is SB202190, and the prostaglandin pathway activator is prostaglandine E2.

The medium may comprise WNT3A conditioned medium at about 50% final volume, R-spondin-1 conditioned medium at about 20% final volume, noggin conditioned medium at about 1-4% final volume, B27 at about 1x according to manufacturer's instructions, N-acetylcysteine at about 1.25 mM, nicotinamide at about 10 mM, EGF at about 50 ng/mL, gastrin at about 10 mM, A-83-01 at about 500 nM, SB202190 at about 3 µM, prostaglandine E2 at about 10 nM, and primocin at about 100 mg/mL.

More generally, the skilled person will recognize that some components of the organoid medium (such as B27 and N-acetylcysteine) are optional, and/or may be substituted for suitable replacement components. Suitable organoid media for different tissues are known in the art (e.g. [8]). The media may be any suitable expansion medium for epithelial stem or progenitor cells, preferably a suitable expansion medium for epithelial stem cells. Culture media suitable for culturing organoids are also described in WO2009/022907, WO2010/090513, WO2012/014076, WO2012/168930, WO2015/173425, WO2016/083613, WO2016/083612, WO2017/149025 and WO2020/234250. The culture media mentioned in these documents are incorporated herein by reference and any of these may be used in the context of the invention.

In some embodiments, the medium comprises a receptor tyrosine kinase ligand, a BMP inhibitor and a Wnt agonist.

For example, in some embodiments, the medium comprises EGF, Noggin and Wnt-conditioned medium. In some embodiments, the medium comprises EGF, Noggin, Rspondin and Wnt surrogate.

In some embodiments, the medium further comprises nicotinamide and a p38 inhibitor, such as SB202190. In some embodiments, the medium further comprises a TGF-beta inhibitor.

For example, preferred organoid media comprise a Wnt agonist (e.g. any one of R-spondin 1-4), a mitogenic growth factor (e.g. selected from EGF, FGF, HGF and BDNF) and a BMP inhibitor (e.g. Noggin) (e.g. as described in WO2010/090513). In some embodiments, the organoid medium further comprises a TGF-beta inhibitor (e.g. A83-01, Tocris) (e.g. as described in WO2012/168930). The addition of a TGF-beta inhibitor is particularly suitable for the culture of human cells. The TGF-beta inhibitor preferably inhibits the ALK4/5/7 signalling pathway.

A preferred organoid medium, which is particularly suitable for culture of intestine or colon organoids, comprises one or more (or preferably all) of a basal medium (such as Advanced DMEM/F12 medium, Gibco) a Wnt ligand (such as Wnt-3a), a Wnt agonist (such as any one of Rspondin 1-4), a BMP inhibitor (such as Noggin), EGF, and a TGF-β inhibitor (such as A83-01, Tocris), and optionally further comprises one or more (or all) of a p38 MAPK inhibitor, gastrin, nicotinamide, prostaglandine E2, N-acetylcysteine, B27 and/or an antimicrobial (such as primocin).

### Specific organoid media

In a preferred embodiment, the organoid medium comprises (i) EGF (e.g. at a concentration of about 50 ng/ml); (ii) Noggin (e.g. at a concentration of about 100 ng/ml); (iii) Rspondin (e.g. at a concentration of about 250 ng/mL); (iv) Wnt surrogate (e.g. NGS-Wnt at a concentration of about 0.5 nM); (v) a p38 inhibitor (e.g. SB-203580 at a concentration of about 10 µM); (vi) a TGF-beta inhibitor (e.g. A83-01 at a concentration of about 500 nM); and (vii) nicotinamide (e.g. at a concentration of about 10 mM).

In another preferred embodiment, the organoid medium comprises (i) EGF (e.g. at a concentration of about 50 ng/ml); (ii) Noggin (e.g. at a concentration of about 100 ng/ml); (iii) Wnt-conditioned medium (e.g. at about 50% final volume); (iv) a p38 inhibitor (e.g. SB-203580 at a concentration of about 10 µM); (v) a TGF-beta inhibitor (e.g. A83-01 at a concentration of about 500 nM); and (vi) nicotinamide (e.g. at a concentration of about 10 mM).

In some embodiments, particularly where the organoids are derived from the lung, the organoid medium comprises one or more receptor tyrosine ligands, a Wnt agonist, a TGF-beta inhibitor and a BMP inhibitor. In some embodiments, the organoid medium comprises FGF, Rspondin, a TGF-beta inhibitor, a BMP inhibitor, a Rho-kinase inhibitor and a p38 inhibitor. In a preferred embodiment, the organoid medium comprises i) FGF (e.g. FGF-7 at a concentration of about 25 ng/ml and FGF-10 at a concentration of about 100 ng/mL); (ii) Rspondin (e.g. Rspondin-3 at a concentration of about 250 ng/mL); (iii) a TGF-beta inhibitor (e.g. A83-01 at a concentration of about 500 nM); (iv) a BMP inhibitor (e.g. Noggin-Fc Fusion Protein conditioned medium at about 2% final volume), (v) a Rho-kinase inhibitor (e.g. Y-27632 at a concentration of about 10 µM), and (vi) a p38 kinase inhibitor (e.g. SB202190 at a concentration of about 500 nM).

In some embodiments, particularly where the organoids are derived from the kidney, the organoid medium comprises one or more receptor tyrosine ligands, a Wnt agonist, and a TGF-beta inhibitor. In some embodiments, the organoid medium comprises EGF, FGF, Rspondin, a TGF-beta inhibitor and a Rho-kinase inhibitor. In a preferred embodiment, the organoid medium comprises i) EGF (e.g. at a concentration of about 50 ng/ml); (ii) FGF (e.g. FGF-10 at a concentration of about 100 ng/ml); (iii) Rspondin (e.g. Rspo1-conditioned medium at about 10% final volume); (iv) a TGF-beta inhibitor (e.g. A83-01 at a concentration of about 500 nM); and (v) a Rho-kinase inhibitor (e.g. Y-27632 at a concentration of about 10 µM).

### Organoid culture duration

In some embodiments, epithelial cells are cultured in organoid medium for at least about 6 hours, at least about 12 hours, at least about 24 hours, at least about 48 hours, at least about 72 hours, at least about 4 days, at least about 5 days, at least about 6 days, or at least about 7 days, to prepare the at least one organoid. Preferably, the epithelial cells are cultured in organoid medium for at least about 24 hours to prepare the at least one organoid.

In some embodiments, the at least one organoid of the invention has been cultured or is capable of culture in organoid media for at least about 2 months, for example at least about 10 weeks, at least about 12 weeks, at least about 14 weeks, at least about 16 weeks, at least about 4 months, at least about 5 months, at least about 6 months, at least about 9 months, or at least about one year.

In some embodiments, the at least one organoid has been cultured or is capable of culture for at least about 5 passages, at least about 10 passages, at least about 15 passages, or at least about 20 passages, preferably for at least about 10 passages.

In some embodiments, the cell number of the organoid increases exponentially over about 5 passages, about 10 passages, about 15 passages, or about 20 passages, preferably over about 5 passages.

In a preferred embodiment, an organoid for use in the claimed methods could be cultured during at least 2, 3, 4, 5, 6, 7, 8, 9, 10 weeks or 1, 2, 3, 4, 5, 6, 7, 8, 9, 10 months or longer. In some embodiments, the organoid is expanded or maintained in culture for at least about 3 months, preferably at least about 4 months, at least about 5 months, at least about 6 months, at least about 7 months, at least about 9 months, or at least about 12 months or more.

### Monolayers

In some embodiments, the organoid is prepared as a monolayer. In some embodiments the organoids comprise single monolayers that are folded (or invaginated) to form two or more layers. It can sometimes be difficult to distinguish between folded (or invaginated) monolayers and regions of stratified cells. In some embodiments an organoid comprises both regions of stratified cells and regions of folded monolayers. In some embodiments the organoids have a section which is formed of multiple layers and a section comprising a single monolayer of cells. In some embodiments the organoids comprise or consist of a single monolayer of cells. In some embodiments, the organoid does not comprise a monolayer. The organoids may possess a layer of cells with at least one bud and a central lumen.

Monolayer culture of organoids generally involves method comprising: digesting or dissociating one or more organoids into a suspension of single cells and/or organoid fragments; seeding a semi-permeable membrane with said suspension; and culturing the cells and/or organoid fragments in the presence of a medium until a monolayer is formed. Specific methods are disclosed in WO2023/281122, which is incorporated by reference in its entirety.

In particular, incorporated by reference are the following examples from WO2023/281122, which are of particular relevance to intestinal organoids:
Example 1, which describes preparation of epithelial monolayers from human normal intestinal organoids (for instance, intestinal organoids of the present invention);
Example 2, describing human GI tract epithelium monolayer establishment, differentiation and characterization;
Example 3, describing development of an *in vitro* biological system to mimic components of IBD pathophysiology, with robust readouts for barrier function pathways (useable for example in the context of the present invention and IBD);
Example 4, describing validation of the robustness of a barrier function assays with intestinal organoid-derived monolayers;
Example 5, describing human GI tract organoid epithelium monolayer establishment; and
Example 6, describing polarisation of human GI tract organoid epithelium monolayers.

### Co-culture

The invention provides co-cultures as described herein, and uses co-cultures described herein in methods of the invention.

### Combining

Methods of the invention may comprise combining the at least one organoid with the at least one stromal cell in a co-culture medium to form the co-culture.

Methods of the invention, such as methods of determining the presence or absence of at least one change in a co-culture (including methods of testing one or more therapeutic agents, and methods of determining the presence or absence of a diagnosis and/or prognosis) may be performed on a co-culture that has already been prepared, that is, the step of combining the at least one organoid and at least one stromal cell need not be a mandatory step in all methods of the invention.

In a preferred embodiment, an organoid medium (optionally including any extracellular matrix, such as basement membrane matrix 'BME' or Matrigel) is removed from the at least one organoid before combining the at least one organoid with the at least one stromal cell. Removal may be performed using a protease, such as Dispase. Extracellular matrix may be disrupted using commercially available kits, such as Cell Recovery Solution^{™} (Corning). An alternative matrix, such as collagen, may be used in place of the removed matrix.

In some embodiments, the at least one organoid and at least one stromal cell are combined using a multidrop dispenser.

In some embodiments, the at least one stromal cell and at least one organoid are combined in equal ratios, i.e. one stromal cell is combined per one organoid ("1:1"). In some embodiments, the at least one stromal cell is combined with the at least one organoid at a ratio of about 0.5. In some embodiments, the at least one stromal cell is combined with the at least one organoid at a ratio of about 2. In some embodiments, the at least one stromal cell is combined with the at least one organoid at a ratio of about 2.5. A higher fibroblast-organoid ratio may lead to an increased proinflammatory profile (for example, increased secretion of IL6 and CXCL2), and/or an increased inflammatory response.

In some embodiments, the at least one stromal cell is combined with the at least one organoid at a ratio of at least about 0.5. In some embodiments, the at least one stromal cell is combined with the at least one organoid at a ratio of at least about 1:1. In some embodiments, the at least one stromal cell is combined with the at least one organoid at a ratio of at least about 2. In some embodiments, the at least one stromal cell is combined with the at least one organoid at a ratio of at least about 2.5.

In some embodiments, the at least one stromal cell is combined with the at least one organoid at a ratio of at most about 0.5. In some embodiments, the at least one stromal cell is combined with the at least one organoid at a ratio of at most about 1:1. In some embodiments, the at least one stromal cell is combined with the at least one organoid at a ratio of at most about 2. In some embodiments, the at least one stromal cell is combined with the at least one organoid at a ratio of at most about 2.5.

In some embodiments, combining the at least one organoid with the at least one stromal cell comprises seeding the at least one organoid and the at least one stromal cell in co-culture medium. In some embodiments, the at least one organoid and at least one stromal cell are seeded simultaneously.

In some embodiments, combining the at least one organoid with the at least one stromal cell comprises a hanging drop method.

In some embodiments, combining the at least one organoid with the at least one stromal cell comprises a multi-well plate format, optionally wherein the multi-well plate comprises an ultra-low attachment plate.

In some embodiments, the at least one organoid may be prepared from epithelial cells at the same time as combining the epithelial cells with the at least one stromal cell, such that co-culture and organoid formation occur together.

### Co-culture medium

The invention provides media (e.g. as described in the examples) for the co-culture of organoids and stromal cells. Co-culture medium may also be described as "differentiation medium", or "combined-colon differentiation medium" ("cCDM").

Co-culture media of the invention advantageously allow the co-culture of stromal cells and organoids. Such co-culture is difficult or even impossible without using the media adaptations employed in the co-culture media of the invention. The inventors have observed for the first time that stromal cell function is preserved in co-culture with organoids according to the invention.

For example, and without wishing to be bound by any theory, a co-culture medium was chosen to identify organoid responses, in the form of one or more changes in co-culture, to factors released by stromal cells. Stromal cells are known to secrete ligands supporting the presence of stem cells and therefore potentially preventing differentiation. The inventors theorized that such responses could be determined according to the different morphology of differentiated organoids (small lumen and thick epithelium) and undifferentiated organoids (large lumen and thin epithelium). The co-culture medium selected also contains an ERK inhibitor (PD0325901), which the inventors theorized would enhance the observability of sensitivity of the co-culture to cytokine damage. The methods of the invention reveal active communication between organoids and stroma. The inclusion of serum (such as fetal calf serum, "FCS", also known as fetal bovine serum, "FBS") in the co-culture medium, has surprisingly been found to support long-term culture of fibroblasts and organoids without disrupting organoid structure.

The co-culture medium may be particularly suitable for tissues derived from the digestive system, for example gastrointestinal tissues such as the intestine or colorectal tissues, as well as for stromal tissues.

The at least one organoid and at least one stromal cell may be combined in cCDM to form the co-culture.

In some embodiments, the medium comprises a basal medium. The basal medium is any suitable basal medium for animal or human cells, subject to any limitations provided herein, such as complete advanced DMEM/F12 medium.

In some embodiments, the medium comprises N-Acetylcysteine ("N-Ac"), optionally at about 1.25 mM.

In some embodiments, the medium comprises a TGF-β inhibitor, optionally A83-01, optionally at about 500 nM.

In some embodiments, the medium comprises B27, optionally at about 1x according to manufacturer's instructions.

In some embodiments, the medium comprises a mitogenic growth factor, such as EGF, optionally at about 50 ng/mL.

In some embodiments, the medium comprises gastrin, optionally at about 5 nM.

In some embodiments, the medium comprises a BMP inhibitor, such as noggin, for example (a) noggin conditioned medium ("NCM"), optionally at about 1-2% final volume, or (b) recombinant noggin, optionally at a concentration of about 100 ng/ml. Particularly preferred is noggin conditioned medium ("NCM"), optionally at about 1-2% final volume.

In some embodiments, the medium comprises an antibiotic, such as primocin, optionally at about 50 µg/mL.

In some embodiments, the medium comprises an R-spondin, such as Rspo3, optionally at about 250 ng/mL.

In some embodiments, the medium comprises a Notch pathway inhibitor. In some embodiments, the medium comprises DAPT, optionally at about 10 µM.

In some embodiments, the medium comprises an ERK inhibitor. In some embodiments, the medium comprises PD0325901, optionally at about 100 nM. Without wishing to be bound by any theory, the inventors believe that organoids co-cultured in medium comprising an ERK inhibitor like PD0325901 exhibit structure and lumen size that is even more reflective of interactions with stromal cells, and more prominently reveals the effects of inflammatory stimuli (such as TNF) on the co-culture.

In some embodiments, the medium comprises a Wnt agonist, such as (a) Wnt conditioned medium (optionally at about 10% final volume) or NGS Wnt (optionally at about 0.1 nM). Particularly preferred is Wnt conditioned medium, such as Wnt3a conditioned medium (optionally at about 10% final volume).

In some embodiments, the medium comprises a serum, such as fetal bovine serum (FBS), optionally at about 5% or about 10% final volume, preferably about 5% final volume.

In some embodiments, the medium comprises an ECM, such as Matrigel, optionally at about 5%.

B27 and N-acetylcysteine are optional components of the medium.

In some embodiments, the medium comprises N-Ac, a TGF-β inhibitor, B27, a mitogenic growth factor, gastrin, a BMP inhibitor, an antibiotic, an R-spondin, a Notch pathway inhibitor, an ERK inhibitor, and a Wnt agonist. In some embodiments, the TGF- inhibitor is A83-01, the mitogenic growth factor is EGF, the R-spondin is Rspo3, the BMP inhibitor is noggin conditioned medium, the antibiotic is primocin, the Notch pathway inhibitor is DAPT, the ERK inhibitor is PD0325901, and/or the Wnt agonist is Wnt conditioned medium or NGS Wnt. In some embodiments, the medium further comprises FBS and an ECM.

In some embodiments, the medium comprises N-Ac, A83-01, B27, EGF, gastrin, noggin conditioned medium, primocin, an R-spondin, DAPT, PD0325901, and a Wnt agonist. In some embodiments, the medium further comprises FBS and an ECM.

In some embodiments, the medium comprises N-Ac at 1.25 mM, A83-01 at 500 nM, about 1x B27 according to manufacturer's instructions, EGF at about 50 ng/mL, gastrin at about 5 nM, noggin conditioned medium at about 1-2%, primocin at about 50 µg/mL, Rspo3 at about 250 ng/mL, DAPT at about 10 µM, PD0325901 at about 100 nM, and Wnt3a conditioned medium at about 10% final volume or about 0.1 nM NGS Wnt. In some embodiments, the medium further comprises FBS at about 5% final volume and Matrigel at about 5% final volume.

Any of the stromal cell media or the organoid media described herein may be used to as a co-culture medium.

Organoid medium described herein may be used as co-culture medium by reducing the concentration of BMP inhibitor, such as noggin, optionally by reducing the concentration of noggin by about 50%, preferably by reducing noggin conditioned medium to about 1-2% final volume.

Organoid medium described herein may be used as co-culture medium by reducing the concentration of Wnt agonist, optionally by reducing the concentration of Wnt agonist by about 80%, preferably by reducing Wnt conditioned medium to about 10% final volume or reducing the concentration of NGS Wnt to about 0.1 nM. Particularly preferred is reducing Wnt conditioned medium, such as reducing Wnt3a conditioned medium (optionally to about 10% final volume).

Organoid medium described herein may be used as co-culture medium by adding a Notch pathway inhibitor to the medium, such as DAPT, for example by adding DAPT to a concentration of about 10 µM.

Organoid medium described herein may be used as co-culture medium by adding an ERK inhibitor to the medium, such as PD0325901, for example by adding PD0325901 to a concentration of about 100 nM.

Organoid medium described herein may be used as co-culture medium by adding serum to the medium, such as FCS, for example by adding FCS to about 5% final volume.

Organoid medium described herein may be used as co-culture medium by adding an ECM to the medium, such as Matrigel, for example by adding Matrigel to a concentration of about 5% final volume.

More generally, the skilled person will recognize that some components of the differentiation medium (such as B27 and N-acetylcysteine) are optional, and/or may be substituted for suitable replacement components. The differentiation medium may be any suitable differentiation medium for organoids and stromal cells, e.g. as described in WO2015/173425, WO2017/149025 and WO2017/220586.

In some embodiments, a differentiation medium suitable for co-culture can comprise one or more of a Wnt agonist, a BMP inhibitor, a mitogenic growth factor and a TGF-beta inhibitor. For example, the differentiation medium comprises a Wnt agonist. The differentiation medium can further comprises a mitogenic growth factor and/or a BMP inhibitor.

In preferred embodiments the differentiation medium comprises a Wnt agonist, a BMP inhibitor, a mitogenic growth factor and a TGF-beta inhibitor. The differentiation medium can further comprise one or more of a p38 inhibitor, a cAMP agonist, a prostaglandin pathway activator, nicotinamide, gastrin, B27 and N-acetylcysteine.

In some embodiments the differentiation medium comprises a basal medium for human or animal cells (such as DMEM/F12 optionally including B27 or Ad-DF+++ (advanced Dulbecco's modified Eagle's/F12 medium supplemented with GultaMax, 1 M HEPES) ), an R-spondin family protein, a mitogenic growth factor (such as EGF), a BMP inhibitor (such as noggin), a TGF-beta inhibitor (such as A83-01), a p38 inhibitor (such as SB202190) and optionally nicotinamide and N-acetylcysteine.

In some embodiments the differentiation medium comprises advanced DMEM/F12 medium including B27, nicotinamide, N-acetylcysteine, noggin, R-spondin 1-4, EGF, Wnt (either WNT conditioned medium (50%, produced using stably transfected L cells) or NGS Wnt), TGF-b type I receptor inhibitor A83-01 and P38 inhibitor SB202190.

The co-culture medium may comprise an extracellular matrix (ECM). Preferred ECMs include BME and Matrigel. These may constitute at least about 5%, at least about 6%, at least about 7%, at least about 8%, at least about 9% or at least about 10% (*v*/*v*) of the co-culture. Preferably, the ECM is Matrigel and constitutes from about 1% to about 10% (*v*/*v*) of the co-culture, for example from about 5% to about 10% (*v*/*v*) of the co-culture, preferably about 5% (*v*/*v*) of the co-culture. In some embodiments, the co-culture medium does not comprise an ECM.

In other embodiments, a co-culture medium may be described as "entryocyte colon differentiation medium" ("eCDM"). Such medium may comprise a PORCN inhibitor, such as iWP2, for example 1.5 µM iWP2.

eCDM may comprise a TGF-β inhibitor, a mitogenic growth factor, and a BMP inhibitor, for example the medium may comprise A-83-01, EGF, and noggin conditioned medium, for example 500 nM A-83-01, 50 ng/ml EGF, and 1-2% noggin conditioned medium.

The medium may further comprise an R-spondin, such as Rspo3 (e.g. 250 ng/ml Rspo3).

The medium may further comprise N-Acetylcysteine (e.g. 1.25 mM N-Acetylcysteine), B27 supplement (e.g. 1x B27 according to manufacturer's instructions), gastrin (e.g. 5 nM gastrin), and/or an antibiotic such as primocin (e.g. 50 µg/ml primocin).

The medium may further comprise a serum such as FBS (e.g. 5% FBS) and/or an ECM, such as Matrigel (e.g. 5% Matrigel).

The medium may further comprise a serum such as FBS (e.g. 5% FBS) and/or an ECM, such as Matrigel (e.g. 5% Matrigel).

Thus, in some embodiments, the co-culture medium comprises a TGF-β inhibitor, a mitogenic growth factor, a BMP inhibitor, an R-spondin, serum, and an ECM, and optionally one or more of N-Acetylcysteine, B27 supplement, gastrin, and an antibiotic.

In some embodiments, the co-culture medium comprises A-83-01, EGF, noggin conditioned medium, an R-spondin, serum, and an ECM, and optionally one or more of N-Acetylcysteine, B27 supplement, gastrin, and primocin.

In some embodiments, the co-culture medium comprises 500 nM A-83-01, 50 ng/ml EGF, 1-2% noggin conditioned medium, 250 ng/ml Rspo3, 5% FBS, and 5% Matrigel, and optionally 1.25 mM N-Acetylcysteine, 1x B27 supplement according to the manufacturer's instructions, 5 nM gastrin, and 50 µg/ml primocin.

### Co-culture duration

In some embodiments, the co-culture of the invention has been cultured or is capable of culture for at least about 2 months, for example at least about 10 weeks, at least about 12 weeks, at least about 14 weeks, at least about 16 weeks, at least about 4 months, at least about 5 months, at least about 6 months, at least about 9 months, at least about one year.

In some embodiments, the co-culture has been cultured or is capable of culture for at least about 5 passages, at least about 10 passages, at least about 15 passages, or at least about 20 passages, preferably for at least about 10 passages.

In some embodiments, the cell number of the co-culture increases exponentially over about 5 passages, about 10 passages, about 15 passages, or about 20 passages. preferably over about 5 passages.

In a preferred embodiment, the co-culture could be cultured during at least 2, 3, 4, 5, 6, 7, 8, 9, 10 weeks or 1, 2, 3, 4, 5, 6, 7, 8, 9, 10 months or longer. In some embodiments, the co-culture is expanded or maintained in culture for at least about 3 months, preferably at least about 4 months, at least about 5 months, at least about 6 months, at least about 7 months, at least about 9 months, or at least about 12 months or more.

### Monolayers

In some embodiments, the co-culture is prepared as a monolayer. Monolayer culture generally involves a method comprising: digesting or dissociating one or more organoids and stromal cells into a suspension of single cells and/or organoid fragments; seeding a semi-permeable membrane with said suspension; and culturing the cells and/or organoid fragments in the presence of a differentiation medium until a monolayer is formed. Specific methods are disclosed in WO2023/281122, which is incorporated by reference in its entirety.

### Components of organoid medium and co-culture medium

Organoid medium may comprise one or more of the components listed in the following subsections. Co-culture medium may comprise one or more of the components listed in the following subsections. Thus, the following subsections apply to both the organoid medium and the co-culture medium, unless otherwise indicated.

### Basal medium

Basal media for cell culture typically contain a large number of ingredients, which are necessary to support maintenance of the cultured cells. Suitable combinations of ingredients can readily be formulated by the skilled person, taking into account the following disclosure. A basal medium for use in the invention will generally comprise a nutrient solution comprising standard cell culture ingredients, such as amino acids, vitamins, lipid supplements, inorganic salts, a carbon energy source, and a buffer, as described in more detail in the literature and below. In some embodiments, the medium is further supplemented with one or more standard cell culture ingredient, for example selected from amino acids, vitamins, lipid supplements, inorganic salts, a carbon energy source, and a buffer. Suitable basal media will be known to the skilled person and are available commercially, e.g. non-limiting examples include Dulbecco's Modified Eagle Media (DMEM), Advanced-DMEM, Minimal Essential Medium (MEM), Knockout-DMEM (KO-DMEM), Glasgow Minimal Essential Medium (G-MEM), Basal Medium Eagle (BME), DMEM/Ham's F12, Advanced DMEM/Ham's F12, Iscove's Modified Dulbecco's Media and Minimal Essential Media (MEM), Ham's F-10, Ham's F-12, Medium 199, and RPMI 1640 Media. For example, the basal medium may be Advanced-DMEM, preferably supplemented with glutamax, penicillin/streptomycin and HEPES.

### Extracellular matrix (ECM)

Epithelial stem cells are normally grown in culture with an exogenous extracellular matrix (ECM) that is known to support cell growth (e.g. see [10]). An organoid medium can comprise an ECM. The ECM may be an exogenous ECM (meaning that it is in addition to any extracellular matrix proteins that are naturally secreted by the epithelial stem cell or population of epithelial stem cells when in contact with the expansion medium of the invention). Any suitable ECM may be used. Cells are preferably cultured in a microenvironment that mimics at least in part a cellular niche in which said cells naturally reside. A cellular niche is in part determined by the cells and by an ECM that is secreted by the cells in said niche. A cellular niche may be mimicked by culturing said cells in the presence of biomaterials or synthetic materials that provide interaction with cellular membrane proteins, such as integrins. An ECM as described herein is thus any biomaterial or synthetic material or combination thereof that mimics the in vivo cellular niche, e.g. by interacting with cellular membrane proteins, such as integrins.

In some embodiments, the ECM is in suspension, i.e. the cells are in contact with the ECM in a suspension system. In some embodiments, the ECM is in the suspension at a concentration of at least 1%, at least 2% or at least 3%. In some embodiments, the ECM is in the suspension at a concentration of from 1% to about 10% or from 1% to about 5%. The suspension method may have advantages for upscale methods. In some embodiments, the ECM is in the form of one or more domes.

One type of ECM is secreted by epithelial cells, endothelial cells, parietal endoderm like cells (e.g. Englebreth Holm Swarm Parietal Endoderm Like cells described in [11]) and connective tissue cells. This ECM comprises of a variety of polysaccharides, water, elastin, and glycoproteins, wherein the glycoproteins comprise collagen, entactin (nidogen), fibronectin, and laminin. Therefore, in some embodiments, the ECM for use in the methods of the invention comprises one or more of the components selected from the list: polysaccharides, elastin, and glycoproteins, e.g. wherein the glycoproteins comprise collagen, entactin (nidogen), fibronectin, and/or laminin. For example, in some embodiments, collagen is used as the ECM. Different types of ECM are known, comprising different compositions including different types of glycoproteins and/or different combination of glycoproteins.

The ECM can be provided by culturing ECM-producing cells, such as for example epithelial cells, endothelial cells, parietal endoderm like cells or fibroblast cells, in a receptacle, prior to the removal of these cells and the addition of isolated tissue fragments or isolated epithelial cells. Examples of extracellular matrix-producing cells are chondrocytes, producing mainly collagen and proteoglycans, fibroblast cells, producing mainly type IV collagen, laminin, interstitial procollagens, and fibronectin, and colonic myofibroblasts producing mainly collagens (type I, III, and V), chondroitin sulfate proteoglycan, hyaluronic acid, fibronectin, and tenascin-C. These are "naturally-produced ECMs". Naturally-produced ECMs can be commercially provided. Examples of commercially available extracellular matrices include: extracellular matrix proteins (Invitrogen) and basement membrane preparations from Engelbreth-Holm-Swarm (EHS) mouse sarcoma cells (e.g. Cultrex^{®} Basement Membrane Extract (Trevigen, Inc.) or MatrigelTM (BD Biosciences)).

In some embodiments, the extracellular matrix is at least 50%, at least 60% or at least 70% Matrigel, optionally 50-100%, 50-80% Matrigel, optionally about 70% Matrigel. In preferred embodiments, the extracellular matrix is at least 70% Matrigel.

In some embodiments, the ECM is a three-dimensional matrix. In some embodiments, the cells are embedded in the ECM. In some embodiments, the cells are attached to an ECM. A medium of the invention may be diffused into a three-dimensional ECM. In other embodiments, the ECM is in suspension, i.e. the cells are in contact with the ECM in a suspension system. In some embodiments, the ECM is in the suspension at a concentration of at least 1%, at least 2% or at least 3%. In some embodiments, the ECM is in the suspension at a concentration of from 1% to about 10% or from 1% to about 5%.

In a preferred method of the invention, cells are cultured in contact with an ECM. In some embodiments, the methods of the invention comprise culturing epithelial stem cells in contact with an extracellular matrix. "In contact" means a physical or mechanical or chemical contact, which means that for separating said resulting organoid or population of epithelial cells from said matrix a force needs to be used. The medium and/or cells may be placed on, embedded in or mixed with the extracellular matrix or synthetic matrix.

In some embodiments, the organoid medium is placed on top of the extracellular matrix or synthetic matrix. The organoid medium can then be removed and replenished as and when required. In some embodiments, the organoid medium is replenished every 1, 2, 3, 4, 5, 6 or 7 days. If components are "added" or "removed" from the media, then this can in some embodiments mean that the media itself is removed from the extracellular matrix or synthetic matrix and then a new media containing the "added" component or with the "removed" component excluded is placed on the extracellular matrix or synthetic matrix.

A three-dimensional matrix supports culturing of three-dimensional epithelial organoids. Therefore in some embodiments, the extracellular matrix or the synthetic matrix is a three-dimensional matrix.

In some embodiments, the medium further comprises an integrin agonist (e.g. as described in WO2020/234250). Specific examples of integrin agonists include anti-integrin antibodies, such as anti-b1 integrin antibodies (e.g. TS2/16, 12G10, 8A2, 15/7, HUTS-4, 8E3, N29 and 9EG7 antibodies). The integrin agonist may be used instead of or in addition to the extracellular matrix.

In some embodiments the ECM is a laminin-containing ECM such as MatrigelTM (BD Biosciences). In some embodiments, the ECM is MatrigelTM (BD Biosciences), which comprises laminin, entactin, and collagen IV. In some embodiments, the ECM comprises laminin, entactin, collagen IV and heparin sulphate proteoglycan (e.g. Cultrex^{®} Basement Membrane Extract Type 2 (Trevigen, Inc.)). In some embodiments, the ECM comprises at least one glycoprotein, such as collagen and/or laminin. Mixtures of naturally-produced or synthetic ECM materials may be used, if desired. In some embodiments, the ECM is BME (`basement membrane extract'), which is a soluble form of basement membrane purified from Engelbreth-Holm-Swarm (EHS) tumor (e.g. Cultrex^{®} BME).

In another embodiment, the ECM may be a synthetic ECM. For instance, a synthetic ECM, such as ProNectin (Sigma Z378666) may be used. In a further example, the ECM may be a plastic, e.g. a polyester, or a hydrogel. In some embodiments, a synthetic matrix may be coated with biomaterials, e.g. one or more glycoprotein, such as collagen or laminin.

A three-dimensional ECM supports culturing of three-dimensional epithelial organoids. The extracellular matrix material will normally be a drop on the bottom of the dish in which cells are suspended (such a method of culture may be described as a "hanging drop method"). Typically, when the matrix solidifies at 37°C, the medium is added and diffuses into the ECM. The cells in the medium stick to the ECM by interaction with its surface structure, for example interaction with integrins.

The organoid medium and/or cells may be placed on, embedded in or mixed with the ECM.

In some embodiments, stromal cells are cultured without an (exogenous) ECM, particularly for example where a poly-L-lysine coating is used in culture. In other embodiments, stromal cells are cultured with an (exogenous) ECM.

### Wnt agonist

The medium can comprise a Wnt agonist. The Wnt signalling pathway and small molecules which activate Wnt signalling are described in [12]. A Wnt agonist is defined herein as an agent that activates or enhances TCF/LEF-mediated transcription in a cell. Wnt agonists are therefore selected from true Wnt agonists that bind and activate the Wnt receptor complex including any and all of the Wnt family proteins, an inhibitor of intracellular β-catenin degradation, a GSK inhibitor (such as CHIR9901) and activators of TCF/LEF. The one or more Wnt agonist in the medium may be selected from a Wnt ligand from the Wnt family of secreted glycoproteins, an inhibitor of intracellular β-catenin degradation, a GSK-3 inhibitor, activators of TCF/LEF, an inhibitor of RNF43 or ZNRF3, and R-spondin family proteins. In some embodiments, the Wnt agonist in the medium comprises an R-spondin family protein and a GSK-3 inhibitor, and optionally further comprises a Wnt ligand from the Wnt family of secreted glycoproteins. One or more, for example, 2, 3, 4 or more Wnt agonists may be used in the medium.

The Wnt agonist in the medium is preferably any agonist able to stimulate the Wnt pathway via the Lgr5 cell surface receptor, i.e. in a preferred embodiment, the Wnt agonist in the medium is an Lgr5 agonist. Known Lgr5 agonists include Rspondin, fragments and derivatives thereof, and anti-Lgr5 antibodies (e.g. see WO2012/140274, in particular Figures 22-24, and [13]). A preferred Lgr5 agonist is Rspondin. Any suitable Rspondin may be used, for example, it may be selected from one or more of Rspondin 1, Rspondin 2, Rspondin 3 and Rspondin 4 or derivatives thereof. For example, any of Rspondin 1 (NU206, Nuvelo, San Carlos, CA), Rspondin 2 ((R&D systems), Rspondin 3, and Rspondin-4) may be used. Any suitable concentration of Rspondin may be used, for example, at least 100 ng/ml, more preferred at least 200 ng/ml, more preferred about 250 ng/ml. An example of an agonistic anti-Lgr5 antibody is 1D9 (available commercially from BD Biosciences, BDB562733, No.:562733). Fragments of Rspondin may be used as the Wnt agonist. For example, in some embodiments the Wnt agonist is a fragment of Rspondin comprising or consisting of the furin domain.

Thus, in one embodiment, the medium comprises an Lgr5 agonist, for example Rspondin, and additionally comprises a further Wnt agonist. In this context, the further Wnt agonist may, for example, be selected from the group consisting of Wnt-3a, a GSK-inhibitor (such as CHIR99021), Wnt-5, Wnt-6a Norrin, and NGS-Wnt. In one embodiment, the medium comprises Rspondin and additionally comprises a soluble Wnt ligand, such as Wnt3a or NGS-Wnt. Addition of a soluble Wnt ligand has been shown to be particularly advantageous for expansion of human epithelial stem cells (as described in WO2012/168930).

The R-spondin family protein (also referred to herein as "R-spondin") may be selected from R-spondin 1, R-spondin 2, R-spondin 3, R-spondin 4 and analogs, fragments, variants and derivatives thereof. In this context, the fragment, variant or derivative is capable of preventing the action of the E3 ligases RNF43/ZNRF3 on the Wnt receptor complex. R-spondin 1, R-spondin 2, R-spondin 3 and R-spondin 4 (also referred to herein as "R-spondin 1-4") are all characterized by two amino-terminal furin-like repeats, which are necessary and sufficient for Wnt signal potentiation, and a thrombospondin domain situated more towards the carboxyl terminus members [14]. Examples of R-spondin fragments, variants and derivatives suitable for use in the invention are known to the skilled person (e.g. see Example 2 of WO2012/140274, which describes furin domain fragments which are capable of enhancing Wnt signalling and which are incorporated herein by reference). Examples of R-spondin family protein analogs include, for example, antibodies that interact with RNF43/ZNRF3/Lgr. Agonistic anti-Lgr5 antibodies that can enhance Wnt signalling are known in the art (e.g. see antibody 1D9 described in Example 3 of [15]).

Many GSK-3 inhibitors are known in the art (e.g. see [16]; and [17]) and are available commercially (e.g. see the list available from Santa Cruz Biotechnology here: https://www.scbt.com/scbt/browse/GSK-3-beta-Inhibitors/ /N-x6oud). Any of these GSK-3 inhibitors are suitable for use in the context of the invention and the skilled person would be able to determine a suitable concentration using IC50 values.

CHIR-99021 (CAS: 252917-06-9; 6-[[2-[[4-(2,4-Dichlorophenyl)-5-(5-methyl-1H-imidazol-2-yl)-2-pyrimidinyl]amino]ethyl]amino]-3-pyridinecarbonitrile; CT99021) is a potent and selective inhibitor of GSK-3. Other aminopyrimidine inhibitors with an IC50 value of 0.6 nM to 7 nM include CHIR98014 (Axon, Cat 1126), CHIR98023, CHIR99021 (see above), TWS119 (Tocris, Cat 3835). Therefore, in some embodiments, the GSK-3 inhibitor is an aminopyrimidine inhibitor, optionally selected from CHIR98014, CHIR98023, CHIR99021 or TWS119. In some embodiments the GSK-3 inhibitor is CHIR-99021.

The Wnt ligand from the Wnt family of secreted glycoproteins may be selected from Wnt-l/Int-1, Wnt-2/Irp (InM-related Protein), Wnt-2b/13, Wnt-3/Int-4, Wnt-3a (R&D systems), Wnt-4, Wnt-5a, Wnt-5b, Wnt-6 (see [18]), Wnt-7a (R&D systems), Wnt-7b, Wnt-8a/8d, Wnt-8b, Wnt-9a/14, Wnt-9b/14b/15, Wnt-10a, Wnt-10b/12, WnM l, and Wnt-16. An overview of human Wnt proteins is provided in [19]. In some embodiments, the Wnt ligand is Wnt-3a, Wnt-5 or Wnt-6a, or optionally is Wnt-3a. Addition of a soluble Wnt ligand has been shown to be particularly advantageous for expansion of human organoids and organoid fragments (e.g. as described in [20]).

In some embodiments, the Wnt agonist in the medium is a Wnt surrogate. Wnt surrogate is a water-soluble Wnt agonist engineered by linking antagonistic Fzd and Lrp5/6-binding modules into a single polypeptide chain, thus forcing receptor heterodimerisation while blocking endogenous Wnt binding. Wnt surrogate supports the growth of a broad range of cultures. Furthermore, Wnt surrogate is a non-lipidated Wnt agonists that can be produced in serum free medium, kept frozen and circumvent the differences in activity of Wnt-conditioned media produced by different laboratories [21]. In some embodiments, the Wnt surrogate is next-generation surrogate Wnt (NGS-Wnt), for example as described in [22]. NGS-Wnt may be provided at a concentration of about 0.1 nM to about 0.5 nM. In some embodiments, the medium comprises NGS-Wnt at a concentration of about 0.5 nM. In some embodiments, the medium comprises NGS-Wnt at a concentration of about 0.1 nM.

In some embodiments, the Wnt agonist in the medium is a Wnt surrogate. Wnt surrogate is a water-soluble Wnt agonist engineered by linking antagonistic Fzd and Lrp5/6-binding modules into a single polypeptide chain, thus forcing receptor heterodimerisation while blocking endogenous Wnt binding. Wnt surrogate supports the growth of a broad range of cultures. Furthermore, Wnt surrogate is a non-lipidated Wnt agonists that can be produced in serum free medium, kept frozen and circumvent the differences in activity of Wnt-conditioned media produced by different laboratories ([23]). In some embodiments, the Wnt surrogate is next-generation surrogate Wnt (NGS-Wnt), for example as described in [24]. NGS-Wnt may be provided at a concentration of about 0.1 nM to about 0.5 nM. In some embodiments, the medium comprises NGS-Wnt at a concentration of about 0.5 nM. In some embodiments, the medium comprises NGS-Wnt at a concentration of about 0.1 nM.

A soluble Wnt agonist, such as Wnt-3a, may be provided in the form of Wnt conditioned media. For example, about 10% to about 50% Wnt conditioned medium may be used.

Rspondin may be provided in the form of Rspo conditioned medium. For example, about 10% to about 30%, e.g. about 10 ng/ml to about 10 µg/ml, preferably about 1 µg/ml, Rspo conditioned medium may be used.

Examples of Rspondin mimics suitable for use in the invention are provided in WO2012/140274, which is incorporated herein by reference.

### Mitogenic growth factors

The medium can comprise a mitogenic growth factor. Mitogenic growth factors typically induce cell division via the mitogen-activated protein kinase signalling pathway. Many receptor tyrosine kinase ligands are mitogenic growth factors. In some embodiments, the mitogenic growth factor can bind to a receptor tyrosine kinase. In some embodiments, the mitogenic growth factor can bind to more than one receptor tyrosine kinase. In some embodiments, the one or more mitogenic growth factor binds to a receptor tyrosine kinase such as EGFR, an FGFR or HGFR, optionally wherein the one or more mitogenic growth factors are selected from EGF, FGF and HGF.

In some embodiments, the mitogenic growth factor binds to EGFR, HER1, HER2, HER3 or HER4. In some embodiments, the mitogenic growth factor binds to EGFR. In some embodiments, a HER2-4 ligand is included in the medium in addition to an EGFR ligand. For example, in some embodiments, neuregulin is included in the medium in addition to EGF. Neuregulin has been shown to be advantageous for culture of lung and breast tissue (e.g. see [25], and [26]). In some embodiments, the one or more mitogenic growth factor in the medium is EGF. Any suitable EGF may be used, for example, EGF obtained from Peprotech.

FGFs stimulate cells by interacting with cell surface tyrosine kinase receptors (FGFR). Four closely related receptors (FGFR1-FGFR4) have been identified. Therefore, in some embodiments, the mitogenic growth factor binds to an FGF receptor family member. FGF receptor family members include (but are not limited to) FGFR1, FGFR2, FGFR3 or FGFR4. FGFR1-FGFR3 genes have been shown to encode multiple isoforms, and these isoforms can be critical in determining ligand specificity. There are several FGFs that bind to the FGF receptor family members, including (but not limited to) FGF2, FGF4, FGF7 and FGF10. These are commercially available. Therefore, in some embodiments, the mitogenic growth factor is an FGF. In some embodiments, the FGF is selected from FGF2, FGF4, FGF7 and FGF 10. In preferred embodiments, the FGF is FGF2 and/or FGF 10. In a most preferred embodiment, the FGF is FGF2 and FGF 10.

Hepatocyte growth factor/scatter factor (HGF/SF) is a morphogenic factor that regulates cell growth, cell motility, and morphogenesis by activating a tyrosine kinase signalling cascade after binding to the proto-oncogenic HGFR. The HGFR is also known as the c-Met receptor. HGF has been shown to be useful in epithelial stem cell culture. Therefore, in some emobdiments the mitogenic growth factor binds HGFR. In some embodiments, the mitogenic growth factor is HGF. Any suitable HGF may be used, for example, HGF obtained from Peprotech.

In some embodiments, more than one mitogenic growth factor is included in the medium, e.g. two or three mitogenic growth factors. For example, in some embodiments, the one or more mitogenic growth factors in the medium are EGF and FGF. In some embodiments, the one or more mitogenic growth factors in the medium are EGF, FGF2 and FGF 10. In some embodiments, the one or more mitogenic growth factors in the medium are EGF, optionally at a final concentration of about 50 ng/ml, FGF2, optionally at a final concentration of about 5 ng/ml, and FGF10, optionally at a final concentration of about 10 ng/ml.

In some embodiments hepatocyte growth factor (HGF) is also present in the presence or absence of EGF and/or FGF.

### BMP inhibitor

The medium can comprise a BMP inhibitor. A BMP inhibitor is defined as an agent that binds to a BMP molecule to form a complex wherein the BMP activity is neutralized, for example by preventing or inhibiting the binding of the BMP molecule to a BMP receptor. Alternatively, said inhibitor is an agent that acts as an antagonist or reverse agonist. This type of inhibitor binds with a BMP receptor and prevents binding of a BMP to said receptor. An example of a latter agent is an antibody that binds a BMP receptor and prevents binding of BMP to the antibody-bound receptor.

A BMP inhibitor may be added to the media in an amount effective to inhibit a BMP-dependent activity in a cell to at most 90%, more preferred at most 80%, more preferred at most 70%, more preferred at most 50%, more preferred at most 30%, more preferred at most 10%, more preferred 0%, relative to a level of a BMP activity in the absence of said inhibitor, as assessed in the same cell type. As is known to a skilled person, a BMP activity can be determined by measuring the transcriptional activity of BMP, for example as exemplified in [27].

Several classes of natural BMP-binding proteins are known, including noggin (Peprotech), Chordin and chordin-like proteins (R&D systems) comprising chordin domains, Follistatin and follistatin-related proteins (R&D systems) comprising a follistatin domain, DAN and DAN-like proteins (R&D systems) comprising a DAN cysteine-knot domain, sclerostin /SOST (R&D systems), decorin (R&D systems), and alpha-2 macroglobulin (R&D systems).

Therefore, in some embodiments, the BMP inhibitor is selected from noggin, DAN, and DAN-like proteins including Cerberus and Gremlin (R&D systems). These diffusible proteins are able to bind a BMP ligand with varying degrees of affinity and inhibit their access to signalling receptors. The addition of any of these BMP inhibitors to the basal culture medium prevents the loss of stem cells. A preferred BMP inhibitor is noggin.

### TGF-β inhibitor

The medium can comprise a TGF-beta (or "TGF-β") inhibitor. The presence of a TGF-beta inhibitor in the expansion media is particularly advantageous for increasing human organoid formation efficiency. A TGF-beta inhibitor is any agent that reduces the activity of the TGF-beta signalling pathway, also referred to herein as the ALK4, ALK5 or ALK7 signalling pathway. A TGF-beta inhibitor according to the present invention may be a protein, peptide, small-molecule, small-interfering RNA, antisense oligonucleotide, aptamer or antibody. The inhibitor may be naturally occurring or synthetic.

In some embodiments the TGF-beta inhibitor is a small molecule inhibitor, such as A83-01 (or "A-83-01"). A83-01 is a commercially available selective inhibitor of ALK4, ALK5 and ALK7 (Tocris cat. no. 2939). It is described in the catalog as a potent inhibitor of TGF-β type I receptor ALK5 kinase, type I activin/nodal receptor ALK4 and type I nodal receptor ALK7 (IC₅₀ values are 12, 45 and 7.5 nM respectively), which blocks phosphorylation of Smad2, and which only weakly inhibits ALK-1, -2, -3, -6 and MAPK activity. Other commercially available inhibitors with similar properties include, but are not limited to A77-01, LY2157299, LY2109761, LY3200882, GW788388, Pirfenidone, RepSox, SB431542, SB505124, SB525334, LY364947, SD-208 and Vactosertib. The IC50 values for these inhibitors are known in the art and the skilled person would be able to select a suitable inhibitor at suitable concentration based on the teaching provided in the examples of this application.

### Nicotinamide

In some embodiments, the medium comprises nicotinamide. Nicotinamide is an amide derivative of vitamin B3, a poly (ADP-ribose) polymerase (PARP) inhibitor, and represents the primary precursor of NAD+. It is available commercially (e.g. from Stemcell Technologies Cat. 07154).

### Prostaglandin pathway activator

In some embodiments, the medium further comprises a prostaglandin pathway activator. The prostaglandin pathway activator may be any one or more of the compounds selected from the list comprising: phospholipids, arachidonic acid (AA), prostaglandin E2 (PGE2), prostaglandin G2 (PGG2), prostaglandin F2 (PGF2), prostaglandin H2 (PGH2), prostaglandin D2 (PGD2). In some embodiments, the activator of the prostaglandin signalling pathway is PGE2 and/or AA. In some embodiments, the activator of the prostaglandin signalling pathway is PGE2.

### cAMP activator

In some embodiments, the medium comprises a cAMP pathway activator. The cAMP pathway activator may be any suitable activator which increases the levels of cAMP in a cell. In some embodiments, the cAMP pathway activator is an adenylyl cyclase activator or a cAMP analog. Examples of suitable adenylyl cyclase activators include forskolin, a forskolin analog and cholera toxin. Examples of forskolin analogs are known in the art and include NKH477 (e.g. catalogue no. Tocris 1603). Examples of cAMP analogs are also known in the art, and include for example, 8-bromo-cAMP. 8-bromo-cAMP is a cell-permeable cAMP analog having greater resistance to hydrolysis by phosphodiesterases than cAMP. In some embodiments, the cAMP pathway activator is therefore selected from forskolin, cholera toxin, NKH477 and 8-bromo-cAMP. In some embodiments, the cAMP pathway activator is forskolin. In some embodiments, the cAMP pathway activator is not cholera toxin.

### Additional components

The medium may be supplemented with one or more of the compounds selected from the group consisting of gastrin, B27, N-acetylcysteine (or "N-Ac"), and N2. Thus in some embodiments the media described above further comprise one or more components selected from the group consisting of: gastrin, B27, N2 and N-Acetylcysteine. B27 (Invitrogen), N-Acetylcysteine (Sigma) N2 (Invitrogen), and Gastrin (Sigma) are believed to control proliferation of the cells and assist with DNA stability. In some embodiments, the medium further comprises B27 and N-acetylcysteine.

In some embodiments the medium further comprises a ROCK inhibitor (Rho-Kinase inhibitor). A ROCK inhibitor is particularly useful for attachment of cells when establishing new cultures and/or when splitting ("passaging") cells. Suitable ROCK inhibitors are known in the art and available commercially (including but not limited to GSK 269962, GSK 429286, H 1152 dihydrochloride, Glycyl-H 1152 dihydrochloride, SR 3677 dihydrochloride, SB 772077B dihydrochloride and Y-27632 dihydrochloride, all available from Tocris). In some embodiments the medium is supplemented with about 5µM to about 20µM or about 8 µM to about 15µM ROCK inhibitor, optionally about 10µM ROCK inhibitor. A particularly preferred ROCK inhibitor is Y-27632.

In some embodiments, it is preferred that the medium, particularly expansion medium, does not comprise an undefined component (such as fetal bovine serum or fetal calf serum or feeder cells). Various different serum replacement formulations are commercially available and are known to the skilled person. Where a serum replacement is used, it may be used at between about 1% and about 30% by volume of the medium, according to conventional techniques. In some embodiments, the medium is serum free and/or feeder free.

A preferred medium is a defined synthetic medium that is buffered at a pH of about 7.4 (preferably with a pH of about 7. 2 - about 7.6 or at least about 7.2 and not higher than about 7.6) with a carbonate-based buffer, while the cells are cultured in an atmosphere comprising between about 5% and about 10% CO2, or at least about 5% and not more than about 10% CO2, preferably about 5% CO2.

### In vitro methods

### General

Methods of the claimed invention may be performed *in vivo, ex vivo, in vitro, in situ, ex situ,* or any combination thereof. Preferably the methods are performed *in vitro.*

Traditionally, cell lines and more recently iPS cells have been used as *ex vivo* cell/organ and/or disease models (for example, see [28]). However, these methods suffer a number of challenges and disadvantages. For example, cell lines cannot be obtained from all patients (only certain biopsies result in successful cell lines) and therefore, cell lines cannot be used in personalised diagnostics and medicine. iPS cells usually require some level of genetic manipulation to reprogramme the cells into specific cell fates. Alternatively, they are subject to culture conditions that affect karyotypic integrity and so the time in culture must be kept to a minimum (this is also the case for human embryonic stem cells). This means that iPS cells cannot accurately represent the *in vivo* situation but instead are an attempt to mimic the behaviour *of in vivo* cells. Cell lines and iPS cells also suffer from genetic instability.

By contrast, the organoids and co-cultures of the invention provide a genetically stable platform which faithfully represents the *in vivo* situation. In some embodiments, the organoids and co-cultures of the invention comprise all differentiated cell types that are present in the corresponding *in vivo* situation. In other embodiments, the organoids and co-cultures may be further differentiated to provide all differentiated cell types that are present *in vivo.* Thus the organoids and co-cultures can be used to gain mechanistic insight into a variety of diseases and therapeutics, to carry out *in vitro* drug screening, to evaluate potential therapeutics, to identify possible targets (e.g. proteins) for future novel (drug) therapy development and/or to explore gene repair coupled with cell-replacement therapy.

Accordingly, the invention also provides use of organoids and co-cultures in an assay assessing epithelial viability, metabolic activity, permeability, barrier function integrity and/or activity of transporter proteins. Methods of assessing viability, permeability and barrier function integrity of organoids and co-cultures and activity of transporter proteins in organoids and co-cultures are described herein.

Co-cultures of the invention may be used in *in vitro* methods, such as methods of investigating (e.g. validating) the co-culture, methods of testing therapeutic agents, and methods of diagnosis and/or prognosis of a disease. In some embodiments, the organoids are used for modelling *in vivo* interactions. These methods can be used to test libraries of chemicals, antibodies, natural products (e.g. plant extracts or microbial compounds), etc., for suitability for use as drugs, diagnostics, cosmetics and/or preventative medicines. For example, the cells are preferably exposed to multiple concentrations of a test agent for a certain period of time. At the end of the exposure period, the cultures are evaluated (e.g. by determining the presence or absence of at least one change).

In some embodiments, the invention provides the use of a co-culture in drug screening, target validation, target discovery, toxicology, toxicology screens, toxicity assays, or as an *ex vivo* cell/organ model. In some embodiments, the invention provides the use of an organoid in an *ex vivo* method to predict a clinical outcome.

The invention provides the use of organoids and co-cultures in research of tissue embryology, cell lineages, and differentiation pathways; research to identify the chemical and/or neuronal signals that lead to the release of the respective hormones; gene expression studies including recombinant gene expression; research of mechanisms involved in tissue injury and repair; research of inflammatory diseases; research of fibrotic diseases; studies of pathogenetic mechanisms; or studies of mechanisms of cell transformation.

### Determining at least one change

Methods of the invention may involve determining the presence or absence of at least one change in the co-culture.

In principle, the presence or absence of a change in any biochemical, genetic, phenotypic or phenomenological property of the co-culture may be determined. The at least one change may be an increase or a decrease in the property.

For example, the at least one change may comprise a change in organoid morphology, a change in organoid size (e.g. area), a change in epithelial cell size (e.g. area), a change in epithelial thickness, a reduction in cell viability, a reduction in cell proliferation, an increase in cell death, a change in secretome profile, a change in cytokine secretion, an increase in cell apoptosis, an increase in caspase activity, and/or a change in the expression of one or more genes, optionally wherein the change in the expression of one or more genes comprises a change in expression of one or more disease biomarkers, one or more fibrosis biomarkers, and/or one or more inflammation biomarkers.

The at least one change may comprise a change in epithelial viability, a change in metabolic activity, a change in permeability, a change in barrier function integrity, and/or a change in activity of transporter proteins. Methods of assessing viability, permeability and barrier function integrity of organoids and co-cultures and activity of transporter proteins and co-cultures are described herein.

In some embodiments, the at least one change comprises a change in organoid aggregation, for example an increase in organoid aggregation. An increase in organoid aggregation may be observed when the at least one organoid is co-cultured with at least one stromal cell, without proinflammatory stimulation (for example, compared to the organoid prior to co-culture). An increase in organoid aggregation may be observed when the at least one organoid is co-cultured with at least one stromal cell, with proinflammatory stimulation (for example, compared to the organoid prior to co-culture). Aggregation in co-cultures prepared with proinflammatory stimulation, may be lesser than aggregation in co-cultures prepared without proinflammatory stimulation. Thus, aggregation may be used as an indicator that at least one organoid has been successfully co-cultured with at least one stromal cell, and aggregation may also be used to determine whether a co-culture exhibits a proinflammatory profile.

In some embodiments, the at least one change comprises a change in organoid-surface adherence, for example a decrease in organoid-surface adherence.

In some embodiments, the at least one change comprises a change in organoid morphology, optionally wherein the change in organoid morphology comprises a change in organoid shape, organoid size (e.g. area), organoid lumen size (e.g. organoid lumen area), epithelial cell shape, or epithelial cell size (e.g. area). Preferably, the at least one change comprises a change in organoid area. Without wishing to be bound by any theory, the inventors have found that organoid size, and cystic morphology in particular, is highly reproducible and specific readout for the functional presence of fibroblasts in co-culture having a proinflammatory profile. Organoid size, preferably organoid area, may be measured by brightfield imaging.

In some embodiments, the at least one change comprises increased area of the at least one organoid, optionally wherein the area is increased to at least about 10,000 µm², at least about 15,000 µm², at least about 20,000 µm², or at least about 25,000 µm².

In some embodiments, the at least one change comprises increased area of the at least one organoid, wherein the increase is of at least about 10%, at least about 20%, at least about 30%, at least about 40%, at least about 50%, at least about 60%, at least about 70%, or at least about 80%.

In some embodiments, the at least one change comprises a change in epithelial thickness, for example a decrease in epithelial thickness, optionally wherein the decrease is by at least about 10%, at least about 20%, at least about 30%, at least about 40%, at least about 50%, at least about 60%, at least about 70%, at least about 80%, or at least about 90%.

In some embodiments, the at least one change comprises a change in expression of one or more genes, preferably a change in expression of one or more disease biomarkers. The term "expressed" is used to describe the presence of a marker within a cell. In order to be considered as being expressed, a marker must be present at a detectable level. By "detectable level" is meant that the marker can be detected using one of the standard laboratory methodologies such as PCR, blotting or FACS analysis. A gene is considered to be expressed by a cell of the organoid, stromal cell, or co-culture of the invention if expression can be reasonably detected after 30 PCR cycles, which corresponds to an expression level in the cell of at least about 100 copies per cell. The terms "express" and "expression" have corresponding meanings. At an expression level below this threshold, a marker is considered not to be expressed. The comparison between the expression level of a marker in a cell of the invention, and the expression level of the same marker in another cell, such as for example an embryonic stem cell, may preferably be conducted by comparing the two cell types that have been isolated from the same species. Preferably this species is a mammal, and more preferably this species is human. Such comparison may conveniently be conducted using a reverse transcriptase polymerase chain reaction (RT-PCR) experiment.

Thus, the presence or absence of at least one change in cellular composition of an organoid may be determined by detecting the expression of one or more marker genes, for example by detecting the presence or absence of at least one change in expression of one or more marker genes. Lgr5 is a marker for Lgr5⁺ stem cells. Ki67 is a marker for proliferating cells, such as Lgr5⁺ stem cells. Goblet cells may be detected by staining for mucus, e.g. by performing Alcian blue staining, or detecting the expression of mucin-2 (Muc2), as described herein. Intestinal alkaline phosphatase (ALPI or ALPI1) is a marker for enterocytes. Lysozyme is a marker for Paneth cells. Chromogranin A is a marker for enteroendocrine cells.

Thus, in some embodiments, the presence or absence of a change in expression of at least one of the following genes is determined: ALPI, MUC2, lysozyme, Ki67 and Lgr5. In some embodiments, the organoid and/or co-culture of the invention expresses Lgr5 and Muc2, e.g. the change is an increase in expression of Lgr5 and Muc2. In some embodiments, the organoid and/or co-culture of the invention does not express ALPI, e.g. the change is a decrease in expression of ALPI. In some embodiments, the organoid and/or co-culture of the invention expresses Lgr5 and Muc2 and does not express ALPI, e.g. the change is an increase in the expression of Lgr5 and Muc2 and a decrease in the expression of ALPI. In some embodiments, the organoid and/or co-culture of the invention expresses lysozyme.

In some embodiments, the at least one change comprises upregulation of expression of a stem cell marker, optionally wherein the stem cell marker is OLFM4, optionally wherein the upregulation is by at least about 10%, at least about 50%, at least about 100%, at least about 200%, at least about 300%, at least about 400%, at least about 500%, or at least about 1,000%.

In some embodiments, the at least one change comprises downregulation of expression of an enterocyte marker, optionally wherein the enterocyte marker is ALPI, optionally wherein the downregulation is by at least about 10%, at least about 20%, at least about 30%, at least about 40%, at least about 50%, at least about 60%, at least about 70%, at least about 80%, or at least about 90%.

In some embodiments, the at least one change comprises downregulation of expression of a proliferation marker, optionally wherein the proliferation marker is KI67, optionally wherein the downregulation is by at least about 10%, at least about 20%, at least about 30%, at least about 40%, at least about 50%, at least about 60%, at least about 70%, at least about 80%, or at least about 90%.

In some embodiments, the at least one change comprises a change in secretome profile, optionally a change in secretion of a cytokine, optionally a change in secretion of IL6 and/or CXCL2. In some embodiments, the at least one change is a change in expression of IL-6 (preferably an increase in expression of IL-6), such as secretion of IL-6 (preferably an increase in secretion of IL-6). In some embodiments, the at least one change is a change in expression of CXCL2 (preferably an increase in expression of CXCL2), such as secretion of CXCL2 (preferably an increase in secretion of CXCL2).

In some embodiments, the change comprises expression of at least about 10,000 pg/ml of IL6, at least about 20,000 pg/ml of IL6, at least about 30,000 pg/ml of IL6, at least about 40,000 pg/ml of IL6, at least about 50,000 pg/ml of IL6, or at least about 100,000 pg/ml of IL6.

In some embodiments, the at least one change comprises a change in caspase activity, optionally comprising a change in caspase 3/7 activity, optionally wherein the caspase activity is increased by at least about 500%, at least about 1,000%, at least about 1,500%, at least about 2,000%, or at least about 2,500%.

In some embodiments, a cytokine is added to the co-culture prior to determining the presence or absence of at least one change in the co-culture, optionally wherein the cytokine is TNF and/or IFNγ, optionally wherein the cytokine is at a concentration of at most about 100 ng/ml, at most about 50 ng/ml, at most about 15 ng/ml, at most about 5 ng/ml, at most about 1 ng/ml, at most about 0.1 ng/ml, or at most about 0.01 ng/ml. Preferably wherein the cytokine is TNF, the concentration is at least about 1 ng/ml. Preferably wherein the cytokine is IFNγ, the concentration is at least about 15 ng/ml.

In some embodiments, a cytokine signalling inhibitor is added to the co-culture prior to determining the presence or absence of at least one change in the co-culture, optionally wherein the cytokine signalling inhibitor reduces activity of the JAK/STAT pathway, optionally wherein the cytokine signalling inhibitor is tofacitinib, for example about 10 µM tofacitinib.

The at least one change in a co-culture comprising at least one organoid may be determined relative to the at least one organoid prior to forming the co-culture. The at least one change in a co-culture, may be determined relative to a reference organoid, such as a reference organoid not incorporated into a co-culture. The at least one change in a co-culture, may be determined relative to a reference organoid, such as a reference organoid that does not comprise stromal cells, optionally wherein the reference organoid does not comprise non-epithelial cells. The at least one change in a co-culture may be determined after at least one agent has been applied to the co-culture, for example relative to the co-culture before the at least one agent was applied. The at least one change in a co-culture may be determined after at least one agent has been applied to the co-culture, for example relative to a reference co-culture to which the at least one agent has not been applied.

In some embodiments, the presence or absence of the at least one change is determined in a co-culture that has been cultured for at least about 12 hours, at least about 24 hours, at least about 48 hours, at least about 72 hours, at least about 96 hours, or at least about 120 hours. Preferably, the presence or absence of the at least one change is determined in a co-culture that has been cultured for at least about 72 hours.

In some embodiments, the presence or absence of the at least one change is determined at least about 1 day, at least about 2 days, at least about 3 days, at least about 4 days, at least about 5 days, at least about 6 days, or at least about 7 days, after the at least one organoid and the at least one stromal cell are combined to form the co-culture. Preferably, the presence or absence of the at least one change is determined at least about 7 days after the at least one organoid and the at least one stromal cell are combined to form the co-culture.

Determining the presence or absence of at least one change may comprise determining the presence or absence of stromal cells in the co-culture, optionally determining the quantity of stromal cells in the co-culture. Determining the quantity of stromal cells in the co-culture may comprise determining the extent of death of stromal cells in the co-culture.

Determining the presence or absence of at least one change may comprise determining the presence or absence of organoid cells in the co-culture, optionally determining the quantity of organoid cells in the co-culture. Determining the quantity of organoid cells in the co-culture may comprise determining the extent of death of organoid cells in the co-culture.

Determining the presence or absence of at least one change may comprise determining the presence or absence of epithelial cells in the co-culture, optionally determining the quantity of epithelial cells in the co-culture. Determining the quantity of epithelial cells in the co-culture may comprise determining the extent of death of epithelial cells in the co-culture.

Determining the presence or absence of at least one change may comprise determining the presence or absence of non-epithelial cells in the co-culture, optionally determining the quantity of non-epithelial cells in the co-culture. Determining the quantity of non-epithelial cells in the co-culture may comprise determining the extent of death of non-epithelial cells in the co-culture.

Determining the presence or absence of at least one change may comprise determining whether the co-culture exhibits a proinflammatory profile, for example after at least one agent has been applied to the co-culture.

### Techniques

Any *in vitro* method disclosed herein, including co-culture validation, testing of therapeutic agents, and/or diagnosis and/or prognosis, and any method involving determining at least one change, may comprise standard laboratory techniques. For example, such *in vitro* methods may comprise any one of whole-genome sequencing, mRNA sequencing, peptidome profiling and/or microscopy. One of more of these techniques can be used to ensure that the co-cultures and/or organoids are uniform and/or meet expectation, in a form of information discovery and/or information verification. For example, they can be used to determine mRNA transcription differences between organoids and co-cultures, and whether these differences in mRNA transcription are mirrored in differences in protein expression. The presence of specific antigens on organoids may also be confirmed, and whether any new antigens develop in co-culture only. The up-regulation of inflammatory factors in the co-culture microenvironment may also be investigated.

In principle, determining the presence or absence of at least one change may be performed using any suitable laboratory method known to the skilled person. In some embodiments, the determining can comprise a cellular proliferation assay, a viability assay, flow cytometric analysis, ELISA for IFN-γ (Interferon gamma), analysis of gene expression, and/or cellular imaging.

Various assays are known in the art to measure cell viability, including assays that determine viability based on cellular membrane integrity (e.g. using nucleic acid dyes Propidium iodide, TO-PRO-3 Iodide or 7-AAD), a cellular function such as enzymatic activity (e.g. using Calcein or a CyQUANT Cytotoxicity Assay Kit) or metabolic activity (e.g. using alarmarBlue Cell Viability Reagent or a yQUANT MTT Cell Viability Assay). In some embodiments, at least 1%, at least 2%, at least 3%, at least 4%, at least 5%, at least 7.5%, at least 10%, at least 20%, at least 30% or at least 40% of the DNA in the organoid or co-culture is damaged e.g. as measured by the Comet assay [29] or a yH2AX assay [30]. A reduction in cell viability may also be determined by CellTiter Glo Luminescent Cell Viability Assay kit (Promega), intracellular flow cytometric staining for active Caspase 3 (BD), or positive stain for death cells. Positive strain for death cells includes non-cell membrane permeable DNA stains such as NucRed Dead 647 ReadyProb.

An increase in cell death may be detected by brightfield imaging.

For determining changes in gene expression, depending on the identity of the marker, the expression of said marker may be assessed by RT-PCR, immuno-histochemistry or histological staining after about 3 days, about 4 days, about 5 days, about 6 days, about 7 days, about 8 days, about 9 days, or more, of culture, as described herein. In some embodiments, the expression of the marker is measured after about 3 days, about 4 days, about 5 days, about 6 days, about 7 days, about 8 days, about 9 days, about 10 days, about 11 days, about 12 days or more, e.g. about 16 days, of culture, as described herein.

In some embodiments, the at least one change comprises a change in cytokine expression, for example a change in expression of IL-6 and/or CXCL2. These may be measured by HTRP or ELISA, respectively.

In some embodiments, the at least one change comprises a change in apoptosis, for example a change in caspase activity (such as caspase 3/7) or a change in cell viability. This may be measured by brightfield imaging with Calcein stain (for cell death) and/or using commercial caspase assays, such as Caspase-Glo.

Image analysis may be used to assess characteristics of cells in culture such as cell morphology; cell structures; evidence for apoptosis or cell lysis; and organoid composition and structure. Many types of imaging analysis are well known in the art, such as electron microscopy, including scanning electron microscopy and transmission electron microscopy, confocal microscopy, stereomicroscopy, fluorescence microscopy. Histological analysis can reveal basic architecture and cell types.

The methods of the invention have high-throughput (HTP) capacity. In some embodiments, the methods of the invention can be performed on 96-well plates and/or on 384-well plates, preferably on 96-well plates.

### Validation

Co-cultures of the invention may be investigated to determine whether their properties meet expectations, for example whether the co-culture has been successfully developed and contains at least one organoid and at least one stromal cell in viable conditions. This is also termed "validation".

Validation may comprise determining the presence or absence of at least one change, as described herein. Therefore, the invention provides a method for determining the presence or absence of at least one change in a co-culture (i.e. a method for validating the co-culture), comprising at least one organoid and at least one stromal cell, wherein the method comprises determining the presence or absence of the at least one change in the co-culture.

Validation may be used, for example, as a quality control step during preparation of co-culture, prior to other methods and uses described herein.

Validation may comprise determining whether the co-culture exhibits interactions between epithelial cells and stromal cells as expected *in vivo.* Validation may comprise determining the presence or absence of at least one change in the at least one stromal cell when combined with the at least one organoid to form the co-culture, compared to the at least one stromal cell prior to being combined with the at least one organoid to form the co-culture. Validation may comprise determining the presence or absence of at least one change in the at least one organoid when combined with the at least one stromal cell to form the co-culture, compared to the at least one organoid prior to being combined with the at least one stromal cell to form the co-culture.

The presence or absence of at least one change described herein, in a co-culture relative to an expected value (such as an expected value for at least one reference organoid not combined with at least one stromal cell to form the co-culture, or to at least one reference co-culture), may be determined as part of validation.

### Inflammation

### Proinflammatory stimuli

Cells, organoids, co-cultures and methods of the invention, may involve proinflammatory stimulation, in which cells, organoids, or co-cultures, are exposed to a proinflammatory stimulus. Proinflammatory stimulation causes the exposed cells to adopt a proinflammatory profile, and is useful for modelling *in vivo* states of health and disease.

Accordingly, the invention provides co-cultures exhibiting a proinflammatory profile, for example co-cultures prepared in media comprising at least one proinflammatory stimulus, as described herein.

In some embodiments, the proinflammatory stimulus comprises a cytokine. Cytokines that cause cells, organoids or co-cultures to adopt a proinflammatory profile are proinflammatory stimuli. These include IL-1β, oncostatin M (OSM), IL-6, IFNα, IFNβ, IFNλ, TGF-β, IL-23, IL-22, IL-4, IL-13, or IL-5. Of particular interest as proinflammatory stimuli are IL-1β and/or OSM. In some embodiments, the proinflammatory stimulus comprises IL-1β. In some embodiments, the proinflammatory stimulus comprises OSM. In some embodiments, the proinflammatory stimulus comprises IL-1β and OSM. In some embodiments, the proinflammatory stimulus is not IL-2. In some embodiments, the proinflammatory stimulus is not TNF or IFNγ., In some embodiments, the proinflammatory stimulus is not IL-2, IFNγ or TNF. Contemplated herein are other proinflammatory stimuli that are not cytokines, such as viral particles, single-stranded nucleic acids, double-stranded nucleic acids, RNA, DNA, and bacterial products (including lipopolysaccharide "LPS").

The step of contacting the co-culture with one or more proinflammatory stimuli may be carried out simultaneously with, or after, combining the at least one organoid and at least one stromal cell to form the co-culture. The step of contacting the co-culture with one or more proinflammatory stimuli may not be carried out before combining the at least one organoid and at least one stromal cell to form the co-culture.

The inventors have found that co-cultures exposed to one or more proinflammatory stimuli do not need to subsequently be re-stimulated, as they maintain up-regulation of proinflammatory genes (including PDPN and TGF-β). Therefore, in some embodiments, exposure to proinflammatory stimuli leads to upregulation of PDPN and/or TGF-β. In some embodiments, a proinflammatory profile comprises upregulated PDPN and/or TGF-β, compared to the absence of proinflammatory stimuli.

The inventors did not observe dose-dependent increases in expression of proinflammatory genes upon exposure to proinflammatory stimuli. Therefore, doses described in the examples are considered to maximally induce a proinflammatory profile in co-cultures.

In some embodiments, at least one proinflammatory stimulus is applied to the co-culture such that the at least one proinflammatory stimulus reaches a concentration of at least about 0.01 ng/ml, at least about 0.1 ng/ml, at least about 1 ng/ml, at least about 1.5 ng/ml, at least about 5 ng/ml, at least about 15 ng/ml, at least about 50 ng/ml, or at least about 100 ng/ml. Particularly preferred is a concentration of at least about 1 ng/ml, optionally at least about 15 ng/ml. These concentrations may refer to each proinflammatory stimulus individually, or to all proinflammatory stimuli collectively. Preferably, these concentrations refer to each proinflammatory stimulus individually. Particularly preferred are proinflammatory stimuli comprising at least about 1 ng/ml IL-1β and at least about 1 ng/ml OSM.

In some embodiments, at least one proinflammatory stimulus is applied to the co-culture such that the at least one proinflammatory stimulus reaches a concentration of about 0.01 ng/ml, about 0.1 ng/ml, about 1 ng/ml, about 1.5 ng/ml, about 5 ng/ml, about 15 ng/ml, about 50 ng/ml, or about 100 ng/ml. Particularly preferred is a concentration of about 1 ng/ml, optionally about 15 ng/ml. These concentrations may refer to each proinflammatory stimulus individually, or to all proinflammatory stimuli collectively. Preferably, these concentrations refer to each proinflammatory stimulus individually. Particularly preferred are proinflammatory stimuli comprising about 1 ng/ml IL-1β and about 1 ng/ml OSM.

In some embodiments, at least one proinflammatory stimulus is applied to the co-culture such that the at least one proinflammatory stimulus reaches a concentration of at most about 0.01 ng/ml, at most about 0.1 ng/ml, at most about 1 ng/ml, at most about 1.5 ng/ml, at most about 5 ng/ml, at most about 15 ng/ml, at most about 50 ng/ml, or at most about 100 ng/ml. Particularly preferred is a concentration of at most about 1 ng/ml, optionally at most about 15 ng/ml. These concentrations may refer to each proinflammatory stimulus individually, or to all proinflammatory stimuli collectively. Preferably, these concentrations refer to each proinflammatory stimulus individually. Particularly preferred are proinflammatory stimuli comprising at most about 1 ng/ml IL-1β and at most about 1 ng/ml OSM.

The at least one proinflammatory stimulus may be added to the co-culture medium at two intervals separated by at least about 24 hours, at least about 48 hours, at least about 72 hours, or at least about 96 hours.

Thus, in some embodiments, determining the presence or absence of at least one change comprises applying at least one proinflammatory stimulus to the co-culture and determining the presence or absence of proinflammatory profile.

In some embodiments, the proinflammatory stimulus is a therapeutic agent.

In some embodiments, the proinflammatory stimulus is a diagnostic agent.

In some embodiments, determining the presence or absence of at least one change comprises determining the presence or absence of a proinflammatory profile.

In some embodiments, the proinflammatory stimulus is not a therapeutic agent.

In some embodiments, the proinflammatory stimulus is not a diagnostic agent.

In some embodiments, determining the presence or absence of at least one change comprises adding at least one proinflammatory stimulus to the co-culture medium, and optionally determining the presence or absence of a proinflammatory profile.

### Inflammatory stimuli

In some embodiments, at least one inflammatory stimulus is applied to a cell, organoid, or co-culture and the presence or absence of at least one change is indicative of whether an inflammatory response is observed in the co-culture. In some embodiments, the at least one inflammatory stimulus comprises a cytokine, such as a damage-inducing cytokine. In some embodiments, the inflammatory cytokine comprises TNF. In some embodiments, the inflammatory cytokine comprises IFNγ. The response of the cell, organoid, or co-culture is affected by whether the cell, organoid or co-culture has undergone proinflammatory stimulation, therefore the cell, organoid or co-culture may be exposed to at least one proinflammatory stimulus, and then exposed to at least one inflammatory stimulus. Without wishing to be bound by any theory, the inventors consider that proinflammatory stimulation leads to adoption of a proinflammatory profile, which in turn leads to an inflammatory response (or an increased inflammatory response) when exposed to inflammatory stimuli.

Accordingly, the invention provides co-cultures exhibiting an inflammatory response, for example co-cultures prepared in media comprising at least one proinflammatory stimulus and/or at least one inflammatory stimulus, as described herein.

In some embodiments, the at least one inflammatory stimulus is applied to the co-culture such that the at least one inflammatory stimulus reaches a concentration of at least about 0.01 ng/ml, at least about 0.1 ng/ml, at least about 1 ng/ml, at least about 1.5 ng/ml, at least about 5 ng/ml, at least about 15 ng/ml, at least about 25 ng/mL, at least about 50 ng/mL, or at least about 100 ng/mL. Particularly preferred is a concentration of at least about 1.5 ng/ml, optionally at least about 15 ng/ml. These concentrations may refer to each inflammatory stimulus individually, or to all inflammatory stimuli collectively. Preferably, these concentrations refer to each inflammatory stimulus individually. Particularly preferred are inflammatory stimuli comprising at least about 15 ng/ml TNF and at least about 15 ng/ml IFNγ.

In some embodiments, the at least one inflammatory stimulus is applied to the co-culture such that the at least one inflammatory stimulus reaches a concentration of about 0.01 ng/ml, about 0.1 ng/ml, about 1 ng/ml, about 1.5 ng/ml, about 5 ng/ml, about 15 ng/ml, about 25 ng/mL, about 50 ng/mL, or about 100 ng/mL. Particularly preferred is a concentration of about 1.5 ng/ml, optionally about 15 ng/ml. These concentrations may refer to each inflammatory stimulus individually, or to all inflammatory stimuli collectively. Preferably, these concentrations refer to each inflammatory stimulus individually. Particularly preferred are inflammatory stimuli comprising about 15 ng/ml TNF and about 15 ng/ml IFNγ.

In some embodiments, the at least one inflammatory stimulus is applied to the co-culture such that the at least one inflammatory stimulus reaches a concentration of at most about 0.01 ng/ml, at most about 0.1 ng/ml, at most about 1 ng/ml, at most about 5 ng/ml, at most about 15 ng/ml, at most about 25 ng/mL, at most about 50 ng/mL, or at most about 100 ng/mL. Particularly preferred is a concentration of at most about 1.5 ng/ml, optionally at most about 15 ng/ml. These concentrations may refer to each inflammatory stimulus individually, or to all inflammatory stimuli collectively. Preferably, these concentrations refer to each inflammatory stimulus individually. Particularly preferred are inflammatory stimuli comprising at most about 15 ng/ml TNF and at most about 15 ng/ml IFNγ.

In some embodiments, at least one change is measured at least about 6 hours after an inflammatory stimulus is applied to the co-culture, optionally at least about 12 hours after, at least about 24 hours after, at least about 24 hours after, at least about 48 hours after, or at least about 72 hours after.

Thus, in some embodiments, determining the presence or absence of at least one change comprises applying at least one inflammatory stimulus to the co-culture and determining the presence or absence of an inflammatory response. In some embodiments, determining the presence or absence of at least one change comprises applying at least one proinflammatory stimulus to the co-culture, applying at least one inflammatory stimulus to the co-culture, and determining the presence or absence of an inflammatory response.

An inflammatory response may comprise increased caspase activity and/or apoptosis in the co-culture.

The presence or absence of an inflammatory response may be determined relative to a reference co-culture, optionally wherein the reference co-culture (a) does not receive the at least one proinflammatory stimulus, (b) does not receive the at least one inflammatory stimulus, and/or (c) is prepared by culturing stromal cells in stromal cell medium and combining stromal cells with at least one reference organoid.

In some embodiments, determining the presence or absence of at least one change comprises determining the presence or absence of an inflammatory response.

In some embodiments, the inflammatory stimulus is a therapeutic agent.

In some embodiments, the inflammatory stimulus is a diagnostic agent.

In some embodiments, determining the presence or absence of at least one change comprises determining the presence or absence of a proinflammatory profile.

In some embodiments, the inflammatory stimulus is not a therapeutic agent.

In some embodiments, the inflammatory stimulus is not a diagnostic agent.

In some embodiments, determining the presence or absence of at least one change comprises adding at least one inflammatory stimulus to the co-culture medium, and optionally determining the presence or absence of an inflammatory response.

In some embodiments, determining the presence or absence of at least one change comprises (a) adding at least one proinflammatory stimulus to the co-culture medium, and determining the presence or absence of a proinflammatory profile; and (b) adding at least one inflammatory stimulus to the co-culture medium, and optionally determining the presence or absence of an inflammatory response. These steps may be performed in the order listed.

### Disease

In some embodiments, the at least one organoid is a disease organoid, such as an organoid with an inflammatory disease phenotype and/or a fibrotic disease phenotype. In some embodiments, the organoid is derived from a subject with a disease, such as an inflammatory disease or a fibrotic disease.

Diseases with a stromal component (e.g. diseases in which stromal cells participate) may be investigated using co-cultures and methods of the invention, for example in the context of treatment, diagnosis and/or prognosis. In principle, any disorder that affects stromal cells may be investigated. Preferred diseases include diseases of the digestive and respiratory systems, especially the intestine and lungs. Exemplary diseases include irritable bowel disease (IBD), ulcerative colitis (UC), coeliac disease, leaky gut syndrome, chronic obstructive pulmonary disease (COPD), and asthma. Particularly preferred diseases include diseases of the digestive system, especially the intestine. Exemplary diseases include irritable bowel disease (IBD), ulcerative colitis (UC), coeliac disease, and leaky gut syndrome.

In some embodiments, at least one organoid may be cultured with at least one stromal cell from a subject with a disease to form a co-culture, and separately, at least one other organoid may be cultured with at least one stromal cell from a subject without the disease as a reference co-culture.

### Fibrosis

Disorders of particular interest include fibrotic disorders, such as intestinal fibrosis, solitary rectal ulcers, radiation enteropathy, and eosinophilic enteropathy. Owing to the presence of at least one stromal cell in the co-culture of the invention, the co-culture is particularly suitable for investigating the suitability of therapeutic agents for treating fibrotic diseases. In some embodiments, the at least one organoid and/or the at least one stromal cell are derived from fibrotic tissue.

### Inflammation

Disorders of particular interest include inflammatory disorders, such as inflammatory bowel disease (IBD), Crohn's disease (CD), and ulcerative colitis (UC). Owing to the ability to apply a proinflammatory stimulus to the co-culture of the invention, the co-culture is particularly suitable for investigating the suitability of therapeutic agents for treating inflammatory diseases. In some embodiments, the at least one organoid and/or the at least one stromal cell are derived from inflamed tissue.

### Therapeutics

The invention provides methods of testing therapeutic agents for treatment of diseases such as those disclosed elsewhere herein. Testing at least one therapeutic agent for a disease may comprise testing for tolerability and/or efficacy of the agent for treating a particular disease, testing tolerability and/or efficacy of the agent for treating a broad group of diseases, or testing tolerability and/or efficacy of the agent outside of the context of any particular disease (e.g. by testing activation of specific molecular pathways).

Therapeutic agents for a disease may include anti-inflammatory agents, such as small-molecule anti-inflammatory agents. Of particular interest are anti-inflammatory agents for treating IBD, UC and/or CD, including corticosteroids (such as prednisone), aminosalicylates (such as mesalamine, balsalazide, and olsalazine), immunosuppressants (such as azathioprine, mercaptopurine, and methotrexate), and anti-inflammatory agents (such as tofacitinib, upadacitinib and ozanimod), biologics (such as infliximab, adalimumab, golimumab, certolizumab, vedolizumab, ustekinumab, and risankizumab), antibiotics (such as ciprofloxacin and metronidazole).

The at least one therapeutic agent may comprise at least one proinflammatory stimulus, such as at least one cytokine, such as IL-1β and/or OSM.

The at least one therapeutic agent may comprise at least one inflammatory stimulus, such as at least one cytokine, such as TNF and/or IFNγ.

Testing a therapeutic agent may involve exposing the co-culture to therapeutic levels of a therapeutic with known or unknown efficacy and/or known or unknown tolerability.

Typically, an agent will be dissolved in solution to a (predicted) therapeutically effective concentration. The solution may be administered to the co-culture by injection (or other appropriate administration) into a vessel in which the co-culture is maintained.

In some embodiments, the agent is an approved or experimental drug, for example for a disease or the disorder of the digestive system, such as inflammatory bowel disease (e.g. Crohn's disease or ulcerative colitis), coeliac disease or leaky gut syndrome. In some embodiments, the agent is tofacitinib, preferably 10 µM tofacitinib.

Testing at least one therapeutic agent may comprise testing the efficacy of the agent for treating a disease. Testing at least one therapeutic agent may comprise testing the tolerability of the agent for treating a disease.

In some embodiments, the method comprises selecting a therapeutic agent based on the efficacy and/or tolerability determined in a method of the invention, and optionally using the therapeutic agent in a method of treatment by administering the agent to a subject.

In addition to testing therapeutic agents using co-cultures of the invention, co-cultures of the invention may be used in methods of identifying at least one target for at least one therapeutic agent. For example, a target gene in the at least one stromal cell and/or the at least one organoid may be genetically modified, and the presence or absence of at least one change in the co-culture may be determined to identify whether the target gene has therapeutic significance (e.g. whether the target gene affects the tolerability and/or efficacy of the at least one therapeutic agent), according to the general principles of determining the presence or absence of at least one change as disclosed elsewhere herein. The genetic modification may comprise insertion, deletion, or mutation of a gene in the genome of the at least one stromal cell. Target genes may include genes encoding cell surface receptors and cell signalling molecules such as cytokines. The presence or absence of at least one change may be determined in any co-culture of the invention comprising the genetic modification, relative to a reference co-culture not comprising the genetic modification. In such embodiments, the at least one therapeutic agent need not be applied to the co-culture. In other embodiments, the presence or absence of at least one change may be determined when the at least one therapeutic agent is applied to a co-culture comprising the genetic modification, compared to the co-culture before the at least one therapeutic agent was applied, and/or compared to a reference co-culture which does not comprise the genetic modification. Accordingly, the methods of testing therapeutic agents described herein may comprise identifying therapeutic targets.

### Personalised medicine

One means of testing therapeutic agents can be described as a `personalised medicine' approach to testing. A personalised medicine approach can involve testing one or more therapeutic agents that are known as suitable for treatment, and determining whether the one or more agents are suitable (e.g. effective and/or tolerable) for treatment of a disorder in the subject (i.e. a particular subject for whom the one or more therapeutic agents is tested). Personalised medicine approaches may use at least one organoid and/or at least one stromal cell derived from the same patient.

### Screening

Another means of testing therapeutic agents can be described as a `screening' approach to testing. A screening approach can involve testing one or more therapeutic agents for efficacy or tolerability in treatment of disease, for example in treatment of a specific disease, and determining whether one or more agents is suitable (e.g. effective and/or tolerable) for the treatment. Screening approaches may use at least one organoid and/or at least one stromal cell derived from different patients. Screening approaches may use at least one organoid and/or at least one stromal cell derived from immortalized cell lines. Screening approaches may use more than 1, for example more than 2, more than 3, more than 4, more than 5, more than 10, more than 20, more than 50, more than 100, or more than 1,000 pairs of at least one organoid and/or at least one stromal cell derived from different patients.

### Diagnostics

Because co-cultures and methods of the invention allow faithful modelling of *in vivo* physiology in an *in vitro* environment, they facilitate diagnosis or prognosis of disease by determining the presence or absence of at least one change (as described elsewhere herein). Diagnosis and/or prognosis may involve applying a diagnostic agent to the co-culture, for example a diagnostic agent that alters expression of a gene, activity of a signaling pathway, function of a receptor, function of a ligand, or alters other cellular process, thereby allowing differential diagnosis or prognosis based on the presence or absence of at least one change following administration.

Diagnosis and/or prognosis may comprise determining the presence or absence of at least one change following application of at least one diagnostic agent to the co-culture, wherein the at least one diagnostic agent comprises at least one agonist or antagonist of NF-κB, MAPK, and/or JAK-STAT pathways. Particularly preferred is at least one agonist or antagonist of the JAK-STAT pathway. Also of interest are modulators of fluid transport and/or ion channel activity.

Diagnosis and/or prognosis may comprise determining the presence or absence of at least one change following application of at least one diagnostic agent to the co-culture, wherein the at least one diagnostic agent comprises at least one cytokine, optionally wherein the at least one diagnostic agent comprises IL-1β or OSM.

The at least one diagnostic agent may comprise at least one proinflammatory stimulus, such as at least one cytokine, such as IL-1β and/or OSM.

The at least one diagnostic agent may comprise at least one inflammatory stimulus, such as at least one cytokine, such as TNF and/or IFNγ.

In some embodiments, the diagnostic agent is a therapeutic agent.

Diagnosis and/or prognosis may comprise determining the presence or absence of at least one change following application of at least one diagnostic agent to the co-culture, wherein the at least one diagnostic agent comprises at least one therapeutic agent, in particular a known therapeutic agent. The presence or absence of at least one change following administration of at least one therapeutic agent (such as a response in the co-culture indicating that the molecular basis for disease symptoms have or have not been treated) may assist in differential diagnosis by inference of the causes of disease.

Diagnosis and/or prognosis may comprise determining the presence or absence of at least one change in the co-culture, compared to a reference co-culture. For example, a co-culture may comprise at least one organoid derived from a first patient with a disease and/or at least one stromal cell from the first patient, and the presence or absence of at least one change may be determined relative to a reference co-culture comprising at least one organoid derived from a second patient without a disease and/or at least one stromal cell from the second patient.

Methods of diagnosis and/or prognosis may comprise analysing the presence or absence of at least one change, and identifying a disease or clinical outcome.

In some embodiments, the method comprises selecting a therapeutic agent for treating a disease based on the diagnosis and/or prognosis determined in the method of the invention, and optionally using the therapeutic agent in a method of treatment of the disease by administering the agent to a subject.

### Conditioned medium

Methods of the invention, particularly methods of testing at least one therapeutic agent and methods of diagnosis and/or prognosis, may involve combining at least one organoid with conditioned medium obtained from at least one stromal cell. Thus, any method involving the combining of at least one organoid and at least one stromal cell to form a co-culture, may instead involve combining at least one organoid with conditioned medium obtained from at least one stromal cell (i.e. not involving the formation of a co-culture). Without wishing to be bound by any theory, this is because the inventors believe that the effects of stromal cells on organoids is attributable at least in part to soluble factors secreted by stromal cells into their surrounding medium. Methods of the invention may comprise a step of isolating collecting conditioned medium from the co-culture and detecting soluble factors in the conditioned medium.

These methods involving conditioned medium may, but need not necessarily, involve a step of isolating conditioned medium from at least one stromal cell. Isolating the conditioned medium may occur at least about 6 hours after culturing the at least one stromal cell in a stromal cell medium, for example at least about 12 hours after, at least about 24 hours after, at least about 48 hours after, or at least about 72 hours after.

Accordingly, the invention provides organoids exhibiting a proinflammatory profile, for example organoids prepared in conditioned media derived from stromal cells, wherein the stromal cells are prepared in stromal cell medium comprising at least one proinflammatory stimulus.

### Additional Methods and Products of the Invention

### Kits

The invention provides kits comprising any organoid, stromal cell or co-culture of the invention.

In some embodiments, the kit comprises one or more of the following: syringe, alcohol swab, cotton ball, gauze pad, instructions for performing the methods of the invention.

### EMBODIMENTS

The invention further provides the following numbered embodiments:
1. A method of preparing a co-culture comprising at least one organoid and at least one stromal cell, the method comprising:
   combining the at least one organoid with the at least one stromal cell in a co-culture medium to form the co-culture,
   optionally wherein:
   (a) the method comprises preparing the at least one stromal cell by culturing stromal cells in a stromal cell medium; and/or
   (b) the method comprises preparing the at least one organoid by culturing epithelial cells in an organoid medium.
2. A method for determining the presence or absence of at least one change in a co-culture comprising at least one organoid and at least one stromal cell, wherein the method comprises:
   combining the at least one organoid with the at least one stromal cell in a co-culture medium to form the co-culture; and
   determining the presence or absence of the at least one change in the co-culture,
   optionally wherein:
   (a) the method comprises preparing the at least one stromal cell by culturing stromal cells in a stromal cell medium; and/or
   (b) the method comprises preparing the at least one organoid by culturing epithelial cells in an organoid medium.
3. A method for determining the presence or absence of at least one change in at least one organoid, wherein the method comprises:
   isolating conditioned medium from at least one stromal cell, and combining the conditioned medium with at least one organoid; and
   determining the presence or absence of at least one change in the organoid,
   optionally wherein:
   (a) the method comprises preparing the at least one stromal cell by culturing stromal cells in a stromal cell medium; and/or
   (b) the method comprises preparing the at least one organoid by culturing epithelial cells in an organoid medium.
4. A method of testing at least one therapeutic agent for a disease, the method comprising:
   combining at least one organoid with at least one stromal cell in a co-culture medium to form a co-culture;
   applying the at least one therapeutic agent to the co-culture; and
   determining the presence or absence of at least one change in the co-culture,
   optionally wherein:
   (a) the method comprises preparing the at least one stromal cell by culturing stromal cells in a stromal cell medium; and/or
   (b) the method comprises preparing the at least one organoid by culturing epithelial cells in an organoid medium.
5. A method of testing at least one therapeutic agent for a disease, the method comprising:
   isolating conditioned medium from at least one stromal cell, and combining the conditioned medium with at least one organoid;
   applying the at least one therapeutic agent to the organoid; and
   determining the presence or absence of at least one change in the organoid,
   optionally wherein:
   (a) the method comprises preparing the at least one stromal cell by culturing stromal cells in a stromal cell medium; and/or
   (b) the method comprises preparing the at least one organoid by culturing epithelial cells in an organoid medium.
6. The method of embodiment 4 or embodiment 5, wherein testing the at least one therapeutic agent comprises testing the tolerability of the at least one therapeutic agent.
7. The method of any one of embodiments 2-6, wherein the presence or absence of the at least one change is determined relative to a reference organoid, optionally wherein the at least one therapeutic agent is not applied to the reference organoid and/or wherein the reference organoid does not comprise stromal cells.
8. The method of any one of embodiments 2-7, wherein the presence or absence of the at least one change is determined relative to a reference co-culture, optionally wherein the at least one therapeutic agent is not applied to the reference co-culture.
9. A method of determining the presence or absence of a diagnosis and/or prognosis of a disease in a subject, the method comprising:
   combining at least one organoid with at least one stromal cell in a co-culture medium to form a co-culture;
   applying at least one diagnostic agent to the co-culture; and
   determining the presence or absence of at least one change in the co-culture,
   optionally wherein:
   (a) the method comprises preparing the at least one stromal cell by culturing stromal cells in a stromal cell medium; and/or
   (b) the method comprises preparing the at least one organoid by culturing epithelial cells in an organoid medium.
10. A method of determining the presence or absence of a diagnosis and/or prognosis of a disease in a subject, the method comprising:
   isolating conditioned medium from at least one stromal cell, and combining the conditioned medium with at least one organoid;
   applying at least one diagnostic agent to the at least one organoid; and
   determining the presence or absence of at least one change in the at least one organoid,
   optionally wherein:
   (a) the method comprises preparing the at least one stromal cell by culturing stromal cells in a stromal cell medium; and/or
   (b) the method comprises preparing the at least one organoid by culturing epithelial cells in an organoid medium.
11. The method of embodiment 9 or embodiment 10, wherein the at least one diagnostic agent is an agonist or an antagonist of the IL-1β and/or OSM pathways, optionally wherein the at least one diagnostic agent is a cytokine, optionally wherein the at least one diagnostic agent comprises IL-1β and/or OSM.
12. The method of any one of embodiments 3, 5, or 10, wherein isolating the conditioned medium occurs at least about 6 hours after preparing the at least one stromal cell.
13. The method of any one of embodiments 2, 4, 6-9, or 11, wherein the presence or absence of the at least one change is determined relative to the at least one organoid prior to forming the co-culture.
14. The method of any one of embodiments 4-13, wherein the disease is an inflammatory disease or a fibrotic disease.
15. The method of embodiment 14, wherein the disease is an inflammatory disease, optionally wherein the inflammatory disease is a gastrointestinal inflammatory disease, optionally wherein the inflammatory disease is inflammatory bowel disease (IBD), optionally wherein the IBD comprises ulcerative colitis (UC) or Crohn's disease (CD).
16. The method of any preceding embodiment, wherein the at least one organoid is derived from primary epithelial cells or immortalized epithelial cells.
17. The method of any preceding embodiment, wherein the at least one organoid is a patient-derived organoid.
18. The method of any preceding embodiment, wherein the epithelial cells are primary epithelial cells or immortalized epithelial cells, optionally wherein the primary epithelial cells are patient-derived epithelial cells.
19. The method of any preceding embodiment, wherein the at least one stromal cell comprises a fibroblast cell, optionally wherein the fibroblast cell is an intestinal fibroblast cell, for example a colon fibroblast or small intestine fibroblast, optionally wherein the fibroblast cell is an immortalized human colon fibroblast, a human colon fibroblast, an immortalized human small intestine fibroblast, or a human small intestine fibroblast.
20. The method of any one of embodiments 4-19, wherein the co-culture, the at least one organoid, and/or the at least one stromal cell:
   (a) is derived from the subject; and/or
   (b) is derived from lung tissue, kidney tissue, pancreatic tissue, or liver tissue; and/or
   (c) is derived from fibrotic tissue; and/or
   (d) is derived from inflamed tissue; and/or
   (e) comprises or consist of mammalian cells, optionally human cells; and/or
   (f) is derived from the intestines, optionally from the colon.
21. The method of any preceding embodiment, wherein the at least one organoid does not comprise epithelial cells derived from lung tissue, kidney tissue, pancreatic tissue, or liver tissue, optionally wherein the at least one organoid does not comprise epithelial cells or stromal cells derived from lung tissue, kidney tissue, pancreatic tissue, or liver tissue.
22. The method of any one of embodiments 4-21, wherein the epithelial cells and/or the stromal cells are:
   (a) derived from the subject; and/or
   (b) derived from lung tissue, kidney tissue, pancreatic tissue, or liver tissue; and/or
   (c) derived from fibrotic tissue; and/or
   (d) mammalian cells, optionally human cells; and/or
   (e) derived from the intestines, optionally from the colon.
23. The method of any preceding embodiment, wherein the at least one organoid and the at least one stromal cell are derived from the same subject, optionally from (a) the same sample from the subject and/or (b) the same tissue in the same subject.
24. The method of any preceding embodiment, wherein the epithelial cells and the stromal cells are derived from the same subject, optionally from (a) the same sample from the subject and/or (b) the same tissue in the same subject.
25. The method of embodiment 23 or embodiment 24, wherein the sample comprises a tissue biopsy, optionally wherein the sample comprises an intestinal tissue biopsy.
26. The method of any one of embodiments 2, 4, 6-9, 11, or 13-25, wherein determining the presence or absence of at least one change in the co-culture comprises determining the presence or absence of stromal cells in the co-culture, optionally determining the quantity of stromal cells in the co-culture.
27. The method of any preceding embodiment, wherein the at least one organoid is prepared by splitting a precursor organoid.
28. The method of any one of embodiments 2, 4, 6-9, 11, or 13-27, wherein combining the at least one organoid with the at least one stromal cell comprises seeding the at least one organoid and the at least one stromal cell simultaneously.
29. The method of any one of embodiments 2, 4, 6-9, 11, or 13-28, wherein the at least one organoid and the at least one stromal cell are combined at a ratio of from about 0.5 to about 2.5 stromal cells seeded per organoid, optionally wherein the ratio is about 0.5 stromal cells seeded per organoid, or about 2.5 stromal cells seeded per organoid.
30. The method of any preceding embodiment, wherein the epithelial cells are cultured in the organoid medium for at least about 24 hours to prepare the at least one organoid.
31. The method of any one of embodiments 2, 4, 6-9, 11, or 13-30, wherein the co-culture is a three-dimensional co-culture, optionally wherein combining the at least one organoid and at least one stromal cell comprises a hanging drop method.
32. The method of any one of embodiments 2, 4, 6-9, 11, or 13-31, wherein the at least one organoid and the at least one stromal cell are combined in a multi-well plate format, optionally wherein the multi-well plate comprises an ultra-low attachment plate.
33. The method of any one of embodiments 2, 4, 6-9, or 11-32, comprising isolating conditioned medium from the co-culture and detecting soluble factors in the conditioned medium.
34. The method of any preceding embodiment, wherein the at least one stromal cell is modified to alter expression of a gene.
35. The method of any preceding embodiment, wherein the epithelial cells are epithelial stem cells.
36. The method of any preceding embodiment, wherein the at least one organoid is characterised by Lgr5 expression.
37. The method of any one of embodiments 2, 4, 6-9, 11, or 13-36, wherein determining the presence or absence of at least one change comprises adding at least one inflammatory stimulus to the co-culture mnedium and determining the presence or absence of an inflammatory response.
38. The method of embodiment 37, wherein the at least one inflammatory stimulus comprises a cytokine, optionally wherein the cytokine comprises TNF and/or IFNγ.
39. The method of embodiment 37 or 38, wherein the inflammatory response comprises increased caspase activity and/or apoptosis in the co-culture.
40. The method of any one of embodiments 37-39, wherein the presence or absence of an inflammatory response is determined relative to a reference co-culture, optionally wherein the reference co-culture (a) does not receive the at least one inflammatory stimulus and/or (b) is prepared by culturing stromal cells in stromal cell expansion medium and combining stromal cells with at least one reference organoid.
41. The method of any one of embodiments 2-40, wherein the at least one change comprises a change in organoid morphology, a change in organoid size or epithelial cell size, a reduction in cell viability, a reduction in cell proliferation, an increase in cell death, a change in secretome profile, a change in cytokine secretion, an increase in cell apoptosis, an increase in caspase activity, and/or a change in the expression of one or more genes, optionally wherein the change in the expression of one or more genes comprises a change in one or more disease biomarkers, one or more fibrosis biomarkers, and/or one or more inflammation biomarkers.
42. The method of any one of embodiments 2-41, wherein determining the presence or absence of at least one change comprises a cellular proliferation assay, a viability assay, flow cytometric analysis, ELISA, analysis of gene expression, and/or cellular imaging.
43. The method of any one of embodiments 2-42, wherein determining the presence or absence of the at least one change comprises determining the extent of death of organoid cells.
44. The method of any one of embodiments 2-43, wherein the at least one change comprises a change in organoid morphology, optionally wherein the change in morphology comprises:
   (a) increased organoid area; and/or
   (b) increased organoid aggregation; and/or
   (c) increased lumen size; and/or
   (d) increased epithelial cell size; and/or
   (e) reduced organoid-surface adherence.
45. The method of any one of embodiments 2-44, where the at least one change comprises increased area of the at least one organoid, optionally wherein the area is increased to at least about 10,000 µm², at least about 15,000 µm², at least about 20,000 µm², or at least about 25,000 µm².
46. The method of embodiment 44 or embodiment 45, wherein the area of the at least one organoid is increased by at least about 10%, at least about 20%, at least about 30%, at least about 40%, at least about 50%, at least about 60%, at least about 70%, or at least about 80%.
47. The method of any one of embodiments 2, 4, 6-9, 11, or 13-46, wherein the at least one change is determined at least about 1 day, at least about 2 days, at least about 3 days, at least about 4 days, at least about 5 days, at least about 6 days, or at least about 7 days, after the at least one organoid and the at least one stromal cell are combined to form the co-culture.
48. The method of any one of embodiments 2-47, wherein the at least one change comprises upregulation of expression of a stem cell marker, optionally wherein the stem cell marker is OLFM4, optionally wherein the upregulation is by at least about 10%, at least about 50%, at least about 100%, at least about 200%, at least about 300%, at least about 400%, at least about 500%, or at least about 1,000%.
49. The method of any one of embodiments 2-48, wherein the at least one change comprises downregulation of expression of an enterocyte marker, optionally wherein the enterocyte marker is ALPI, optionally wherein the downregulation is by at least about 10%, at least about 20%, at least about 30%, at least about 40%, at least about 50%, at least about 60%, at least about 70%, at least about 80%, or at least about 90%.
50. The method of any one of embodiments 2-49, wherein the at least one change comprises downregulation of expression of a proliferation marker, optionally wherein the proliferation marker is KI67, optionally wherein the downregulation is by at least about 10%, at least about 20%, at least about 30%, at least about 40%, at least about 50%, at least about 60%, at least about 70%, at least about 80%, or at least about 90%.
51. The method of any one of embodiments 2-50, wherein the at least one change comprises a decrease in epithelial thickness, optionally wherein the decrease is by at least about 10%, at least about 20%, at least about 30%, at least about 40%, at least about 50%, at least about 60%, at least about 70%, at least about 80%, or at least about 90%.
52. The method of any one of embodiments 2, 4, 6-9, 11, or 13-51, wherein the co-culture is cultured for at least about 12 hours, at least about 24 hours, at least about 48 hours, at least about 72 hours, at least about 96 hours, or at least about 120 hours, prior to determining the presence or absence of at least one change in the co-culture.
53. The method of any one of embodiments 2, 4, 6-9, 11, or 13-52, wherein the presence or absence of the at least one change is determined after the at least one organoid and at least one stromal cell are combined to form the co-culture, relative to before the at least one organoid and at least one stromal cell are combined to form the co-culture.
54. The method of any one of embodiments 2-53, wherein the at least one change comprises a change in secretome profile, optionally a change in secretion of a cytokine, optionally a change in secretion of IL-6 and/or CXCL2.
55. The method of any one of embodiments 2-54, wherein the at least one change comprises expression of at least about 10,000 pg/ml of IL-6, at least about 20,000 pg/ml of IL-6, at least about 30,000 pg/ml of IL-6, at least about 40,000 pg/ml of IL-6, at least about 50,000 pg/ml of IL-6, or at least about 100,000 pg/ml of IL-6.
56. The method of any one of embodiments 2-55, wherein the at least one change comprises a change in caspase activity, optionally comprising a change in caspase 3/7 activity, optionally wherein the caspase activity is increased by at least about 500%, at least about 1,000%, at least about 1,500%, at least about 2,000%, or at least about 2,500%.
57. The method of any one of embodiments 2, 4, 6-9, 11, or 13-56, wherein at least one inflammatory stimulus is added to the co-culture medium prior to determining the presence or absence of at least one change in the co-culture, optionally wherein the at least one inflammatory stimulus comprises a cytokine, optionally wherein the cytokine comprises TNF and/or IFNγ, optionally wherein the cytokine is at a concentration of at most about 100 ng/ml, at most about 50 ng/ml, at most about 15 ng/ml, at most about 5 ng/ml, at most about 1 ng/ml, at most about 0.1 ng/ml, or at most about 0.01 ng/ml.
58. The method of any one of embodiments 2, 4, 6-9, 11, or 13-57, wherein a cytokine signalling inhibitor is added to the co-culture prior to determining the presence or absence of at least one change in the co-culture, optionally wherein the cytokine signalling inhibitor reduces activity of the JAK/STAT pathway, optionally wherein the cytokine signalling inhibitor is tofacitinib.
59. The method of any preceding embodiment, wherein the organoid medium comprises a mitogenic growth factor, a BMP inhibitor, and an R-spondin, optionally wherein the organoid medium comprises a Wnt agonist, a mitogenic growth factor, a BMP inhibitor, and an R-spondin.
60. The method of any preceding embodiment, wherein the stromal cell medium comprises a basal medium, an amino acid supplement, an antibiotic, and optionally a serum.
61. The method of any preceding embodiment, wherein the organoid medium and/or stromal cell medium comprises an extracellular matrix, optionally wherein the extracellular matrix is Matrigel^{®}, optionally wherein the Matrigel^{®} is 5% or 10% Matrigel^{®}, optionally wherein the Matrigel^{®} is 5% Matrigel.
62. The method of any one of embodiments 2, 4, 6-9, 11, or 13-61, wherein the co-culture medium comprises:
   (A) a mitogenic growth factor, a BMP inhibitor, and an R-spondin, optionally wherein the co-culture medium comprises a Wnt agonist, a mitogenic growth factor, a BMP inhibitor, and an R-spondin, optionally wherein the co-culture medium comprises N-Ac, a TGF-β inhibitor, a mitogenic growth factor, gastrin, a BMP inhibitor, an antibiotic, an R-spondin, a Notch pathway inhibitor, an ERK inhibitor, and a Wnt agonist; and/or
   (B) a serum, optionally fetal calf serum, optionally 5% fetal calf serum.
63. The method of any one of embodiments 2, 4, 6-9, 11, or 13-62, wherein the organoid medium and/or co-culture medium does not comprise an extracellular matrix.
64. The method of any one of embodiments 2, 4, 6-9, 11, or 13-63, wherein determining the presence or absence of at least one change comprises adding at least one proinflammatory stimulus to the co-culture medium, optionally wherein determining the presence or absence of at least one change comprises (a) adding at least one proinflammatory stimulus to the co-culture medium, determining the presence or absence of a proinflammatory profile, and/or (b) adding at least one inflammatory stimulus to the co-culture medium, and determining the presence or absence of an inflammatory response.
65. The method of embodiment 64, wherein the at least one proinflammatory stimulus is added to the co-culture medium prior to the addition of any inflammatory stimulus to the co-culture medium, optionally at least about 6 hours, at least about 12 hours, at least about 24 hours, at least about 48 hours, at least about 72 hours, or at least about 96 hours, prior to the addition of any inflammatory stimulus to the co-culture medium.
66. The method of any one of embodiments 37-40, 57, or 64-65, wherein at least one proinflammatory stimulus is added to the co-culture medium before the at least one inflammatory stimulus is added to the co-culture medium, optionally wherein the at least one proinflammatory stimulus is added to the co-culture medium at least about 6 hours, at least about 12 hours, at least about 24 hours, at least about 48 hours, at least about 72 hours, or at least about 96 hours, before the at least one inflammatory stimulus is added to the co-culture medium.
67. The method of any one of embodiments 64-66, wherein the at least one proinflammatory stimulus comprises at least one cytokine.
68. The method of embodiment 67, wherein the at least one cytokine comprises IL-1β and/or oncostatin M (OSM).
69. The method of any one of embodiments 64-68, wherein the at least one proinflammatory stimulus is added to the organoid co-culture medium at two intervals separated by at least about 24 hours, at least about 48 hours, at least about 72 hours, or at least about 96 hours.
70. The method of any one of embodiments 64-69, wherein the at least one proinflammatory stimulus comprises IL-1β, optionally wherein the concentration of IL-1β is at least about 20 ng/ml, optionally wherein the concentration is at least about 5 ng/ml, at least about 1 ng/ml, at least about 0.1 ng/ml, or at least about 0.01 ng/ml, preferably wherein the concentration is at least about 1 ng/ml.
71. The method of any one of embodiments 64-70, wherein the at least one proinflammatory stimulus comprises IL-1β, optionally wherein the concentration of IL-1β is at most about 20 ng/ml, optionally wherein the concentration is at most about 5 ng/ml, at most about 1 ng/ml, at least about 0.1 ng/ml, or at least about 0.01 ng/ml preferably wherein the concentration is at most about 1 ng/ml.
72. The method of any one of embodiments 64-71, wherein the at least one proinflammatory stimulus comprises IL-1β, optionally wherein the concentration of IL-1β is from about 0.01 ng/ml to about 20 ng/ml, optionally wherein the concentration is from about 0.1 ng/ml to about 20 ng/ml, from about 1 ng/ml to about 20 ng/ml, or from about 1 ng/ml to about 5 ng/ml, optionally wherein the concentration is about 1 ng/ml.
73. The method of any one of embodiments 64-69, wherein the at least one proinflammatory stimulus comprises OSM, optionally wherein the concentration of OSM is at least about 20 ng/ml, optionally wherein the concentration is at least about 5 ng/ml, at least about 1 ng/ml, at least about 0.1 ng/ml, or at least about 0.01 ng/ml, preferably wherein the concentration is at least about 1 ng/ml.
74. The method of any one of embodiments 64-69 or 73, wherein the at least one proinflammatory stimulus comprises OSM, optionally wherein the concentration of OSM is at most about 20 ng/ml, optionally wherein the concentration is at most about 5 ng/ml, at most about 1 ng/ml, at least about 0.1 ng/ml, or at least about 0.01 ng/ml.
75. The method of any one of embodiments 64-69 or 73-74, wherein the at least one proinflammatory stimulus comprises OSM, optionally wherein the concentration of OSM is from about 0.01 ng/ml to about 20 ng/ml, optionally wherein the concentration is from about 0.1 ng/ml to about 20 ng/ml, from about 1 ng/ml to about 20 ng/ml or from about 1 ng/ml to about 5 ng/ml, optionally wherein the concentration is about 1 ng/ml.
76. The method of any one of any one of embodiments 64-75, wherein the at least one proinflammatory stimulus comprises IL-1β and OSM, optionally wherein the at least one proinflammatory stimulus comprises at least about 1 ng/ml IL-1β and at least about 1 ng/ml OSM.
77. A co-culture as recited in any one of embodiments 2, 4, 6-9, 11, or 13-76.
78. A co-culture medium as recited in any one of embodiments 2, 4, 6-9, 11, or 13-76.
79. A stromal cell medium as recited in any preceding claim.
80. A co-culture substantially as herein described.
81. A co-culture medium substantially as herein described.
82. A stromal cell medium as substantially herein described.

### EXAMPLES

Other features, objects, and advantages of the present invention are apparent in the examples that follow. It should be understood, however, that the examples, while indicating embodiments of the present invention, are given by way of illustration only, not limitation. Various changes and modifications within the scope of the invention will become apparent to those skilled in the art from the examples.

All patent and literature references cited in the present specification are hereby incorporated by reference in their entirety.

### Media

The following media are used in the Examples. Quantities of reagents are given in the stated units, or as a percentage of the final volume of the medium (or % v/v), or as a multiple according to manufacturer's instructions (e.g. "1x").

**Ad-DF+++**

| **Reagent** | **Quantity** |
|---|---|
| Completed Advanced DMEM/F12 medium (Gibco^{™}), "Ad-DF" | 500 ml (basal medium) |
| GlutaMax | 2 mM |
| HEPES | 10 mM |
| Penicillin and Streptomycin | 100 units/mL |

**Ad-DF+++ modified with additional HEPES**

| **Reagent** | **Quantity** |
|---|---|
| Completed Advanced DMEM/F12 medium (Gibco^{™}), "Ad-DF" | 500 ml (basal medium) |
| GlutaMax | 2 mM |
| HEPES | 25 mM |
| Penicillin and Streptomycin | 100 units/mL |

**Organoid expansion media (or "colon normal medium", "CNM")**

| **Reagent** | **Quantity** |
|---|---|
| Completed Advanced DMEM/F12 medium (Gibco^{™}), "Ad-DF" | N/A (basal medium) |
| N-acetylcysteine (Sigma-Aldrich) ("N-Ac") | 1.25 mM |
| TGF-β inhibitor A-83-01 (Tocris) | 500 nM |
| B27 Supplement (Gibco) | 1x |
| EGF | 50 ng/ml |
| Gastrin | 5 nM |
| Noggin-conditioned medium ("NCM", Immunoprecise, catalog number N002 (https://products.ipatherapeutics.com/product/n002-200ml/) | 1-4% |
| Nicotinamide | 10 mM |
| Primocin | 50 µg/ml |
| Rspo3 | 250 ng/ml |
| SB202190 (p38i) | 10 µM |
| Wnt3a conditioned medium or NGS Wnt | 50% or 0.5 nM |

**Co-culture differentiation medium ("combined-colon differentiation medium", "cCDM")**

| **Reagent** | **Quantity** |
|---|---|
| Completed Advanced DMEM/F 12 medium (Gibco^{™}), "Ad-DF" | N/A (basal medium) |
| N-acetylcysteine (Sigma-Aldrich) ("N-Ac") | 1.25 mM |
| TGF-β inhibitor A-83-01 (Tocris) | 500 nM |
| B27 Supplement (Gibco) | 1x |
| EGF | 50 ng/ml |
| Gastrin | 5 nM |
| NCM (Immunoprecise, catalog number N002 (https://products.ipatherapeutics.com/product/n002-200ml/) | 1-2% |
| Primocin | 50 µg/ml |
| Rspo3 | 250 ng/ml |
| DAPT | 10 µM |
| PD0325901 | 100 nM |
| Wnt3a conditioned medium or NGS Wnt | 10% |

**cCDM + serum**

| **Reagent** | **Quantity** |
|---|---|
| cCDM | 1x |
| FBS | 5% |
| Matrigel | 5% |

Where incubation was performed, this is at 37 °C and 5% CO₂, overnight, unless indicated otherwise. Incubation may be performed for longer period, refreshing media every 2-3 days.

Where indicated, experiments involved fibroblast cell lines shown in Table 1:

| **Fibroblast line** | **Code** | **Organ** | **Origin** |
|---|---|---|---|
| Immortalized Human Colon | Clone 2 | Colon | Immortalized |
| Immortalized Human Small Intestine Fibroblasts | IM-SIF | Small Intestine | Immortalized |
| Human Small Intestine Fibroblasts | SIF | Small Intestine | Primary |
| Immortalized Human Colon Fibroblasts | IM-CoF | Colon | Immortalized |
| Human Colon Fibroblasts | CoF | Colon | Primary |

### Example 1: Preparation of fibroblasts and organoids

This example provides exemplary protocols used for the preparation of fibroblasts and organoids, for subsequent use in **Example 2.**

### Fibroblast preparation

Fibroblast samples stored at -80°C were thawed and refreshed using Ad-DF+++ media with 10% FBS and 25 mM HEPES. Fibroblast cells were then transferred to a T75 flask coated with poly-L lysine, and incubated.

To trypsinize the fibroblasts, media was aspirated and cells were washed with 10 mL DPBS (Dulbecco's phosphate buffered saline). The DPBS was then aspirated. 3 mL TrypLE was added to the flask before rocking to ensure complete coverage. The flask was then incubated at 37°C for 2 min.

The TrypLE with fibroblast cells was then transferred to fresh Ad-DF+++ media (5 ml Ad-DF+++ per 1 ml TrypLE) for neutralization, before centrifugation at 1500 rpm (450 x g) for 5 minutes. Finally, the supernatant was aspirated and cells were re-suspended in 1 mL Ad-DF+++ media and counted.

### Organoid preparation

Biopsies from human colon tissue were collected in 50 mL canonical tubes containing ice-cold 10-15 mL Advanced DMEM/F12 medium completed with Penicillin/Streptomycin (from 100× stock at 10,000 U/mL Penicillin and 10K µM/mL Streptomycin), HEPES (from 100× stock at 1M), GlutaMAX (from 100× stock; all Gibco^{™}) and Rho kinase inhibitor Y-27632 (Sigma-Aldrich). Biopsies were kept on ice and immediately processed or can be stored for up to 24 h at 4°C until start of isolation.

Colon mucosa is treated with EDTA to liberate the crypts for derivation of colon organoid. Muscle layer and fat are removed under a dissection microscope using surgical scissors and forceps. Cleaned tissue is cut into thin strips of approximately 1-2 mm. One strip is fixed in 4% formaldehyde (Sigma-Aldrich) for histological analysis and one strip is snap frozen (in dry ice or liquid nitrogen) and stored at -80°C for gene and/or protein analysis.

Remaining strips were washed 3 times with fresh chelation solution (5.6 mM Na₂ HPO₄, 8.0 mM KH₂PO₄, 96.2 mM NaCl, 1.6 mM KCl, 43.4 mM sucrose, and 54.9 mM D-sorbitol dissolved in sterile water; all Sigma-Aldrich). Washed strips were incubated in chelation solution completed with 2 mM ethylenediaminetetraacetic acid (EDTA; in-house) and 0.5 mM DL-dithiotreitol (DTT; Sigma-Aldrich) for 30 minutes at 4°C in a rotating wheel (cold room).

Tubes were vigorously shaken to liberate the colonic crypts out of the mesenchyme. If no crypts were visible, the incubation was repeated with fresh completed chelation solution. Tissue fragments were allowed to settle for 1-2 minutes and the supernatant containing the crypts was transferred to a new tube. 5-10 mL foetal calf serum (FCS; Sigma-Aldrich) was added and the crypts were centrifuged at 300×g for 5 minutes at 4°C.

Crypts were washed 3 times in completed Advanced DMEM/F 12. Crypts were resuspended in a mixture of Matrigel/BME and media (70%), plated at different densities in domes, and placed for 30 minutes into a humidified incubator at 37°C and 5% CO₂. Upon Matrigel/BME solidification, CNM supplemented with Rho kinase inhibitor Y-27632 was added. Organoids forming from the crypts were passaged every 7-10 days.

Subsequently, organoid cultures may undergo primary analysis using whole-genome sequencing, mRNA sequencing and peptidome profiling.

### Splitting organoids

Organoid cultures were disrupted ('split') by pipetting Matrigel/BME drops up and down the growth medium using a 1 mL volume micropipette (P1000 Gilson). Disrupted organoids were centrifuged at 500×g for 5 minutes. Pelleted organoids were taken up in an excess of complete Advanced DMEM/F12 and centrifuged at 500×g for 5 minutes. Organoid fragments were re-plated in 70% Matrigel in CNM with Rho kinase inhibitor Y-27632 (1/1000) at a desired density and placed in humidified incubator at 37°C and 5% CO₂.

Medium was refreshed every 3-4 days. Organoids were passaged every 7-10 days.

### Collection of organoids

20 µL 100x Dispase solution was added to each well of a 6-well plate (2 ml) containing the organoids prepared above, before incubating the plate at 37°C and 5% CO₂ for 30 minutes. Organoids were collected from all wells by pipetting through a 100 µm filter (pre-wet twice using 10 mL Ad-DF+++ media) using a P1000 pipette, into a 50 mL plastic tube. The 100 µm filter containing the organoids was then washed twice using 10 mL Ad-DF+++ media. The flow-through was filtered through a 20 µm filter (pre-wet twice using 10 mL Ad-DF+++ media). The 20 µm filter (containing the organoids) was inverted and washed using Ad-DF+++ media (four times flow-through volume) supplemented with Rho kinase inhibitor Y-27632 and the flow through centrifuged at 1500 rpm (450 × g) for 3 min at 8°C.

Finally, the supernatant was carefully aspirated and the organoid pellet re-suspended in 1 mL of cCDM + serum per full 6-well plate before counting.

### Example 2: Forming the co-culture

Organoid and fibroblast cultures prepared as described in **Example 1** were placed on ice. Organoids and fibroblasts were mixed in cCDM containing 5% serum and 5% Matrigel and Rho kinase inhibitor Y-27632. Solutions were kept cold and 100-200 µl were dispense to each well of a 96-well plate.

Co-cultures were liberated from BME using Cell Recovery Solution and fixed in 4% paraformaldehyde. Fixed whole-mounts were stained with Phalloidin to mark polymerised actin and DAPI to label nuclei. Whole-mounts were mounted onto slide in ProLong Gold anti-fade mounting medium and imaged on a Leica SP8X confocal microscope (data not shown).

### Example 3: Inducing a proinflammatory profile in co-cultures

Co-cultures prepared as described in **Examples 1-2** were induced to exhibit a proinflammatory profile by exposure to proinflammatory stimuli. **Figure 1** shows a schematic of the procedure for this and subsequent Examples.

Specifically, stock solutions of OSM and IL-1β were diluted with 0.6% Tween in PBS, 1:1, to reach a concentration of 1 ng/mL each. OSM and IL-1β solutions were added together to co-cultures using a Tecan D300, and incubated at 37°C and 5% CO₂ for 72 hours.

In Examples 4-9, fibroblast cultures, organoid cultures, and co-cultures of the fibroblasts and organoids, were prepared according to **Examples 1-2.** OSM (1 ng/mL) and IL-1β (1 ng/mL) were added to the co-cultures according to **Example 3** where "proinflammatory stimuli" or "proinflammatory stimulation" are specified in **Examples 4-9.** Co-cultures were then incubated on plates for 72 hours at 37°C and 5% CO₂ prior to cytokine or caspase analysis, or for 96 hours at 37°C and 5% CO₂ prior to imaging analysis.

### Example 4: Imaging shows increased organoid size when co-cultures are prepared with proinflammatory stimulation

This Example uses imaging analysis to assess the morphology of co-cultured organoids. It shows that organoids and fibroblasts could be successfully co-cultured and display normal organoid morphology consistent with organoids being cultured in cCDM (e.g. expected size, area and appearance under brightfield imaging), and exhibit increased aggregation due to cell-cell interactions mediated by fibroblasts.

This Example also shows that proinflammatory stimulation of organoids causes an increase in the area of organoids and affects aggregation (relative to co-cultures without proinflammatory stimulation), suggesting that organoid size is an indicator of the proinflammatory profile of a co-culture.

Organoids in co-culture were first stained with Calcein AM. 48 hours after the organoids and fibroblasts were combined in co-culture, 50 µL DMSO was added to a vial of Calcein AM and mixed. 88 µL of the mixture was added to 4,312 µL of Ad-DF+++ media. 10 µL of the final Calcein AM mixture was added to co-cultures before 5-10 seconds of shaking in a Tecan D300. Plates were then incubated for 2 hours at 37°C and 5% CO₂. Imaging were then performed to assess organoid morphology.

As shown in Figure 2A, co-cultures exposed to proinflammatory stimuli exhibited increased cystic morphology compared to the small, compact morphology of organoids cultured alone, or co-cultures not exposed to proinflammatory stimuli. Aggregation was observed when organoids were co-cultured with fibroblasts, compared to organoids not co-cultured with fibroblasts. Aggregation is less pronounced when co-cultures are prepared with proinflammatory stimuli.

As shown in **Figure 2B****,** organoid area increases significantly in co-cultures with a proinflammatory profile.

As shown in **Figure 2C****,** exposure to alternative stimuli such as IL-6 (20 ng/mL), LIGHT (20 ng/mL), or a combination of both, did not cause a change in organoid size.

As shown in **Figure 2D and 2E****,** similar results were obtained using alternative primary organoids "A", "B" and "C" (each derived from small intestine).

As shown in **Figure 2F****,** similar results were obtained when testing the immortalized and primary fibroblast cell lines shown above in **Table 1,** in the absence ("-") and presence ("+") of proinflammatory stimulation.

As shown in **Figure 2G and 2H****,** similar results were obtained when the duration of culture was extended to day 3 ("D3") and day 6 ("D6"), for different ratios of fibroblast cells to organoids ("F/O").

### Example 5: Expression measurements show increased secretion of markers indicative of a proinflammatory profile, when co-cultures are prepared with proinflammatory stimuli

Plate contents were centrifuged for 5 minutes at 1500 rpm and supernatant was harvested. 100 µL was collected and the remaining supernatant was centrifuged again. A further 30 µL was collected for a total of 130 µL supernatant. Supernatant vessels were sealed and stored at -80°C until used. IL-6 and CXCL2 expression was analysed by HTRF (CisBio / Revvity, "Human IL-6 HTRF Kit", https://uk.cisbio.eu/human-il6-kit-40419) and ELISA (Bio-Techne Ltd., "Human CXCL2/GRO beta DuoSet ELISA", https://www.mdsystems.com/products/human-cxcl2-gro-beta-duoset-elisa_dy276-05), respectively, according to the supplier's protocol.

As shown in **Figure 3A****,** no appreciable IL-6 expression was observed for organoids, fibroblasts, or co-cultures in the absence of proinflammatory stimulation. Proinflammatory stimulation induced expression of IL-6 in fibroblasts cultured alone, and in co-cultures, but not in organoids cultured alone.

As shown in **Figure 3B****,** proinflammatory stimulation promoted some expression of CXCL2 in fibroblasts and organoids separately (compared to fibroblasts and organoids when cultured alone without proinflammatory stimulation), but showed a clear synergistic induction in co-cultures with proinflammatory stimulation (compared to co-cultures without proinflammatory stimulation).

As shown in **Figures 3C and 3D****,** similar results were obtained in the different fibroblast cell lines shown in **Table** 1 above in the absence ("-") and presence ("+") of proinflammatory stimulation.

As shown in **Figure 3E****,** similar CXCL2 results were observed in the three different primary organoids "A", "B" and "C" (derived from small intestine).

Higher fibroblast-organoid ratios led to increased secretion of IL6 and CXCL2, as shown in **Figures 3F and 3G****,** independent of increasing the concentration of proinflammatory stimuli (from 0 ng/ml, to 5 ng/ml, to 15 ng/ml for each of IL-1β and OSM). Thus, an increased fibroblast presence relative to organoids, leads to an increased proinflammatory profile in the co-culture when prepared with proinflammatory stimulation.

### Example 6: Caspase measurements show increased apoptosis when co-cultures are prepared with proinflammatory stimuli and challenged with inflammatory stimuli

72 hours after organoids and fibroblasts were combined in co-culture, apoptosis in co-culture was triggered using inflammatory stimuli (in the form of damage-inducing cytokines), and measured 24 hours later, in co-cultures prepared using proinflammatory stimuli and in co-cultures not prepared using proinflammatory stimuli.

Stock solutions of TNF and IFNγ were diluted 1:1 with 0.6% Tween in PBS, and were added to the co-cultures at concentrations of 15 ng/mL and 15 ng/mL respectively. Plates were then incubated for 24 hours.

Apoptosis was assessed using a Caspase-Glo assay (e.g. Promega) to determine relative caspase 3/7 activity. Briefly, 95 µL of room-temperature caspase reagent was added to each plate. Plates were then shaken for 1 minute before incubation in the dark at room temperature for 30 minutes. A 160-µL sample from each plate was then transferred to a black wall plate for analysis.

As shown in **Figure 4A****,** in the absence of proinflammatory stimuli, fibroblasts in co-culture protect against the elevated caspase activity induced by inflammatory stimuli. This protection is lost when co-cultures were prepared with proinflammatory stimuli, resulting in increased apoptosis relative to co-cultures not prepared using proinflammatory stimuli. Furthermore, the proinflammatory stimulus increases caspase activity baseline (i.e. apoptosis in the presence of proinflammatory stimuli but absent inflammatory stimuli).

As shown in **Figure 4B****,** similar results were obtained in the diverse fibroblast cell lines given in **Table 1** above.

As shown in **Figure 4C****,** similar results were obtained using the three alternative primary organoids "A", "B" and "C" (all derived from small intestine).

Caspase activity was measured with different fibroblast-organoid ratios and concentrations of TNF/IFNγ (0 ng/mL, 1 ng/mL, 5 ng/mL, 15 ng/mL, 50 ng/mL, and 100 ng/mL - data not shown). As expected, co-cultures exposed to proinflammatory stimuli followed by inflammatory stimuli exhibited a greater increase in apoptosis than co-cultures exposed to inflammatory stimuli alone.

### Example 7: Proinflammatory stimuli exert separable effects on apoptosis and organoid size; tofacitinib ameliorates the increased apoptosis in co-cultures with a proinflammatory profile, on exposure to inflammatory stimuli

Tofacitinib is a small-molecule JAK inhibitor, used in the treatment of inflammatory conditions including UC. The effect of 10 µM tofacitinib on the parameters measured in **Examples 4 and 6** was assessed.

As shown in Figure 5A, tofacitinib treatment results in a small change in morphology (measured in accordance with **Example 4),** but as shown in **Figure 5B****,** tofacitinib greatly diminishes caspase activity upon exposure to inflammatory cytokines (measured in accordance with **Example 6).**

Also apparent from **Figures 5A-5B** is that the proinflammatory stimulus IL-1β appears to have a dominant effect on morphological change **(****Figure 5A****),** whereas OSM appears to have a dominant effect on apoptosis **(****Figure 5B****).** This suggests that different proinflammatory stimuli act via non-redundant pathways, leading to different phenotypic changes in co-culture. This has implications for the modelling, diagnosis, and treatment of inflammatory disorders.

### Example 8: Conditioned media from fibroblasts prepared with proinflammatory stimulation, triggers a proinflammatory profile in organoids cultured alone

The inventors hypothesised that the effects of proinflammatory stimulation on co-cultures may be mediated at least in part by soluble effectors secreted by fibroblasts into their surrounding medium ("conditioned medium"). Thus, conditioned medium was isolated from fibroblasts cultured alone with proinflammatory stimulation, and the conditioned medium was combined with organoids, to determine whether organoid morphology as measured in **Example 4** could be replicated without combining organoids and fibroblasts.

As shown in **Figure 6****,** an increase in organoid area and aggregation is apparent when organoids are exposed to conditioned media from fibroblasts cultured with proinflammatory stimulation, comparable to when organoids and fibroblasts are co-cultured with proinflammatory stimulation. This suggests that the inflammatory response of co-culture is mediated at least in part by soluble factors originating from fibroblasts.

### Example 9: Gene expression analysis of co-cultured organoid

Gene expression levels were investigated for several gene types in organoids cultured in expansion medium (CNM) or differentiation medium (cCDM) + serum, in the presence or absence of conditioned media from fibroblasts cultured with and without proinflammatory stimulation, per the methods of **Example 8.**

As shown in **Figure 7****,** the expression profiles of key inflammatory genes are altered.

### REFERENCES

[1] Gennaro (2000) Remington: The Science and Practice of Pharmacy. 20th edition, ISBN: 0683306472.
*[2]* Molecular Biology Techniques: An Intensive Laboratory Course, (Ream et al., eds., 1998, Academic Press).
*[3]* Methods In Enzymology (S. Colowick and N. Kaplan, eds., Academic Press, Inc.)
*[4]* Handbook of Experimental Immunology, Vols. I-IV (D.M. Weir and C.C. Blackwell, eds, 1986, Blackwell Scientific Publications)
[5] Sambrook et al. (2001) Molecular Cloning: A Laboratory Manual, 3rd edition (Cold Spring Harbor Laboratory Press).
[6] Ausubel et al. (eds) (2002) Short protocols in molecular biology, 5th edition (Current Protocols).
[7] PCR (Introduction to Biotechniques Series), 2nd ed. (Newton & Graham eds., 1997, Springer Verlag).
[8] Clevers, Cell. 2016 Jun 16;165(7):1586-1597
[9] Co et al. (2019) Cell Reports 26, 2509-2520
[10] M van de Wetering et al. (2015) Cell.;161(4):933-45
[11] Hayashi et al. (2004) Matrix Biology 23:47 62
[12] Nusse and Clevers (2017) Cell 169(6):985-999
[13] De Lau, W. et al. Nature, 2011 Jul 4;476(7360):293-7
[14] Lau et al. Genome Biol. 2012;13(3):242 (2012
[15] WO2012/140274
[16] Greengard, P., and Meijer, L. (2004) Structural basis for the synthesis of indirubins as potent and selective inhibitors of glycogen synthase kinase-3 and cyclin-dependent kinases. J Med Chem 47: 935-946
[17] Thomas Kramer, Boris Schmidt, and Fabio Lo Monte, "Small-Molecule Inhibitors of GSK-3: Structural Insights and Their Application to Alzheimer's Disease Models," International Journal of Alzheimer's Disease, vol. 2012, Article ID 381029, 32 pages, 2012. https://doi.org/10.1155/2012/381029
[18] Kirikoshi H et al 2001 Biochem Biophys Res Com 283 798-805
[19] "THE WNT FAMILY OF SECRETED PROTEINS", R&D Systems Catalog, 2004
[20] WO2012/168930
[21] Janda CY, et al. (2017) Nature;545(7653):234-237
[22] Miao, Y. et al. (2020) Cell Stem Cell. 27 (5), 840-851
[23] Janda CY, et al. Surrogate Wnt agonists that phenocopy canonical Wnt and β-catenin signalling. Nature. 2017 May 11;545(7653):234-237
[24] Miao, Y. et al. (Next-generation surrogate Wnts support organoid growth and deconvolute Frizzled pleiotropy in vivo. Cell Stem Cell. 27 (5), 840-851 (2020)
[25] WO2016/083613
[26] WO2016/083612
[27] Zilberberg et al., 2007. BMC Cell Biol. 8:41
[28] Robinton et al. Nature 481, 295, 2012
[29] Olive and Banath (2006) Nature Protocols volume 1, pages 23-29
[30] Ivashkevich et al (2012) Cancer Lett. 327(1-2): 123-133. doi: 10.1 016/j .canlet.2011.12.025.

## Claims

1. A method for determining the presence or absence of at least one change in a co-culture comprising at least one organoid and at least one stromal cell, wherein the method comprises:
combining the at least one organoid with the at least one stromal cell in a co-culture medium to form the co-culture; and
determining the presence or absence of the at least one change in the co-culture, optionally wherein:
(a) the method comprises preparing the at least one stromal cell by culturing stromal cells in a stromal cell medium; and/or
(b) the method comprises preparing the at least one organoid by culturing epithelial cells in an organoid medium.

2. A method of testing at least one therapeutic agent for a disease, the method comprising:
combining at least one organoid with at least one stromal cell in a co-culture medium to form a co-culture;
applying the at least one therapeutic agent to the co-culture; and
determining the presence or absence of at least one change in the co-culture,
optionally wherein:
(a) the method comprises preparing the at least one stromal cell by culturing stromal cells in a stromal cell medium; and/or
(b) the method comprises preparing the at least one organoid by culturing epithelial cells in an organoid medium.

3. A method of determining the presence or absence of a diagnosis and/or prognosis of a disease in a subject, the method comprising:
combining at least one organoid with at least one stromal cell in a co-culture medium to form a co-culture;
applying at least one diagnostic agent to the co-culture; and
determining the presence or absence of at least one change in the co-culture,
optionally wherein:
(a) the method comprises preparing the at least one stromal cell by culturing stromal cells in a stromal cell medium; and/or
(b) the method comprises preparing the at least one organoid by culturing epithelial cells in an organoid medium.

4. The method of any preceding claim, wherein determining the presence or absence of at least one change in the co-culture comprises:
(a) determining the presence or absence of stromal cells in the co-culture, optionally determining the quantity of stromal cells in the co-culture; and/or
(b) adding at least one inflammatory stimulus to the co-culture medium and determining the presence or absence of an inflammatory response, optionally wherein:
i. the at least one inflammatory stimulus comprises a cytokine, optionally wherein the cytokine comprises TNF and/or IFNγ; and/or
ii. the inflammatory response comprises increased caspase activity and/or apoptosis in the co-culture; and/or
(c) adding at least one proinflammatory stimulus to the co-culture medium, optionally wherein:
i. the at least one proinflammatory stimulus is added to the co-culture medium prior to the addition of any inflammatory stimulus to the co-culture medium; and/or
ii. the at least one proinflammatory stimulus comprises at least one cytokine, optionally wherein the at least one cytokine comprises IL-1β and/or oncostatin M (OSM).

5. The method of any preceding claim, wherein determining the presence or absence of at least one change comprises (a) adding at least one proinflammatory stimulus to the co-culture medium, and determining the presence or absence of a proinflammatory profile, and optionally (b) adding at least one inflammatory stimulus to the co-culture medium, and determining the presence or absence of an inflammatory response.

6. The method of any one of claims 2-5, wherein the disease is an inflammatory disease or a fibrotic disease, optionally wherein the disease is an inflammatory disease, optionally wherein the inflammatory disease is a gastrointestinal inflammatory disease, optionally wherein the inflammatory disease is inflammatory bowel disease (IBD), optionally wherein the IBD comprises ulcerative colitis (UC) or Crohn's disease (CD).

7. The method of any preceding claim, wherein the at least one change comprises a change in organoid morphology, a change in organoid size or epithelial cell size, a reduction in cell viability, a reduction in cell proliferation, an increase in cell death, a change in secretome profile, a change in cytokine secretion, an increase in cell apoptosis, an increase in caspase activity, and/or a change in the expression of one or more genes, optionally wherein the change in the expression of one or more genes comprises a change in one or more disease biomarkers, one or more fibrosis biomarkers, and/or one or more inflammation biomarkers.

8. The method of any preceding claim, wherein the at least one change comprises a change in secretome profile, optionally a change in secretion of a cytokine, optionally a change in secretion of IL-6 and/or CXCL2.

9. The method of any preceding claim, wherein the presence or absence of the at least one change is determined relative to a reference organoid, optionally wherein the reference organoid does not comprise stromal cells.

10. The method of any preceding claim, wherein the at least one organoid is a patient-derived organoid.

11. The method of any preceding claim, wherein the at least one stromal cell comprises a fibroblast cell, optionally wherein the fibroblast cell is an intestinal fibroblast cell, for example a colon fibroblast or small intestine fibroblast, optionally wherein the fibroblast cell is an immortalized human colon fibroblast, a human colon fibroblast, an immortalized human small intestine fibroblast, or a human small intestine fibroblast.

12. The method of any preceding claim, wherein the co-culture, the least one organoid, and/or the at least one stromal cell:
(a) is derived from the subject; and/or
(b) is derived from lung tissue, kidney tissue, pancreatic tissue, or liver tissue; and/or
(c) is derived from fibrotic tissue; and/or
(d) is derived from inflamed tissue; and/or
(e) comprises or consists of mammalian cells, optionally human cells; and/or
(f) is derived from the intestines, optionally from the colon.

13. The method of any preceding claim, wherein the at least one organoid and the at least one stromal cell are derived from the same subject, optionally from (a) the same sample from the subject and/or (b) the same tissue in the same subject.

14. The method of any preceding claim, wherein the co-culture medium comprises a mitogenic growth factor, a BMP inhibitor, and an R-spondin, optionally wherein the co-culture medium comprises a Wnt agonist, a mitogenic growth factor, a BMP inhibitor, and an R-spondin, optionally wherein the co-culture medium comprises N-Ac, a TGF-β inhibitor, a mitogenic growth factor, gastrin, a BMP inhibitor, an antibiotic, an R-spondin, a Notch pathway inhibitor, an ERK inhibitor, and a Wnt agonist.

15. The method of any preceding claim, wherein the co-culture medium comprises a serum, optionally fetal calf serum, optionally 5% fetal calf serum.
